# EUROPEAN PATENT APPLICATION

(11) **EP 3 085 695 A1**
(43) Date of publication of application: **26.10.2016**
(21) Application number: 14871626.9
(22) Date of filing: 17.12.2014
(51) Int. Cl.: C07D 253/06, A61K 31/53, A61P 3/10, A61P 7/00, A61P 9/06, A61P 9/10, A61P 9/12, A61P 13/10, A61P 13/12, A61P 15/08, A61P 15/10, A61P 17/00, A61P 17/04, A61P 25/00, A61P 25/04, A61P 25/08, A61P 25/14, A61P 25/18, A61P 25/22, A61P 25/24

(54) **SUBSTITUTED TRIAZINONE COMPOUND AND T-TYPE CALCIUM CHANNEL INHIBITOR**

(30) Priority: 17.12.2013 JP 2013259970
(71) Applicant: Nissan Chemical Industries, Ltd., Chiyoda-ku Tokyo 101-0054 (JP)
(72) Inventor: ADACHI, Michiaki, Funabashi-shi Chiba 274-8507 (JP); MINAMI, Masataka, Funabashi-shi Chiba 274-8507 (JP); EGI, Jun, Funabashi-shi Chiba 274-8507 (JP); HIRAI, Yuichi, Shiraoka-shi Saitama 349-0294 (JP); IWAMOTO, Toshimasa, Funabashi-shi Chiba 274-8507 (JP); SHINTANI, Yusuke, Funabashi-shi Chiba 274-8507 (JP); OKADA, Takuya, Funabashi-shi Chiba 274-8507 (JP); TAKAHASHI, Daiki, Shiraoka-shi Saitama 349-0294 (JP)
(74) Representative: Hoffmann Eitle
(86) International application number: PCT/JP2014/083427
(87) International publication number: WO 2015/093534

(57) **Abstract**

It is an object to provide a novel triazinone compound which has an inhibitory activity on a T-type voltage-dependent calcium channel, and is specifically useful for prevention or treatment of pain, chronic kidney disease and atrial fibrillation. A novel triazinone compound of Formula (I): 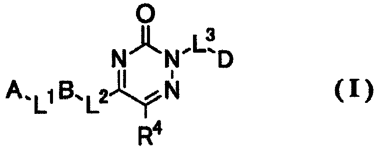 wherein each substituent in the formula is defined in detail in the description, R⁴ means a hydrogen atom, or a C₁₋₆ alkoxy group, etc., L¹ and L² each independently mean a single bond, or NR², etc., L³ means a C₁₋₆ alkylene group, etc., A means a C₆₋₁₄ aryl group or a 5 to 10-membered heteroaryl group which may be substututed, B means a C₃₋₁₁ cycloalkylene group, etc., D means a C₆₋₁₄ aryl amino group or a 5 to 10-membered heteroaryl group which may be substituted, etc., a tautomer of the compound, a pharmaceutically acceptable salt of the compound, or a solvate of the compound, the tautomer, or the pharmaceutically acceptable salt.

## Description

### TECHNICAL FIELD

The present invention relates to a novel triazinone compound having an inhibitory activity on a T-type voltage-dependent calcium channel.

### BACKGROUND ART

Voltage-dependent calcium channels are a transmembrane multisubunit proteins that control inflow of extracellular calcium ions into cells. The voltage-dependent calcium channels are further classified into various types in mammalian cells. Major types of the voltage-dependent calcium channels include L-type, T-type, N-type, P/Q type, and R-type calcium channels, which play respective roles in various tissues including skeletal muscles, cardiac muscles, lungs, smooth muscles, and brain. Among these types, the "T-type" (or "low-voltage activated-type") calcium channel is named after its characteristic that has a shorter (T = transient) opening time than the L-type calcium channel that has a longer (L = long-lasting) opening time [Non-Patent Document 1].

The T-type calcium channel has channels characteristics, which are known to be a factor to open the L-type calcium channel and a factor to fluctuate the action potential of sodium channels. Here, hyperexcitability of nerves due to an abnormality (abnormal firing) in fluctuations of the action potential of the sodium channels is believed to be a pathogenesis of neuropathic pains. The T-type calcium channels are supposed to relate to the abnormal firing, and blocking of the T-type calcium channels are believed to suppress the abnormal firing itself and to suppress pains [Non-Patent Document 2].

More specifically, the T-type calcium channels identified in various mammals including humans include three subtypes, α1G (Cav3.1), α1H (Cav3.2), and α1I (Cav3.3). Among the tree subtypes of the T-type calcium channels, α1H is expressed in the dorsal root ganglion (DRG) and the dorsal horn of the spinal cord, which relate to pain transmission [Non-Patent Document 2, Non-Patent Document 12]. In studies using α1H knockout mice, analgesic action has been reported in acute pain models (tail clip, tail flip, and hot plate tests), inflammatory pain models (capsaicin and formalin-induced tests), and visceral pain models (acetic acid and magnesium sulfate inductions). During the tests, no abnormality was observed in general behavior [Non-Patent Document 3].

Analgesic action has also been identified in neuropathic pain model rats (CCD) to which an antisense gene of α1H is administered to suppress the expression of α1H in the spinal cord [Non-Patent Document 4]. In addition, in the case of suppressing the expression in the DRG, analgesic action has been identified in neuropathic pain model rats (CCI) [Non-Patent Document 5].

As for the action on pains associated with diabetic neuropathy, in the DRG of pain model rats having diabetic neuropathy prepared by administration of streptozotocin, an increase in gene expression of α1H [Non-Patent Document 6] and an increase in T-type calcium channel current [Non-Patent Document 7] have been reported, and the pain suppressive action has also been identified by the intrathecal administration of an antisense gene of α1H to the pain model rats [Non-Patent Document 8]. It has been reported that the onset of pain has been completely suppressed in α1H knockout mice to which streptozotocin has been administered, and the expression of α1H in the DRG has increased and the administration of a T-type calcium channel inhibitor has provided an analgesic action in ob/ob mice as diabetic model mice [Non-Patent Document 9]. From these findings, compounds having the inhibitory activity on the T-type calcium channel can be used as a therapeutic agent for pain.

The T-type calcium channels are considered to relate to pains such as neuropathic pain, inflammatory pain, and cancer pain, and also pathology of various diseases and disorders including epilepsy, essential tremor, schizophrenia, Parkinson's disease, depression, anxiety, sleep disorder, sleep disturbance, mental illness, schizophrenia, cardiac arrhythmia, hypertension, cancer, diabetes, overactive bladder, chronic kidney disease, sterility, and sexual dysfunction [Non-Patent Document 2, Non-Patent Document 3, Non-Patent Document 10, Non-Patent Document 11, Non-Patent Document 13, Non-Patent Document 14, Non-Patent Document 15, and Non-Patent Document 16].

Treatment methods for such diseases involve many problems, and thus there is a demand for development of novel pharmaceutical products. Although some compounds having the T-type calcium channel inhibitory activity have been reported (for example, see Patent Documents 1 to 4), there is a demand for further development of medicinal agents.

### Prior Art Documents

### Patent Documents

Patent Document 1: International Publication WO 2009/146540
Patent Document 2: International Publication WO 2011/115813
Patent Document 3: International Publication WO 2011/035159
Patent Document 4: International Publication WO 2013/147183

### Non-Patent Documents

Non-Patent Document 1: Physiology of Neuron, Kyoto University Press, pp. 231-260 (2009)
Non-Patent Document 2: British Journal of Pharmacology, Vol. 163, pp. 484-495 (2011)
Non-Patent Document 3: Genes, Brain and Behavior, Vol. 6, pp. 425-431 (2007)
Non-Patent Document 4: Acta Pharmacologica Sinica, Vol. 27 (No. 12), pp. 1547-1552 (2006)
Non-Patent Document 5: The EMBO Journal, Vol. 24, pp. 315-324 (2005)
Non-Patent Document 6: Journal ofNeurochemistry, Vol. 119 (No. 3), pp. 594-603 (2011)
Non-Patent Document 7: Journal of Neuroscience, Vol. 27 (No. 12), pp. 3305-3316 (2007)
Non-Patent Document 8: Pain, Vol. 145 (Nos. 1-2), pp. 184-195 (2009)
Non-Patent Document 9: Diabetes, Vol. 58, pp. 2656-2665 (2009)
Non-Patent Document 10: The Journal of Pharmacology and Experimental Therapeutics, Vol. 335, No. 2, pp. 409-417 (2010)
Non-Patent Document 11: Journal of Assisted Reproduction and Genetics, Vol. 28, No. 1, pp. 23-30 (2011)
Non-Patent Document 12: Physiological Reviews, Vol. 83, pp. 117-161 (2003)
Non-Patent Document 13: Neurourology and Urodynamics, Vol. 26, pp. 870-878 (2007)
Non-Patent Document 14: BJU International, Vol. 99 (No. 2), pp. 436-441 (2006)
Non-Patent Document 15: American Journal of Hypertension, Vol. 30, No. 8, pp. 1620-1631 (2012)
Non-Patent Document 16: Bioorganic and Medicinal Chemistry Letters, Vol. 23 No. 1, pp. 119-124 (2013)

### SUMMARY OF THE INVENTION

### Problem to be Solved by the Invention

The present invention aims to provide a novel triazinone compounds which are T-type voltage-dependent calcium channel inhibitors. The present invention also aims to provide pharmaceutical compositions containing the compounds of the present invention.

### Means for Solving the Problem

As a result of intensive studies for developing inhibitors of a T-type voltage-dependent calcium channels, the inventors of the present invention have found that the compounds of the present invention have a high inhibitory activity on the T-type voltage-dependent calcium channels and have accomplished the present invention.

Specifically, the present invention has the following aspects:
(1) A compound of Formula (I): 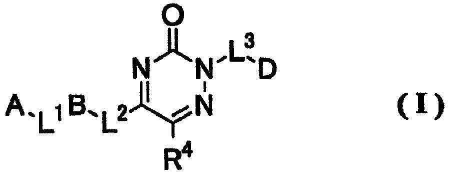 [wherein
   R⁴ means a hydrogen atom, a halogen atom, a cyano group, a nitro group, a carboxy group, a carbamoyl group, a sulfamoyl group, a sulfo group, a tetrazolyl group, a C₁₋₆ alkyl group, a C₂₋₆ alkenyl group, a C₂₋₆ alkynyl group, a C₁₋₆ alkoxy group, a C₁₋₆ alkylthio group, a mono-C₁₋₆ alkyl amino group, or a di-C₁₋₆ alkyl amino group (the C₁₋₆ alkyl group, the C₂₋₆ alkenyl group, the C₂₋₆ alkynyl group, the C₁₋₆ alkoxy group, the C₁₋₆ alkylthio group, the mono-C₁₋₆ alkylamino group, and the di-C₁₋₆ alkylamino group are unsubstituted or substituted with one or more substituents independently selected from a substituent group V⁸);
   L¹ and L² each independently mean a single bond, NR², O, S, SO, SO₂ or a C₁₋₆ alkylene group (the C₁₋₆ alkylene group is unsubstituted or substituted with one or more substituents independently selected from the substituent group V⁸ and a single methylene group in the C₁₋₆ alkylene group is optionally replaced by O, S, SO₂, C=O, C=S, or NR³);
   B means a 3 to 13-membered heterocyclylene group (the 3 to 13-membered heterocyclylene group is unsubstituted or substituted with one or more substituents independently selected from a substituent group V⁶ and a single methylene group in the 3 to 13-membered heterocyclylene group is optionally replaced by a 1,1-C₃₋₇ cycloalkylene group);
   A means a hydrogen atom, a C₃₋₁₁ cycloalkyl group, a C₃₋₁₁ cycloalkenyl group, a 3 to 13-membered heterocyclyl group, a C₆₋₁₄ aryl group, or a 5 to 10-membered heteroaryl group (the C₃₋₁₁ cycloalkyl group, the C₃₋₁₁ cycloalkenyl group, the 3 to 13-membered heterocyclyl group, the C₆₋₁₄ aryl group, and the 5 to 10-membered heteroaryl group are unsubstituted or substituted with one or more substituents independently selected from the substituent group V⁶);
   L³ means a C₁₋₆ alkylene group (the C₁₋₆ alkylene group is unsubstituted or substituted with one or more substituents independently selected from the substituent group V⁸ and a single methylene group in the C₁₋₆ alkylene group is optionally replaced by C=O or C=S);
   D means a 3 to 13-membered heterocyclyl group, a C₆₋₁₄ aryl group, or a 5 to 10-membered heteroaryl group (the 3 to 13-membered heterocyclyl group, the C₆₋₁₄ aryl group, and the 5 to 10-membered heteroaryl group are unsubstituted or substituted with one or more substituents independently selected from the substituent group V⁶);
   R² and R³ each independently mean a hydrogen atom, a C₁₋₆ alkyl group, a C₂₋₆ alkenyl group, a C₂₋₆ alkynyl group (the C₁₋₆ alkyl group, the C₂₋₆ alkenyl group, and the C₂₋₆ alkynyl group are unsubstituted or substituted with one or more substituents independently selected from the substituent group V⁸), a C₃₋₁₁ cycloalkyl group, a 3 to 13-membered heterocyclyl group, a C₆₋₁₄ aryl group, or a 5 to 10-membered heteroaryl group (the C₃₋₁₁ cycloalkyl group, the 3 to 13-membered heterocyclyl group, the C₆₋₁₄ aryl group, and the 5 to 10-membered heteroaryl group are unsubstituted or substituted with one or more substituents independently selected from the substituent group V⁶);
   the substituent group V⁶ means a substituent group consisting of substituents constituting the substituent group V⁸, C₁₋₆ alkyl groups, C₂₋₆ alkenyl groups, and C₂₋₆ alkynyl groups (the C₁₋₆ alkyl groups, the C₂₋₆ alkenyl groups, and the C₂₋₆ alkynyl groups are unsubstituted or substituted with one or more substituents independently selected from a substituent group V¹);
   the substituent group V⁸ means a substituent group consisting of substituents constituting a substituent group V⁸, C₁₋₁₀ alkoxy groups, C₁₋₆ alkylthio groups, C₁₋₆ alkylcarbonyl groups, C₁₋₆ alkylsulfonyl groups, C₁₋₆ alkoxycarbonyl groups, mono-C₁₋₆ alkylamino groups, di-C₁₋₆ alkylamino groups, mono-C₁₋₆ alkylaminocarbonyl groups, di-C₁₋₆ alkylaminocarbonyl groups, mono-C₁₋₆ alkylaminosulfonyl groups, di-C₁₋₆ alkylaminosulfonyl groups, C₁₋₆ alkylcarbonylamino groups, C₁₋₆ alkylcarbonyloxy groups, C₁₋₆ alkylsulfonylamino groups, C₁₋₆ alkylsulfonyloxy groups (the C₁₋₁₀ alkoxy groups, the C₁₋₆ alkylthio groups, the C₁₋₆ alkylcarbonyl groups, the C₁₋₆ alkylsulfonyl groups, the C₁₋₆ alkoxycarbonyl groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the mono-C₁₋₆ alkylaminosulfonyl groups, the di-C₁₋₆ alkylaminosulfonyl groups, the C₁₋₆ alkylcarbonylamino groups, the C₁₋₆ alkylcarbonyloxy groups, the C₁₋₆ alkylsulfonylamino groups, and the C₁₋₆ alkylsulfonyloxy groups are unsubstituted or substituted with one or more substituents independently selected from the substituent group V¹), C₃₋₆ cycloalkoxy groups, C₆₋₁₄ aryloxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylsulfonylamino groups, C₃₋₆ cycloalkylsulfonyloxy groups, C₃₋₆ cycloalkylthio groups, C₃₋₁₁ cycloalkyl groups, 3 to 13-membered heterocyclyl groups, C₆₋₁₄ aryl groups, and 5 to 10-membered heteroaryl groups (the C₃₋₆ cycloalkoxy groups, the C₆₋₁₄ aryloxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylsulfonylamino groups, the C₃₋₆ cycloalkylsulfonyloxy groups, the C₃₋₆ cycloalkylthio groups, the C₃₋₁₁ cycloalkyl groups, the 3 to 13-membered heterocyclyl groups, the C₆₋₁₄ aryl groups, and the 5 to 10-membered heteroaryl groups are unsubstituted, or substituted with one or more substituents independently selected from a substituent group V²);
   the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group;
   the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups, (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups); and
   the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups)], a tautomer of the compound, a pharmaceutically acceptable salt of the compound, or a solvate of the compound, the tautomer, or the pharmaceutically acceptable salt.
(2) The compound, the tautomer of the compound, the pharmaceutically acceptable salt of the compound, or the solvate of the compound, the tautomer, or the pharmaceutically acceptable salt according to (1), in which L³ is a C₁₋₃ alkylene group.
(3) The compound, the tautomer of the compound, the pharmaceutically acceptable salt of the compound, or the solvate of the compound, the tautomer, or the pharmaceutically acceptable salt according to (2), in which L³ is a methylene group.
(4) The compound, the tautomer of the compound, the pharmaceutically acceptable salt of the compound, or the solvate of the compound, the tautomer, or the pharmaceutically acceptable salt according to any one of (1) to (3), in which R⁴ is a hydrogen atom.
(5) The compound, the tautomer of the compound, the pharmaceutically acceptable salt of the compound, or the solvate of the compound, the tautomer, or the pharmaceutically acceptable salt according to any one of (1) to (4), in which L¹ and L² are single bonds;
   B is a 3 to 13-membered heterocyclylene group (the 3 to 13-membered heterocyclylene group is unsubstituted or substituted with one or more substituents independently selected from a substituent group V⁵ and a single methylene group in the 3 to 13-membered heterocyclylene group is optionally replaced by a 1,1-C₃₋₇ cycloalkylene group);
   the substituent group V⁵ means a substituent group consisting of a hydroxy group, halogen atoms, a cyano group, a nitro group, an amino group, C₁₋₆ alkyl groups, C₂₋₆ alkenyl groups, C₂₋₆ alkynyl groups, C₁₋₆ alkoxy groups, C₁₋₆ alkylthio groups, C₁₋₆ alkoxycarbonyl groups, mono-C₁₋₆ alkylamino groups, di-C₁₋₆ alkylamino groups, C₁₋₆ alkylsulfonylamino groups, C₁₋₆ alkylsulfonyloxy groups (the C₁₋₆ alkyl groups, the C₂₋₆ alkenyl groups, the C₂₋₆ alkynyl groups, the C₁₋₆ alkoxy groups, the C₁₋₆ alkylthio groups, the C₁₋₆ alkoxycarbonyl groups, the mono-C₁₋₆ alkylamino groups, the di-C₁₋₆ alkylamino groups, the C₁₋₆ alkylsulfonylamino groups, and the C₁₋₆ alkylsulfonyloxy groups are unsubstituted, or substituted with one or more substituents independently selected from a substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents independently selected from the substituent group V²);
   the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups, (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
   the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups); and
   the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group.
(6) The compound, the tautomer of the compound, the pharmaceutically acceptable salt of the compound, or the solvate of the compound, the tautomer, or the pharmaceutically acceptable salt according to (5), in which B is a 4 to 7-membered heterocyclylene group (the 4 to 7-membered heterocyclylene group is unsubstituted or substituted with one or more substituents independently selected from a substituent group V³ and a single methylene group in the 4 to 7-membered heterocyclylene group is optionally replaced by a 1,1-C₃₋₇ cycloalkylene group); and
   the substituent group V³ means a substituent group consisting of a hydroxy group, halogen atoms, a cyano group, a nitro group, an amino group, C₁₋₆ alkyl groups, C₁₋₆ haloalkyl groups, C₃₋₆ cycloalkyl groups, C₁₋₆ alkoxy groups, C₁₋₆ haloalkoxy groups, mono-C₁₋₆ alkylamino groups, di-C₁₋₆ alkylamino groups, and C₁₋₆ alkylsulfonylamino groups.
(7) The compound, the tautomer of the compound, the pharmaceutically acceptable salt of the compound, or the solvate of the compound, the tautomer, or the pharmaceutically acceptable salt according to (6), in which B is a 6-membered heterocyclylene group (the 6-membered heterocyclylene group is unsubstituted or substituted with one or more substituents independently selected from the group consisting of a hydroxy group, halogen atoms, and an amino group).
(8) The compound, the tautomer of the compound, the pharmaceutically acceptable salt of the compound, or the solvate of the compound, the tautomer, or the pharmaceutically acceptable salt according to (5), in which A is a hydrogen atom;
   B is a tetrahydroisoquinoline-diyl group or a tetrahydrocarboline-diyl group (the tetrahydroisoquinoline-diyl group and the tetrahydrocarboline-diyl group are unsubstituted, or substituted with one or more substituents independently selected from a substituent group V³, and further a single methylene group in the tetrahydroisoquinoline-diyl group and the tetrahydrocarboline-diyl group is optionally replaced by a 1,1-C₃₋₇ cycloalkylene group); and
   the substituent group V³ means a substituent group consisting of a hydroxy group, halogen atoms, a cyano group, a nitro group, an amino group, C₁₋₆ alkyl groups, C₁₋₆ haloalkyl groups, C₃₋₆ cycloalkyl groups, C₁₋₆ alkoxy groups, C₁₋₆ haloalkoxy groups, mono-C₁₋₆ alkylamino groups, di-C₁₋₆ alkylamino groups, and C₁₋₆ alkylsulfonylamino groups.
(9) The compound, the tautomer of the compound, the pharmaceutically acceptable salt of the compound, or the solvate of the compound, the tautomer, or the pharmaceutically acceptable salt according to (8), in which A-L¹-B is either a structure of Formula (II-1) or a structure of Formula (II-2): 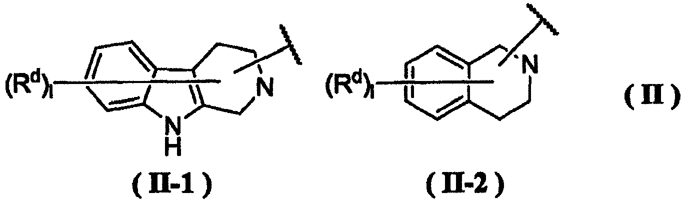 (wherein 1 is 1 to 3; R^{d} is optionally substituted for a tetrahydrocarboline ring or a tetrahydroisoquinoline ring at any positions; R^{d} means a hydroxy group, a halogen atom, an amino group, a C₁₋₆ alkyl group, a C₁₋₆ haloalkyl group, a C₁₋₆ alkoxy group, a C₁₋₆ haloalkoxy group, a mono-C₁₋₆ alkylamino group, a di-C₁₋₆ alkylamino group, or a C₁₋₆ alkylsulfonylamino group; and when 1 is 2 or 3, R^{d}s are the same as or different from each other).
(10) The compound, the tautomer of the compound, the pharmaceutically acceptable salt of the compound, or the solvate of the compound, the tautomer, or the pharmaceutically acceptable salt according to (1) to (7), in which A is a C₆₋₁₄ aryl group or a 5 to 10-membered heteroaryl group (the C₆₋₁₄ aryl group and the 5 to 10-membered heteroaryl group are unsubstituted or substituted with one or more substituents independently selected from a substituent group V⁵);
   the substituent group V⁵ means a substituent group consisting of a hydroxy group, halogen atoms, a cyano group, a nitro group, an amino group, C₁₋₆ alkyl groups, C₂₋₆ alkenyl groups, C₂₋₆ alkynyl groups, C₁₋₆ alkoxy groups, C₁₋₆ alkylthio groups, C₁₋₆ alkoxycarbonyl groups, mono-C₁₋₆ alkylamino groups, di-C₁₋₆ alkylamino groups, C₁₋₆ alkylsulfonylamino groups, C₁₋₆ alkylsulfonyloxy groups (the C₁₋₆ alkyl groups, the C₂₋₆ alkenyl groups, the C₂₋₆ alkynyl groups, the C₁₋₆ alkoxy groups, the C₁₋₆ alkylthio groups, the C₁₋₆ alkoxycarbonyl groups, the mono-C₁₋₆ alkylamino groups, the di-C₁₋₆ alkylamino groups, the C₁₋₆ alkylsulfonylamino groups, and the C₁₋₆ alkylsulfonyloxy groups are unsubstituted, or substituted with one or more substituents independently selected from a substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents independently selected from a substituent group V²);
   the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
   the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino group, C₁₋₆ alkylthio groups, and C₁₋₆ alkylsulfonyl groups); and
   the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group.
(11) The compound, the tautomer of the compound, the pharmaceutically acceptable salt of the compound, or the solvate of the compound, the tautomer, or the pharmaceutically acceptable salt according to (10), in which A is a phenyl group or a 5 to 6-membered heteroaryl group (the phenyl group and the 5 to 6-membered heteroaryl group are unsubstituted or substituted with one or more substituents independently selected from a substituent group V⁴); and
   the substituent group V⁴ means a substituent group consisting of a hydroxy group, halogen atoms, a cyano group, a nitro group, an amino group, C₁₋₆ alkyl groups, C₁₋₆ alkoxy groups, mono-C₁₋₆ alkylamino groups, di-C₁₋₆ alkylamino groups, C₁₋₆ alkylsulfonylamino groups, C₁₋₆ alkylsulfonyloxy groups, C₃₋₆ cycloalkyl groups, C₃₋₆ cycloalkoxy groups, mono-C₃₋₆ cycloalkylamino groups, di-C₃₋₆ cycloalkylamino groups, and C₃₋₆ cycloalkylthio groups (the C₁₋₆ alkyl groups, the C₁₋₆ alkoxy groups, the mono-C₁₋₆ alkylamino groups, the di-C₁₋₆ alkylamino groups, the C₁₋₆ alkylsulfonylamino groups, the C₁₋₆ alkylsulfonyloxy groups, the C₃₋₆ cycloalkyl groups, the C₃₋₆ cycloalkoxy groups, the mono-C₃₋₆ cycloalkylamino groups, the di-C₃₋₆ cycloalkylamino groups, and the C₃₋₆ cycloalkylthio groups are unsubstituted or substituted with one or more substituents independently selected from the group consisting of a hydroxy group, halogen atoms, an amino group, a nitro group, a cyano group, C₁₋₆ alkoxy groups, C₁₋₃ haloalkoxy groups, 3 to 13-membered heterocyclyl groups, C₆₋₁₄ aryl groups, and 5 to 10-membered heteroaryl groups (the 3 to 13-membered heterocyclyl groups, the C₆₋₁₄ aryl groups, and the 5 to 10-membered heteroaryl groups are unsubstituted or substituted with one or more substituents independently selected from the group consisting of a carboxy group, a carbamoyl group, a sulfamoyl group, a phosphono group, a sulfo group, a tetrazolyl group, a formyl group, a nitro group, a cyano group, halogen atoms, a hydroxy group, an amino group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, Q₁₋₆ alkylthio groups, and C₁₋₆ alkylsulfonyl groups)).
(12) The compound, the tautomer of the compound, the pharmaceutically acceptable salt of the compound, or the solvate of the compound, the tautomer, or the pharmaceutically acceptable salt according to (11), in which A is a phenyl group (the phenyl group has one or more substituents independently selected from the group consisting of halogen atoms, a nitro group, C₁₋₆ alkyl groups, C₁₋₆ haloalkyl groups, C₁₋₆ alkoxy groups, and C₁₋₆ haloalkoxy groups).
(13) The compound, the tautomer of the compound, the pharmaceutically acceptable salt of the compound, or the solvate of the compound, the tautomer, or the pharmaceutically acceptable salt according to (11), in which A is a thienyl group, a pyridyl group, a pyrazolyl group, or a furanyl group (the thienyl group, the pyridyl group, the pyrazolyl group, and the furanyl group have one or more substituents independently selected from the group consisting of halogen atoms, C₁₋₆ alkyl groups, C₁₋₆ haloalkyl groups, C₁₋₆ alkoxy groups, and C₁₋₆ haloalkoxy groups).
(14) The compound, the tautomer of the compound, the pharmaceutically acceptable salt of the compound, or the solvate of the compound, the tautomer, or the pharmaceutically acceptable salt according to (11), in which A is a thienyl group or a pyridyl group (the thienyl group and the pyridyl group have one or more substituents independently selected from the group consisting of halogen atoms, C₁₋₆ alkyl groups, C₁₋₆ haloalkyl groups, C₁₋₆ alkoxy groups, and C₁₋₆ haloalkoxy groups).
(15) The compound, the tautomer of the compound, the pharmaceutically acceptable salt of the compound, or the solvate of the compound, the tautomer, or the pharmaceutically acceptable salt according to any one of (1) to (14), in which D is a C₆₋₁₄ aryl group or a 5 to 10-membered heteroaryl group (the C₆₋₁₄ aryl group and the 5 to 10-membered heteroaryl group are unsubstituted or substituted with one or more substituents independently selected from a substituent group V⁵);
   the substituent group V⁵ means a substituent group consisting of a hydroxy group, halogen atoms, a cyano group, a nitro group, an amino group, C₁₋₆ alkyl groups, C₂₋₆ alkenyl groups, C₂₋₆ alkynyl groups, C₁₋₆ alkoxy groups, C₁₋₆ alkylthio groups, C₁₋₆ alkoxycarbonyl groups, mono-C₁₋₆ alkylamino groups, di-C₁₋₆ alkylamino groups, C₁₋₆ alkylsulfonylamino groups, C₁₋₆ alkylsulfonyloxy groups (the C₁₋₆ alkyl groups, the C₂₋₆ alkenyl groups, the C₂₋₆ alkynyl groups, the C₁₋₆ alkoxy groups, the C₁₋₆ alkylthio groups, the C₁₋₆ alkoxycarbonyl groups, the mono-C₁₋₆ alkylamino groups, the di-C₁₋₆ alkylamino groups, the C₁₋₆ alkylsulfonylamino groups, and the C₁₋₆ alkylsulfonyloxy groups are unsubstituted, or substituted with one or more substituents independently selected from a substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents independently selected from a substituent group V²);
   the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₁₋₁₆ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₁₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
   the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
   the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group.
(16) The compound, the tautomer of the compound, the pharmaceutically acceptable salt of the compound, or the solvate of the compound, the tautomer, or the pharmaceutically acceptable salt according to (15), in which D is a phenyl group (the phenyl group is unsubstituted or substituted with one or more substituents independently selected from a substituent group V⁴); and
   the substituent group V⁴ means a substituent group consisting of a hydroxy group, halogen atoms, a cyano group, a nitro group, an amino group, C₁₋₆ alkyl groups, C₁₋₆ alkoxy groups, mono-C₁₋₆ alkylamino groups, di-C₁₋₆ alkylamino groups, C₁₋₆ alkylsulfonylamino groups, C₁₋₆ alkylsulfonyloxy groups, C₃₋₆ cycloalkyl groups, C₃₋₆ cycloalkoxy groups, mono-C₃₋₆ cycloalkylamino groups, di-C₃₋₆ cycloalkylamino groups, and C₃₋₆ cycloalkylthio groups (the C₁₋₆ alkyl groups, the C₁₋₆ alkoxy groups, the mono-C₁₋₆ alkylamino groups, the di-C₁₋₆ alkylamino groups, the C₁₋₆ alkylsulfonylamino groups, the C₁₋₆ alkylsulfonyloxy groups, the C₃₋₆ cycloalkyl groups, the C₃₋₆ cycloalkoxy groups, the mono-C₃₋₆ cycloalkylamino groups, the di-C₃₋₆ cycloalkylamino groups, and the C₃₋₆ cycloalkylthio groups are unsubstituted or substituted with one or more substituents independently selected from the group consisting of a hydroxy group, halogen atoms, an amino group, a nitro group, a cyano group, C₁₋₆ alkoxy groups, C₁₋₃ haloalkoxy groups, 3 to 13-membered heterocyclyl groups, C₆₋₁₄ aryl groups, or 5 to 10-membered heteroaryl groups (the 3 to 13-membered heterocyclyl groups, the C₆₋₁₄ aryl groups, and the 5 to 10-membered heteroaryl groups are unsubstituted or substituted with one or more substituents independently selected from the group consisting of a carboxy group, a carbamoyl group, a sulfamoyl group, a phosphono group, a sulfo group, a tetrazolyl group, a formyl group, a nitro group, a cyano group, halogen atoms, a hydroxy group, an amino group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups)).
(17) The compound, the tautomer of the compound, the pharmaceutically acceptable salt of the compound, or the solvate of the compound, the tautomer, or the pharmaceutically acceptable salt according to (16), in which D is a phenyl group (the phenyl group has one or more substituents independently selected from the group consisting of halogen atoms, a nitro group, C₁₋₆ alkyl groups, or C₁₋₆ alkoxy groups (the C₁₋₆ alkyl groups and the C₁₋₆ alkoxy groups are unsubstituted or substituted with one or more substituents independently selected from the group consisting of halogen atoms, C₁₋₆ alkoxy groups, C₁₋₃ haloalkoxy groups, and a phenyl group (the phenyl group is unsubstituted or substituted with one or more halogen atoms))).
(18) The compound, the tautomer of the compound, the pharmaceutically acceptable salt of the compound, or the solvate of the compound, the tautomer, or the pharmaceutically acceptable salt according to (15), in which D is a 5 to 6-membered heteroaryl group (the 5 to 6-membered heteroaryl group is unsubstituted or substituted with one or more substituents independently selected from a substituent group V⁴); and
   the substituent group V⁴ means a substituent group consisting of a hydroxy group, halogen atoms, a cyano group, a nitro group, an amino group, C₁₋₆ alkyl groups, C₁₋₆ alkoxy groups, mono-C₁₋₆ alkylamino groups, di-C₁₋₆ alkylamino groups, C₁₋₆ alkylsulfonylamino groups, C₁₋₆ alkylsulfonyloxy groups, C₃₋₆ cycloalkyl groups, C₃₋₆ cycloalkoxy groups, mono-C₃₋₆ cycloalkylamino groups, di-C₃₋₆ cycloalkylamino groups, and C₃₋₆ cycloalkylthio groups (the C₁₋₆ alkyl groups, the C₁₋₆ alkoxy groups, the mono-C₁₋₆ alkylamino groups, the di-C₁₋₆ alkylamino groups, the C₁₋₆ alkylsulfonylamino groups, the C₁₋₆ alkylsulfonyloxy groups, the C₃₋₆ cycloalkyl groups, the C₃₋₆ cycloalkoxy groups, the mono-C₃₋₆ cycloalkylamino groups, the di-C₃₋₆ cycloalkylamino groups, and the C₃₋₆ cycloalkylthio groups are unsubstituted or substituted with one or more substituents independently selected from the group consisting of a hydroxy group, halogen atoms, an amino group, a nitro group, a cyano group, C₁₋₆ alkoxy groups, C₁₋₃ haloalkoxy groups, 3 to 13-membered heterocyclyl groups, C₆₋₁₄ aryl groups, and 5 to 10-membered heteroaryl groups (the 3 to 13-membered heterocyclyl groups, the C₆₋₁₄ aryl groups, and the 5 to 10-membered heteroaryl groups are unsubstituted or substituted with one or more substituents independently selected from the group consisting of a carboxy group, a carbamoyl group, a sulfamoyl group, a phosphono group, a sulfo group, a tetrazolyl group, a formyl group, a nitro group, a cyano group, halogen atoms, a hydroxy group, an amino group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups)).
(19) The compound, the tautomer of the compound, the pharmaceutically acceptable salt of the compound, or the solvate of the compound, the tautomer, or the pharmaceutically acceptable salt according to (18), in which D is a 5 to 6-membered heteroaryl group (the 5 to 6-membered heteroaryl group is unsubstituted or substituted with one or more substituents independently selected from the group consisting of halogen atoms, a nitro group, C₁₋₆ alkyl groups, C₁₋₆ haloalkyl groups, C₁₋₆ alkoxy groups, C₁₋₆ haloalkoxy groups (the C₁₋₆ alkyl groups, the C₁₋₆ haloalkyl groups, the C₁₋₆ alkoxy groups, and the C₁₋₆ haloalkoxy groups are unsubstituted or substituted with one or more substituents independently selected from the group consisting of a nitro group, C₁₋₆ alkoxy groups, C₁₋₃ haloalkoxy groups, and C₆₋₁₄ aryl groups (the C₆₋₁₄ aryl groups are unsubstituted or substituted with one or more halogen atoms)), C₁₋₆ alkylsulfonylamino groups and C₁₋₆ alkylsulfonyloxy groups (the C₁₋₆ alkylsulfonylamino groups and the C₁₋₆ alkylsulfonyloxy groups are unsubstituted or substituted with one or more substituents independently selected from the group consisting of halogen atoms, a nitro group, C₁₋₆ alkoxy groups, and C₁₋₃ haloalkoxy groups)).
(20) The compound, the tautomer of the compound, the pharmaceutically acceptable salt of the compound, or the solvate of the compound, the tautomer, or the pharmaceutically acceptable salt according to (19), in which D is a thienyl group or a thiazolyl group (the thienyl group and the thiazolyl group have one or more substituents independently selected from the group consisting of halogen atoms, a nitro group, C₁₋₆ alkyl groups, C₁₋₆ haloalkyl groups, C₁₋₆ alkoxy groups, and C₁₋₆ haloalkoxy groups).
(21) A T-type calcium channel inhibitor comprising the compound, the tautomer of the compound, the pharmaceutically acceptable salt of the compound, or the solvate of the compound, the tautomer, or the pharmaceutically acceptable salt as described in any one of (1) to (20), as an active component.
(22) A preventive agent, a therapeutic agent, and/or an improving agent for a disease treatable by a T-type calcium channel inhibitory activity comprising the T-type calcium channel inhibitor as described in (21) as an active component.
(23) A therapeutic agent for neuropathic pain comprising the T-type calcium channel inhibitor as described in (21) as an active component.
(24) A medicine comprising the compound, the tautomer of the compound, the pharmaceutically acceptable salt of the compound, or the solvate of the compound, the tautomer, or the pharmaceutically acceptable salt as described in any one of (1) to (20), as an active component.

### Effects of the Invention

The present invention can provide novel triazinone compounds that have an excellent T-type calcium channel inhibitory activity and are specifically useful for prevention or treatment of neuropathic pain.

### MODES FOR CARRYING OUT THE INVENTION

The present invention will now be described in further detail.

In the present invention, "n-" means normal, "i-" means iso, "s-" and "sec-" mean secondary, "t-" and "tert-" are tertiary, "o-" means ortho, "m-" means meta, "p-" means para, "(E)-" means an E form, "(Z)-" means a Z form, "Ph" means phenyl, "Bu" means butyl, "Boc" means tertiary-butoxycarbonyl, "Cbz" means benzyloxycarbonyl, "Ts" means p-toluenesulfonyl, and "Bn" means benzyl.

First, terms used for the explanation of chemical structures in the present specification will be described.

The "halogen atom" means a fluorine atom, a chlorine atom, a bromine atom, or an iodine atom.

The "C₁₋₃ alkyl group" means a methyl group, an ethyl group, a n-propyl group, or an isopropyl group.

The "C₁₋₆ alkyl group" means a linear or branched alkyl group having a carbon atom number of 1 to 6, and specific examples include methyl group, ethyl group, n-propyl group, isopropyl group, n-butyl group, isobutyl group, t-butyl group, n-pentyl group, and n-hexyl group.

The "C₁₋₃ alkylene group" means a methylene group, an ethylene group, a propane-1,3-diyl group, or a propane-1,2-diyl group.

The "C₁₋₆ alkylene group" means a divalent substituent formed by removing a single hydrogen atom at any position of the "C₁₋₆ alkyl group" defined above, and specific examples include methylene group, ethylene group, propane-1,3-diyl group, propane-1,2-diyl group, 2,2-dimethyl-propane-1,3-diyl group, hexane-1,6-diyl group, and 3-methylbutane-1,2-diyl group.

The "C₁₋₃ haloalkyl group" means a substituent formed by substituting one or more halogen atoms independently selected from a substituent group consisting of a fluorine atom, a chlorine atom, a bromine atom, and an iodine atom for one or more hydrogen atoms at any positions of the "C₁₋₃ alkyl group" defined above, and specific examples include trifluoromethyl group, 2,2,2-trifluoroethyl group, pentafluoroethyl group, and 3-chloro-n-propyl group.

The "C₁₋₆ haloalkyl group" means a substituent formed by substituting one or more halogen atoms independently selected from a substituent group consisting of a fluorine atom, a chlorine atom, a bromine atom, and an iodine atom for one or more hydrogen atoms at any positions of the "C₁₋₆ alkyl group" defined above, and specific examples include trifluoromethyl group, pentafluoroethyl group, 2,2,2-trifluoro-1,1-dimethyl-ethyl group, 3-chloro-n-propyl group, and 4-chloro-n-butyl group.

The "C₃₋₁₁ cycloalkane" means a monocyclic-, condensed-, bridged-, or spiro-system aliphatic hydrocarbon ring having a ring-constituting carbon atom number of 3 to 11, and specific examples include cyclopropane, cyclobutane, cyclopentane, cyclohexane, cycloheptane, cyclooctane, adamantane, bicyclo[3.1.0]octane, bicyclo[2.2.1]heptane, and spiro[5.5]undecane.

The "C₃₋₁₁ cycloalkyl group" means a monovalent substituent formed by removing a single hydrogen atom at any position of the "C₃₋₁₁ cycloalkane" defined above.

The "C₃₋₁₁ cycloalkylene group" means a divalent substituent formed by removing two hydrogen atoms at any positions on different carbons of the "C₃₋₁₁ cycloalkane" defined above.

The "C₃₋₆ cycloalkane" means a cycloalkane having a ring-constituting carbon atom number of 3 to 6 among the "C₃₋₁₁ cycloalkanes" defined above, and specific examples include cyclopropane, cyclobutane, cyclopentane, and cyclohexane.

The "C₃₋₆ cycloalkyl group" means a monovalent substituent formed by removing a single hydrogen atom at any position of the "C₃₋₆ cycloalkane" defined above.

The "C₃₋₆ cycloalkylene group" means a divalent substituent formed by removing two hydrogen atoms at any positions on different carbons of the "C₃₋₆ cycloalkane" defined above.

The "C₃₋₇ cycloalkane" means a cycloalkane having a ring-constituting carbon atom number of 3 to 7 among the "C₃₋₁₁ cycloalkanes" defined above, and specific examples include cyclopropane, cyclobutane, cyclopentane, cyclohexane, and cycloheptane.

The "1,1-C₃₋₇ cycloalkylene group" means a divalent substituent formed by removing two hydrogen atoms on the same carbon of the "C₃₋₇ cycloalkane" defined above. The group is specifically exemplified by the structures shown below.

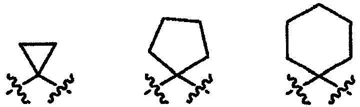

The "C₄₋₇ cycloalkane" means a cycloalkane having a ring-constituting carbon atom number of 4 to 7 among the "C₃₋₁₁ cycloalkanes" defined above, and specific examples include cyclobutane, cyclopentane, cyclohexane, and cycloheptane.

The "C₄₋₇ cycloalkylene group" means a divalent substituent formed by removing two hydrogen atoms at any positions on different carbons of the "C₄₋₇ cycloalkane" defined above.

The "C₃₋₁₁ cycloalkene" means a non-aromatic ring formed by replacing one or more of any bonds of the "C₃₋₁₁ cycloalkane" defined above by double bonds, and specific examples include cyclopropene, cyclobutene, cyclopentene, cyclohexene, cyclohexa-1,3-diene, cyclohexa-1,4-diene, bicyclo[2.2.1]hepta-2,5-diene, spiro[2.5]oct-4-ene, and 1,2,5,6-tetrahydronaphthalene.

The "C₃₋₁₁ cycloalkenyl group" means a monovalent substituent formed by removing a single hydrogen atom at any position of the "C₃₋₁₁ cycloalkene" defined above.

The "C₃₋₁₁ cycloalkenylene group" means a divalent substituent formed by removing two hydrogen atoms at any positions on different carbons of the "C₃₋₁₁ cycloalkene" defined above.

The "C₂₋₆ alkenyl group" means a linear or branched alkenyl group having at least one double bond and a carbon atom number of 2 to 6, and specific examples include ethenyl (vinyl) group, 1-propenyl group, 2-propenyl (allyl) group, isopropenyl group, 1-butenyl group, 2-butenyl group, 3-butenyl (homoallyl) group, 4-pentenyl group, and 5-hexenyl group.

The "C₂₋₆ alkenylene group" means a divalent substituent formed by removing a single hydrogen atom at any position of the "C₂₋₆ alkenyl group" defined above, and specific examples include ethene-1,1-diyl group, ethene-1,2-diyl group, propene-1,1-diyl group, propene-1,2-diyl group, propene-1,3-diyl group, but-1-ene-1,4-diyl group, but-1-ene-1,3-diyl group, but-2-ene-1,4-diyl group, buta-1,3-diene-1,4-diyl group, pent-2-ene-1,5-diyl group, hex-3-ene-1,6-diyl group, and hexa-2,4-diene-1,6-diyl group.

The "C₂₋₆ haloalkenyl group" means a substituent formed by substituting one or more halogen atoms independently selected from a substituent group consisting of a fluorine atom, a chlorine atom, a bromine atom, and an iodine atom for one or more hydrogen atoms at any positions of the "C₂₋₆ alkenyl group" defined above.

The "C₂₋₆ alkynyl group" means a linear or branched alkynyl group having at least one triple bond and a carbon atom number of 2 to 6, and specific examples include ethynyl group, 1-propynyl group, 2-propynyl group, 1-butyny group, 2-butyny group, 3-butyny group, 4-pentynyl group, and 5-hexynyl group.

The "C₂₋₆ alkynylene group" means a divalent substituent formed by removing a single hydrogen atom at any position of the "C₂₋₆ alkynyl group" defined above. Specific examples of the group include ethyne-1,2-diyl group, propyne-1,3-diyl group, but-1-yne-1,4-diyl group, but-1-yne-1,3-diyl group, but-2-yne-1,4-diyl group, pent-2-yne-1,5-diyl group, pent-2-yne-1,4-diyl group, and hex-3-yne-1,6-diyl group.

The "C₁₋₆ alkoxy group" means a linear or branched alkoxy group having a carbon atom number of 1 to 6, and specific examples include methoxy group, ethoxy group, n-propoxy group, isopropoxy group, n-butoxy group, isobutoxy group, t-butoxy group, n-pentyloxy group, and n-hexyloxy group.

The "C₁₋₁₀ alkoxy group" means a linear or branched alkoxy group having a carbon atom number of 1 to 10, and specific examples include methoxy group, ethoxy group, n-propoxy group, isopropoxy group, n-butoxy group, isobutoxy group, t-butoxy group, n-pentyloxy group, n-hexyloxy group, n-heptyloxy group, n-octyloxy group, n-nonyloxy group, and n-decyloxy group.

The "C₃₋₆ cycloalkoxy group" means a group formed by bonding a single "C₃₋₆ cycloalkyl group" to an oxy group, and specific examples include cyclopropoxy group, cyclobutoxy group, cyclopentyloxy group, and cyclohexyloxy group.

The "C₁₋₃ alkoxy group" means methoxy group, ethoxy group, n-propoxy group, or isopropoxy group.

The "C₁₋₆ haloalkoxy group" means a substituent formed by substituting one or more halogen atoms independently selected from a substituent group consisting of a fluorine atom, a chlorine atom, a bromine atom, and an iodine atom for one or more hydrogen atoms at any positions of the "C₁₋₆ alkoxy group" defined above, and specific examples include trifluoromethoxy group, pentafluoroethoxy group, 2,2,2-trifluoro-1,1-dimethyl-ethoxy group, 3-chloro-n-propyloxy group, and 4-chloro-n-butoxy group.

The "C₁₋₃ haloalkoxy group" means a substituent formed by substituting one or more halogen atoms independently selected from a substituent group consisting of a fluorine atom, a chlorine atom, a bromine atom, and an iodine atom for one or more hydrogen atoms at any positions of the "C₁₋₃ alkoxy group" defined above, and specific examples include trifluoromethoxy group, 2,2,2-trifluoroethoxy group, pentafluoroethoxy group, and 3-chloro-n-propyloxy group.

The "C₆₋₁₄ aromatic hydrocarbon ring" means a monocyclic or polycyclic aromatic hydrocarbon ring in which all the atoms constituting the ring are carbon atoms and the number of carbon atoms is 6 to 14, and includes bicyclic condensed rings composed of a monocyclic aromatic hydrocarbon ring and a monocyclic cycloalkanes or cycloalkenes. Specific examples of the ring include benzene, pentalene, naphthalene, azulene, anthracene, phenanthrene, indene, indane, dihydronaphthalene, and tetrahydronaphthalene.

The "C₆₋₁₄ aryl group" means a monovalent substituent formed by removing a single hydrogen atom at any position on the aromatic ring of the "C₆₋₁₄ aromatic hydrocarbon ring" defined above.

The "C₆₋₁₄ arylene group" means a divalent substituent formed by removing two hydrogen atoms at any positions on the aromatic ring of the "C₆₋₁₄ aromatic hydrocarbon ring" defined above.

The "5 to 10-membered aromatic heterocycle" means a monocyclic or condensed aromatic heterocycle having a ring-constituting atom number of 5 to 10 and containing 1 to 5 hetero atoms as the atoms constituting the ring (the hetero atom means a nitrogen atom, an oxygen atom, or a sulfur atom), and specific examples include furan, thiophene, pyrrole, imidazole, triazole, tetrazole, thiazole, pyrazole, oxazole, isoxazole, isothiazole, thiadiazole, oxadiazole, pyridine, pyrazine, pyridazine, pyrimidine, triazine, purine, pteridine, quinoline, isoquinoline, naphthyridine, quinoxaline, cinnoline, quinazoline, phthalazine, imidazopyridine, imidazothiazole, imidazooxazole, benzothiazole, benzoxazole, benzimidazole, indole, isoindole, indazole, pyrrolopyridine, thienopyridine, furopyridine, benzothiadiazole, benzooxadiazole, pyridopyrimidine, benzofuran, benzothiophene, and thienofuran.

When having a C=N double bond, the "5 to 10-membered aromatic heterocycle" includes N-oxides of the aromatic heterocycles.

The "5 to 10-membered aromatic heterocycle containing NH" means an aromatic heterocycle having NH among the "5 to 10-membered aromatic heterocycles" defined above, and specific examples include pyrrole, imidazole, triazole, tetrazole, pyrazole, purine, benzimidazole, and pyrrolopyridine.

The "5 to 10-membered heteroaryl group" means a monovalent substituent formed by removing a single hydrogen atom at any position of the "5 to 10-membered aromatic heterocycle" defined above.

The "5 to 10-membered heteroaryl group containing NH" means a heteroaryl group having NH among the "5 to 10-membered heteroaryl groups" defined above.

The "5 to 10-membered heteroarylene group" means a divalent substituent formed by removing two hydrogen atoms at any positions of the "5 to 10-membered aromatic heterocycle" defined above.

The "5 to 10-membered heteroarylene group containing NH" means a heteroarylene group having NH among the "5 to 10-membered heteroarylene groups" defined above.

The "5 to 6-membered aromatic heterocycle" means a monocyclic aromatic heterocycle having a ring-constituting atom number of 5 to 6 among the "5 to 10-membered aromatic heterocycles" defined above, and specific examples include pyrrole, pyrazole, imidazole, triazole, tetrazole, pyridine, pyridazine, pyrimidine, pyrazine, triazine, furan, thiophene, thiazole, isothiazole, oxazole, isoxazole, oxadiazole, and thiadiazole.

The "5 to 6-membered heteroaryl group" means a monovalent substituent formed by removing a single hydrogen atom at any position of the "5 to 6-membered aromatic heterocycle" defined above.

The "3 to 13-membered non-aromatic heterocycle" means a monocyclic non-aromatic heterocycle that
1) has a ring-constituting atom number of 3 to 13,
2) contains 1 to 5 hetero atoms as the atoms constituting the ring (the hetero atom means a nitrogen atom, an oxygen atom, or a sulfur atom),
3) may contain a carbonyl group, a thiocarbonyl group, a double bond, or a triple bond, and
4) in the case where a sulfur atom is contained as the atom constituting the ring, the sulfur atom may have optionally replaced by a sulfinyl group or a sulfonyl group, and
5) means a monocyclic-non-aromatic heterocycle, a condensed-non-aromatic heterocycle (in a condensed non-aromatic heterocycle, a non-aromatic ring may be condensed with a non-aromatic ring or an aromatic ring), bridged-non-aromatic heterocycle, or spiro-system non-aromatic heterocycles, and
   specific examples include aziridine, azetidine, pyrrolidine, piperidine, azepane, azocane, tetrahydrofuran, tetrahydropyran, morpholine, thiomorpholine, piperazine, thiazolidine, 1,4-dioxane, imidazoline, thiazoline, 1,2-benzopyran, isochroman, chroman, indoline, isoindoline, indazoline, azaindan, azatetrahydronaphthalene, azachroman, tetrahydrobenzofuran, tetrahydrobenzothiophene, 1,2,3,4-tetrahydroisoquinoline, 1,2,3,4-tetrahydroquinoline 3,4-dihydro-2H-benzo[b][1,4]dioxepine, indan-1-one, 6,7-dihydro-5H-cyclopentapyrazine, 6,7-dihydro-5H-cyclopenta[b]pyridine, 5,6-dihydro-4H-cyclopenta[b]thiophene, 4,5,6,7-tetrahydro-benzo[b]thiophene, 2,3-dihydro-isoindol-1-one, 3,4-dihydro-2H-isoquinolin-1-one, 3,4-dihydro-2H-benzo[b]oxepin-5-one, pyridone, 1-H-benzo[d]imidazol-2(3H)-thione, 2,3,4,5-tetrahydro-1H-pyrido[3,4-b]indole, 2,3,4,9-tetrahydro-1H-pyrido[3,4-b]indole, 2,3,4,5-tetrahydro-1H-pyrrolo[3,4-c]quinoline, 2,3,4,5-tetrahydro-1H-pyrrolo[2,3-c]quinoline, tetrahydro-β-carboline, tetrahydro-γ-carboline, and benzo[d]oxazol-2(3H)-one.

The "3 to 13-membered non-aromatic heterocycle containing NH" means a non-aromatic heterocycle having NH among the "3 to 13-membered non-aromatic heterocycles" defined above, and specific examples include aziridine, azetidine, pyrrolidine, piperidine, piperazine, morpholine, thiomorpholine, thiazolidine, imidazoline, thiazoline, indoline, isoindoline, indazoline, 1,2,3,4-tetrahydroisoquinoline, 1,2,3,4-tetrahydroquinoline, 6,7-dihydro-5H-cyclopenta[b]pyridine, 2,3-dihydro-isoindol-1-one, 3,4-dihydro-2H-isoquinolin-1-one, pyridone, 1-H-benzo[d]imidazol-2(3H)-thione, 2,3,4,5-tetrahydro-1H-pyrido[3,4-b]indole, 2,3,4,9-tetrahydro-1H-pyrido[3,4-b]indole, 2,3,4,5-tetrahydro-1H-pyrroto[3,4-c]quinoline, 2,3,4,5-tetrahydro-1H-pyrrolo[2,3-c]quinoline, tetrahydro-β-carboline, tetrahydro-γ-carboline, and benzo[d]oxazole-2(3H)-one.

The "3 to 13-membered heterocyclyl group" means a monovalent substituent formed by removing a single hydrogen atom at any position of the "3 to 13-membered non-aromatic heterocycle" defined above. In the case of the condensed ring including an aromatic ring, the condensed ring is substituted at the non-aromatic ring side.

The "3 to 13-membered heterocyclyl group containing NH" means a heterocyclyl group having NH among the "3 to 13-membered heterocyclyl groups" defined above. In the case of the condensed ring including an aromatic ring, the hydrogen is substituted at the non-aromatic ring side.

The "3 to 13-membered heterocyclylene group" means a divalent substituent formed by removing two hydrogen atoms at any positions on different atoms of the "3 to 13-membered non-aromatic heterocycle" defined above. In the case of the condensed ring including an aromatic ring, at least one hydrogen atom is substituted at the non-aromatic ring side.

The "3 to 13-membered heterocyclylene group containing NH" means a heterocyclylene group having NH among the "3 to 13-membered heterocyclylene groups" defined above. In the case of the condensed ring including an aromatic ring, at least one hydrogen atom is substituted at the non-aromatic ring side.

The "4 to 7-membered non-aromatic heterocycle" means a monocyclic non-aromatic heterocycle that
1) has a ring-constituting atom number of 4 to 7,
2) contains 1 to 3 hetero atoms as the atoms constituting the ring (the hetero atom means a nitrogen atom, an oxygen atom, or a sulfur atom),
3) may contain a carbonyl group, a double bond, or a triple bond, and
4) in the case where a sulfur atom is contained as the atom constituting the ring, the sulfur atom may be optionally replaced by a sulfinyl group or a sulfonyl group, and specific examples include azetidine, pyrrolidine, pyrrolidinone, oxazolidine, isoxazolidine, thiazolidine, isothiazolidine, piperazine, piperazinone, piperidine, piperidinone, morpholine, thiomorpholine, oxetane, tetrahydrofuran, 1,3-dioxolane, tetrahydropyran, 1,4-dioxane, oxepane, and homomorpholine.

The "4 to 7-membered heterocyclyl group" means a monovalent substituent formed by removing a single hydrogen atom at any position of the "4 to 7-membered non-aromatic heterocycle" defined above.

The "4 to 7-membered heterocyclylene group" means a divalent substituent formed by removing two hydrogen atoms at any positions on different atoms of the "4 to 7-membered non-aromatic heterocycle" defined above.

The "6-membered non-aromatic heterocycle" means a monocyclic non-aromatic heterocycle that
1) has a ring-constituting atom number of 6,
2) contains 1 to 3 hetero atoms as the atoms constituting the ring (the hetero atom means a nitrogen atom, an oxygen atom, or a sulfur atom),
3) may contain a carbonyl group, a double bond, or a triple bond, and
4) in the case where a sulfur atom is contained as the atom constituting the ring, the sulfur atom may be optionally replaced by a sulfinyl group or a sulfonyl group, and specific examples include piperazine, piperazinone, piperidine, piperidinone, morpholine, thiomorpholine, and 1,4-dioxane.

The "6-membered heterocyclyl group" means a monovalent substituent formed by removing a single hydrogen atom at any position of the "6-membered non-aromatic heterocycle" defined above.

The "6-membered heterocyclylene group" means a divalent substituent formed by removing two hydrogen atoms at any positions on different atoms of the "6-membered non-aromatic heterocycle" defined above.

The "C₁₋₆ alkylthio group" means a group formed by bonding the single "C₁₋₆ alkyl group" to -S-, and specific examples include methylthio group, ethylthio group, n-propylthio group, isopropylthio group, n-butylthio group, isobutylthio group, t-butylthio group, n-pentylthio group, and n-hexylthio group.

The "C₃₋₆ cycloalkylthio group" means a group formed by bonding the single "C₃₋₆ cycloalkyl group" to -S-, and specific examples include cyclopropylthio group, cyclobutylthio group, cyclopentylthio group, and cyclohexylthio group.

The "C₁₋₆ haloalkylthio group" means a substituent formed by substituting one or more halogen atoms independently selected from a substituent group consisting of a fluorine atom, a chlorine atom, a bromine atom, and an iodine atom for one or more hydrogen atoms at any positions of the "C₁₋₆ alkylthio group" defined above.

The "C₁₋₆ alkylsulfonyl group" means a group formed by bonding the single "C₁₋₆ alkyl group" to a sulfonyl group, and specific examples include methylsulfonyl group, ethylsulfonyl group, n-propylsulfonyl group, isopropylsulfonyl group, n-butylsulfonyl group, isobutylsulfonyl group, t-butylsulfonyl group, n-pentylsulfonyl group, and n-hexylsulfonyl group.

The "C₁₋₆ alkylsulfonylamino group" means a group formed by bonding the single "C₁₋₆ alkylsulfonyl group" to an amino group, and specific examples include methylsulfonylamino group, ethylsulfonylamino group, n-propylsulfonylamino group, isopropylsulfonylamino group, n-butylsulfonylamino group, isobutylsulfonylamino group, t-butylsulfonylamino group, n-pentylsulfonylamino group, and n-hexylsulfonylamino group.

The "C₁₋₆ alkylsulfonyloxy group" means a group formed by bonding the single "C₁₋₆ alkylsulfonyl group" to an oxy group, and specific examples include methylsulfonyloxy group, ethylsulfonyloxy group, n-propylsulfonyloxy group, isopropylsulfonyloxy group, n-butylsulfonyloxy group, isobutylsulfonyloxy group, t-butylsulfonyloxy group, n-pentylsulfonyloxy group, and n-hexylsulfonyloxy group.

The "C₃₋₆ cycloalkylsulfonyl group" means a group formed by bonding the single "C₃₋₆ cycloalkyl group" to a sulfonyl group, and specific examples include cyclopropylsulfonyl group, cyclobutylsulfonyl group, cyclopentylsulfonyl group, and cyclohexylsulfonyl group.

The "C₃₋₆ cycloalkylsulfonylamino group" means a group formed by bonding the single "C₃₋₆ cycloalkylsulfonyl group" to an amino group, and specific examples include cyclopropylsulfonylamino group, cyclobutylsulfonylamino group, cyclopentylsulfonylamino group, and cyclohexylsulfonylamino group.

The "C₃₋₆ cycloalkylsulfonyloxy group" means a group formed by bonding the single "C₃₋₆ cycloalkylsulfonyl group" to an oxy group, and specific examples include cyclopropylsulfonyloxy group, cyclobutylsulfonyloxy group, cyclopentylsulfonyloxy group, and cyclohexylsulfonyloxy group.

The "C₁₋₆ haloalkylsulfonyl group" means a substituent formed by substituting one or more halogen atoms independently selected from a substituent group consisting of a fluorine atom, a chlorine atom, a bromine atom, and an iodine atom for one or more hydrogen atoms at any positions of the "C₁₋₆ alkylsulfonyl group" defined above.

The "C₁₋₆ alkoxycarbonyl group" means a group formed by bonding the single "C₁₋₆ alkoxy group" to a carbonyl group, and specific examples include methoxycarbonyl group, ethoxycarbonyl group, n-propoxycarbonyl group, isopropoxycarbonyl group, n-butoxycarbonyl group, isobutoxycarbonyl group, t-butoxycarbonyl group, n-pentyloxycarbonyl group, and n-hexyloxycarbonyl group.

The "mono-C₁₋₆ alkylamino group" means a group formed by bonding the single "C₁₋₆ alkyl group" to an amino group, and specific examples include methylamino group, ethylamino group, n-propylamino group, isopropylamino group, n-butylamino group, isobutylamino group, t-butylamino group, n-pentylamino group, and n-hexylamino group.

The "di-C₁₋₆ alkylamino group" means a group formed by bonding the two "C₁₋₆ alkyl groups", which may be the same as or different from each other, to an amino group, and specific examples include dimethylamino group, diethylamino group, di-n-propylamino group, diisopropylamino group, di-n-butylamino group, diisobutylamino group, di-t-butylamino group, di-n-pentylamino group, di-n-hexylamino group, N-ethyl-N-methylamino group, N-methyl-N-n-propylamino group, N-isopropyl-N-methylamino group, N-n-butyl-N-methylamino group, N-isobutyl-N-methylamino group, N-t-butyl-N-methylamino group, N-methyl-N-n-pentylamino group, N-n-hexyl-N-methylamino group, N-ethyl-N-n-propylamino group, N-ethyl-N-isopropylamino group, N-n-butyl-N-ethylamino group, N-ethyl-N-isobutylamino group, N-t-butyl-N-ethylamino group, N-ethyl-N-n-pentylamino group, and N-ethyl-N-n-hexylamino group.

The "mono-C₃₋₆ cycloalkylamino group" means a group formed by bonding the single "C₃₋₆ cycloalkyl group" to an amino group, and specific examples include cyclopropylamino group, cyclobutylamino group, cyclopentylamino group, and cyclohexylamino group.

The "di-C₃₋₆ cycloalkylamino group" means a group formed by bonding the two "C₃₋₆ cycloalkyl groups", which may be the same as or different from each other, to an amino group, and specific examples include dicyclopropylamino group, dicyclobutylamino group, dicyclopentylamino group, and dicyclohexylamino group.

The "C₁₋₆ alkylcarbonyl group" means a group formed by bonding the single "C₁₋₆ alkyl group" to a carbonyl group, and specific examples include acetyl group, propionyl group, butyryl group, isobutyryl group, pentanoyl group, 3-methylbutanoyl group, pivaloyl group, hexanoyl group, and heptanoyl group.

The "C₃₋₆ cycloalkylcarbonyl group" means a group formed by bonding the single "C₃₋₆ cycloalkyl group" to a carbonyl group, and specific examples include cyclopropylcarbonyl group, cyclobutylcarbonyl group, cyclopentylcarbonyl group, cyclohexylcarbonyl group, and cycloheptylcarbonyl group.

The "C₁₋₆ haloalkylcarbonyl group" means a substituent formed by substituting one or more halogen atoms independently selected from a substituent group consisting of a fluorine atom, a chlorine atom, a bromine atom, and an iodine atom for one or more hydrogen atoms at any positions of the "C₁₋₆ alkylcarbonyl group" defined above.

The "mono-C₁₋₆ alkylaminocarbonyl group" means a group formed by bonding the single "mono-C₁₋₆ alkylamino group" to a carbonyl group, and specific examples include methylaminocarbonyl group, ethylaminocarbonyl group, n-propylaminocarbonyl group, isopropylaminocarbonyl group, n-butylaminocarbonyl group, isobutylaminocarbonyl group, t-butylaminocarbonyl group, n-pentylaminocarbonyl group, and n-hexylaminocarbonyl group.

The "di-C₁₋₆ alkylaminocarbonyl group" means a group formed by bonding the single "di-C₁₋₆ alkylamino group" to a carbonyl group, and specific examples include dimethylaminocarbonyl group, diethylaminocarbonyl group, di-n-propylaminocarbonyl group, diisopropylaminocarbonyl group, di-n-butylaminocarbonyl group, diisobutylaminocarbonyl group, di-t-butylaminocarbonyl group, di-n-pentylaminocarbonyl group, di-n-hexylaminocarbonyl group, N-ethyl-N-methylaminocarbonyl group, N-methyl-N-n-propylaminocarbonyl group, N-isopropyl-N-methylaminocarbonyl group, N-n-butyl-N-methylaminocarbonyl group, N-isobutyl-N-methylaminocarbonyl group, N-t-butyl-N-methylaminocarbonyl group, N-methyl-N-n-pentylaminocarbonyl group, N-n-hexyl-N-methylaminocarbonyl group, N-ethyl-N-n-propylaminocarbonyl group, N-ethyl-N-isopropylaminocarbonyl group, N-n-butyl-N-ethylaminocarbonyl group, N-ethyl-N-isobutylaminocarbonyl group, N-t-butyl-N-ethylaminocarbonyl group, N-ethyl-N-n-pentylaminocarbonyl group, and N-ethyl-N-n-hexylaminocarbonyl group.

The "C₁₋₆ alkylcarbonylamino group" means a group formed by bonding the single "C₁₋₆ alkylcarbonyl group" to an amino group, and specific examples include methylcarbonylamino group, ethylcarbonylamino group, n-propylcarbonylamino group, isopropylcarbonylamino group, n-butylcarbonylamino group, isobutylcarbonylamino group, t-butylcarbonylamino group, n-pentylcarbonylamino group, and n-hexylcarbonylamino group.

The "C₁₋₆ alkylcarbonyloxy group" means a group formed by bonding the single "C₁₋₆ alkylcarbonyl group" to an oxy group, and specific examples include methylcarbonyloxy group, ethylcarbonyloxy group, n-propylcarbonyloxy group, isopropylcarbonyloxy group, n-butylcarbonyloxy group, isobutylcarbonyloxy group, t-butylcarbonyloxy group, n-pentylcarbonyloxy group, and n-hexylcarbonyloxy group.

The "mono-C₁₋₆ alkylaminosulfonyl group" means a group formed by bonding the single "mono-C₁₋₆ alkylamino group" to a sulfonyl group, and specific examples include methylaminosulfonyl group, ethylaminosulfonyl group, n-propylaminosulfonyl group, isopropylaminosulfonyl group, n-butylaminosulfonyl group, isobutylaminosulfonyl group, t-butylaminosulfonyl group, n-pentylaminosulfonyl group, and n-hexylaminosulfonyl group.

The "di-C₁₋₆ alkylaminosulfonyl group" means a group formed by bonding the single "di-C₁₋₆ alkylamino group" to a sulfonyl group, and specific examples include dimethylaminosulfonyl group, diethylaminosulfonyl group, di-n-propylaminosulfonyl group, diisopropylaminosulfonyl group, di-n-butylaminosulfonyl group, diisobutylaminosulfonyl group, di-t-butylaminosulfonyl group, di-n-pentylaminosulfonyl group, di-n-hexylaminosulfonyl group, N-ethyl-N-methylaminosulfonyl group, N-methyl-N-n-propylaminosulfonyl group, N-isopropyl-N-methylaminosulfonyl group, N-n-butyl-N-methylaminosulfonyl group, N-isobutyl-N-methylaminosulfonyl group, N-t-butyl-N-methylaminosulfonyl group, N-methyl-N-n-pentylaminosulfonyl group, N-n-hexyl-N-methylaminosulfonyl group, N-ethyl-N-n-propylaminosulfonyl group, N-ethyl-N-isopropylaminosulfonyl group, N-n-butyl-N-ethylaminosulfonyl group, N-ethyl-N-isobutylaminosulfonyl group, N-t-butyl-N-ethylaminosulfonyl group, N-ethyl-N-n-pentylaminosulfonyl group, and N-ethyl-N-n-hexylaminosulfonyl group.

The "C₆₋₁₄ arylcarbonyl group" means a group formed by bonding the single "C₆₋₁₄ aryl group" to a carbonyl group, and specific examples include phenylcarbonyl group, 1-naphthylcarbonyl group, and 2-naphthylcarbonyl group.

The "C₆₋₁₄ arylcarbonyloxy group" means a group formed by bonding the single "C₆₋₁₄ arylcarbonyl group" to an oxy group, and specific examples include phenylcarbonyloxy group, 1-naphthylcarbonyloxy group, and 2-naphthylcarbonyloxy group.

The "C₆₋₁₄ aryloxy group" means a group formed by bonding the single "C₆₋₁₄ aryl group" to an oxy group, and specific examples include phenoxy group, 1-naphthyloxy group, and 2-naphthyloxy group.

The binding form of B in the present invention will be described. In the present invention, as clearly shown in Formula (I), the formula of B means that B bonds to L¹ at the left side and bonds to L² at the right side on the page space in the formula of B.

Preferred structures for each substituent in the present invention will be described next.

The substituent R⁴ is preferably a hydrogen atom, a halogen atom, a nitro group, a C₁₋₆ alkyl group, a C₁₋₆ alkoxy group, a mono-C₁₋₆ alkylamino group, or a di-C₁₋₆ alkyl amino group (the C₁₋₆ alkyl group, the C₁₋₆ alkoxy group, the mono-C₁₋₆ alkylamino group, and the di-C₁₋₆ alkylamino group are unsubstituted or substituted with one or more substituents independently selected from a substituent group V⁷).

Here, the substituent group V⁷ means a substituent group consisting of a hydroxy group, halogen atoms, a cyano group, a nitro group, C₁₋₆ alkoxy groups, C₁₋₆ alkylthio groups, C₁₋₆ alkoxycarbonyl groups, C₁₋₆ alkylcarbonyloxy groups, C₁₋₆ alkylsulfonylamino groups, C₁₋₆ alkylsulfonyloxy groups (the C₁₋₆ alkoxy groups, the C₁₋₆ alkylthio groups, the C₁₋₆ alkoxycarbonyl groups, the C₁₋₆ alkylcarbonyloxy groups, the C₁₋₆ alkylsulfonylamino groups, and the C₁₋₆ alkylsulfonyloxy groups are unsubstituted or substituted with one or more substituents independently selected from the substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents independently selected from the substituent group V²).

The substituent R⁴ is more preferably a hydrogen atom, a halogen atom, a nitro group, or a C₁₋₆ alkyl group (the C₁₋₆ alkyl group is unsubstituted or substituted with one or more substituents independently selected from the substituent group V³);

The substituent R⁴ is further more preferably a hydrogen atom.

A is preferably a C₆₋₁₄ aryl group or a 5 to 10-membered heteroaryl group (the C₆₋₁₄ aryl group and the 5 to 10-membered heteroaryl group are unsubstituted or substituted with one or more substituents independently selected from the substituent group V⁵).

A is more preferably a phenyl group or a 5 to 6-membered heteroaryl group (the phenyl group and the 5 to 6-membered heteroaryl group are unsubstituted or substituted with one or more substituents independently selected from the substituent group V⁴).

A is further more preferably any one of Formulae (IV-1) to (IV-3) illustrated in (IV): 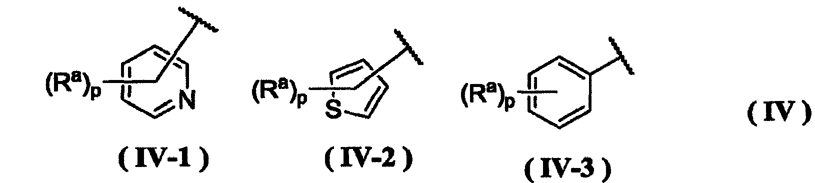 (wherein p is 1 to 3; R^{a} means a substituent selected from the substituent group V⁴; and when p is 2 or 3, R^{a}s may be the same as or different from each other).

A is particularly preferably any one of Formulae (IV-1) to (IV-3) illustrated in (IV): 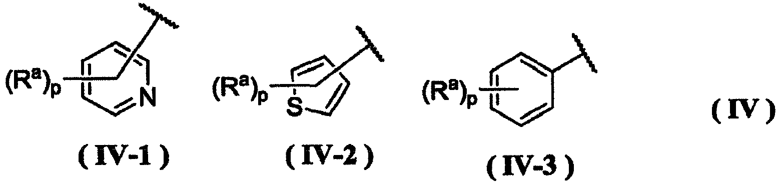 (wherein p is 1; R^{a} means a halogen atom, a nitro group, a C₁₋₆ alkyl group, a C₁₋₆ haloalkyl group, a C₁₋₆ alkoxy group, or a C₁₋₆ haloalkoxy group).

As another preferable structure, A is either Formula (VII-1) or Formula (VII-2) illustrated in (VII): 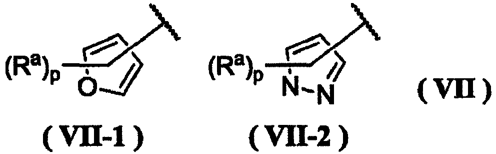 (wherein p is 1; R^{a} means a halogen atom, a nitro group, a C₁₋₆ alkyl group, a C₃₋₆ haloalkyl group, a C₁₋₆ alkoxy group, or a C₁₋₆ haloalkoxy group).

As another preferable structure,
A is preferably a hydrogen atom.

Subsequently, the preferable structures of the combination of L¹, L², A, and B will be described.

The preferable combination of L¹, L², A, and B is:
that L¹ and L² are single bonds;
that A is a phenyl group or a 5 to 6-membered heteroaryl group (the phenyl group and the 5 to 6-membered heteroaryl group are unsubstituted or substituted with one or more substituents independently selected from the substituent group V⁵); and
and that B is a 4 to 7-membered heterocyclylene group (the 4 to 7-membered heterocyclylene group is unsubstituted or substituted with one or more substituents independently selected from the substituent group V⁵ and further a single methylene group in the 4 to 7-membered heterocyclylene group is optionally replaced by a 1,1-C₃₋₇ cycloalkylene group).

The more preferable combination of L¹, L², A, and B is:
that L¹ and L² are single bonds;
that A is a phenyl group or a 5 to 6-membered heteroaryl group (the phenyl group and the 5 to 6-membered heteroaryl group are unsubstituted or substituted with one or more substituents independently selected from the substituent group V⁴); and
that B is a 6-membered heterocyclylene group (the 6-membered heterocyclylene group is unsubstituted or substituted with one or more substituents independently selected from the substituent group V³).

The more preferable combination of L¹, L², A, and B is:
that L¹ and L² are single bonds;
that A is a phenyl group or a 5 to 6-membered heteroaryl group (the phenyl group and the 5 to 6-membered heteroaryl group are unsubstituted or substituted with one or more substituents independently selected from the substituent group V⁴); and
that B is any one of Formulae (III-1) to (III-3) illustrated in (III): 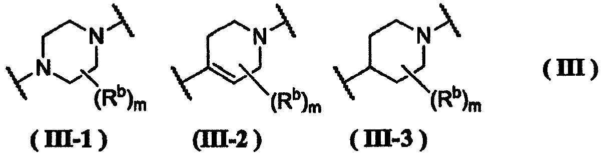
(wherein m is 0 or 1; and R^{b} means a hydroxy group, a halogen atom, an amino group, a C₁₋₆ alkyl group, or a C₁₋₆ alkoxy group).

The further more preferable combination of L¹, L², A, and B is:
that L¹ and L² are single bonds;
that A is a phenyl group or a 5 to 6-membered heteroaryl group (the phenyl group and the 5 to 6-membered heteroaryl group are unsubstituted or substituted with one or more substituents independently selected from the substituent group V⁴); and
that B is any one of Formulae (III-1) to (III-3) illustrated in (III): 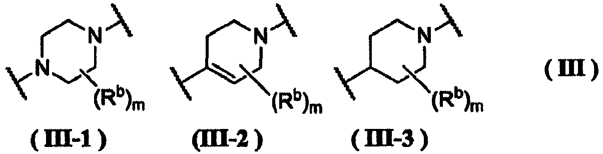
(wherein m is 0 or 1; and R^{b} means a hydroxy group, a halogen atom, or an amino group).

As another preferable structure,
the preferable combination of L¹, L², A, and B is:
that L¹ and L² are single bonds; that A is a hydrogen atom; and that B is a tetrahydroisoquinoline-diyl group or a tetrahydrocarboline-diyl group (the tetrahydroisoquinoline-diyl group and the tetrahydrocarboline-diyl group are unsubstituted, or substituted with one or more substituents independently selected from the substituent group V³, and further a single methylene group in the tetrahydroisoquinoline-diyl group and the tetrahydrocarboline-diyl group is optionally replaced by a 1,1-C₃₋₇ cycloalkylene group).

The more preferable combination of L¹, L², A, and B is:
that L² is a single bond; and
thatA-L¹-B is a structure of either Formula (II-1) or Formula (II-2): 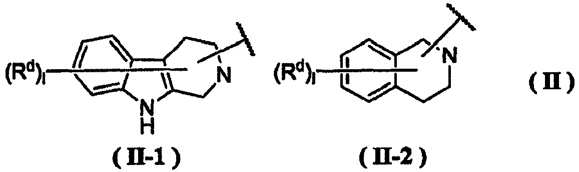
(wherein 1 is 1 to 3; R^{d} is optionally substituted for a tetrahydrocarboline ring or a tetrahydroisoquinoline ring at any positions; R^{d} means a hydroxy group, a halogen atom, an amino group, a C₁₋₆ alkyl group, a C₁₋₆ haloalkyl group, a C₁₋₆ alkoxy group, a C₁₋₆ haloalkoxy group, a mono-C₁₋₆ alkylamino group, a di-C₁₋₆ alkylamino group, or a C₁₋₆ alkylsulfonylamino group; and when 1 is 2 or 3, R^{d}s are the same as or different from each other).

The further preferable combination of L¹, L², A, and B is:
that L² is a single bond; and
that A-L¹-B is a structure of either Formula (II-1) or Formula (II-2): 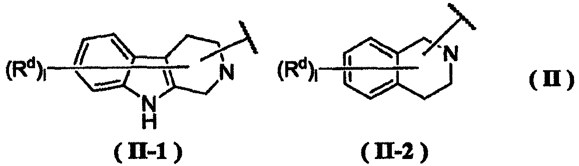
(wherein 1 is 1 or 2; R^{d} is optionally substituted for a tetrahydrocarboline ring or a tetrahydroisoquinoline ring at any positions; R^{d} means a hydroxy group, a halogen atom, a C₁₋₃ alkyl group, a C₁₋₃ haloalkyl group, a C₁₋₃ alkoxy group, or a C₁₋₃ haloalkoxy group; and when 1 is 2, R^{d}s are the same as or different from each other).

L³ is preferably a C₁₋₃ alkylene group (the C₁₋₃ alkylene group is unsubstituted or substituted with one or more substituents independently selected from the substituent group V³ and a single methylene group in the C₁₋₆ alkylene group is optionally replaced by C=O or C=S).

L³ is more preferably a C₁₋₃ alkylene group.

L³ is further more preferably a methylene group.

D is preferably a C₆₋₁₄ aryl group (the C₆₋₁₄ aryl group is unsubstituted or substituted with one or more substituents independently selected from the substituent group V⁵).

D is more preferably a phenyl group (the phenyl group is unsubstituted or substituted with one or more substituents independently selected from the substituent group V⁴).

D is further more preferably a phenyl group (the phenyl group has one or more substituents independently selected from the group consisting of halogen atoms, a nitro group, C₁₋₆ alkyl groups, and C₁₋₆ alkoxy groups (the C₁₋₆ alkyl groups and the C₁₋₆ alkoxy groups are unsubstituted or substituted with one or more substituents independently selected from the group consisting of halogen atoms, C₁₋₆ alkoxy groups, C₁₋₃ haloalkoxy groups, and a phenyl group (the phenyl group is unsubstituted or substituted with one or more halogen atoms))).

As another preferable structure,
D is preferably a 5 to 10-membered heteroaryl group (the 5 to 10-membered heteroaryl group is unsubstituted or substituted with one or more substituents independently selected from the substituent group V⁵).

D is more preferably a 5 to 6-membered heteroaryl group (the 5 to 6-membered heteroaryl group is unsubstituted or substituted with one or more substituents independently selected from the substituent group V⁴).

D is more preferably any one of Formulae (VIII-1) to (VIII-5) illustrated in (VIII): 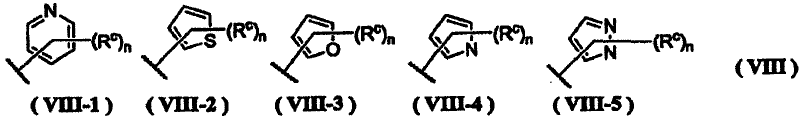 (wherein n is 1 to 3; R^{c} means a substituent selected from the substituent group V⁴; and when n is 2 or 3, R^{c}s are the same as or different from each other), or

D is either Formula (IX-1) or Formula (IX-2) illustrated in (IX): 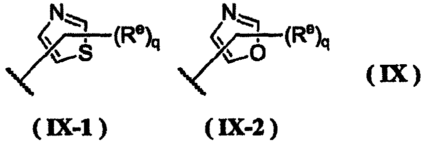 (wherein q is 1 to 2; R^{e} means a substituent selected from the substituent group V⁴; and when q is 2, R^{e}s are the same as or different from each other).

D is more preferably any one of Formulae (V-1) to (V-4) illustrated in (V): 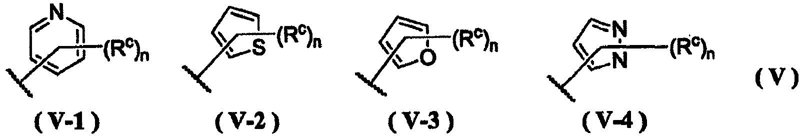 (wherein n is 1 to 3; R^{c} means a substituent selected from the substituent group V⁴; and when n is 2 or 3, R^{c}s are the same as or different from each other), or
Formula (VI-1) illustrated in (VI): 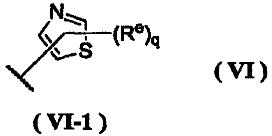 (wherein q is 1 to 2; R^{e} means a substituent selected from the substituent group V⁴; and when q is 2, R^{e}s are the same as or different from each other).

D is further more preferably any one of Formulae (IV-1) to (V-4) illustrated in (V): 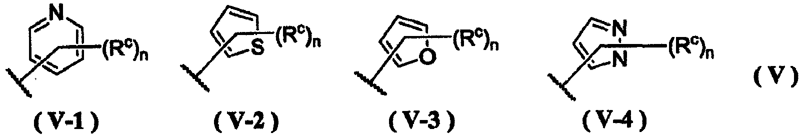 (wherein n is 1 to 3; R^{c} means a halogen atom, a nitro group, a C₁₋₆ alkyl group, a C₁₋₆ haloalkyl group, a C₁₋₆ alkoxy group, or a C₁₋₆ haloalkoxy group; and when n is 2 or 3, R^{c}s are the same as or different from each other), or
Formula (VI-1) illustrated in (VI): 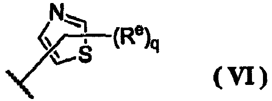 (wherein q is 1 to 2; R^{e} means a halogen atom, a nitro group, a C₁₋₆ alkyl group, a C₃₋₆ haloalkyl group, a C₁₋₆ alkoxy group, or a C₁₋₆ haloalkoxy group; and when q is 2, R^{e}s are the same as or different from each other).

D is particularly preferably Formula (V-2) illustrated in (V): 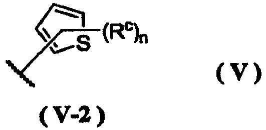 (wherein n is 1; R^{c} means a halogen atom, a C₁₋₆ alkyl group, a C₁₋₆ haloalkyl group, a C₁₋₆ alkoxy group, or a C₁₋₆ haloalkoxy group), or
Formula (VI-1) illustrated in (VI): 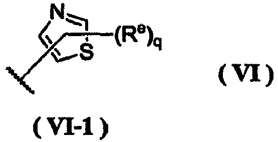 (wherein q is 1; R^{e} means a halogen atom, a C₁₋₆ alkyl group, a C₁₋₆ haloalkyl group, a C₁₋₆ alkoxy group, or a C₁₋₆ haloalkoxy group).

R² is preferably a hydrogen atom.

R³ is preferably a hydrogen atom.

Compounds preferably used for the T-type calcium channel inhibitor and for the preventive agent, the therapeutic agent, and/or the improving agent for a disease treatable by a T-type calcium channel inhibitory activity of the present invention are shown below.

1) A compound of Formula (I): 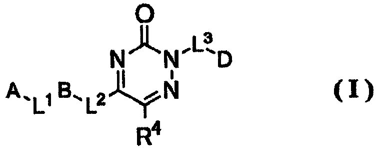 [wherein
R⁴ means a hydrogen atom, a halogen atom, a cyano group, a nitro group, a carboxy group, a carbamoyl group, a sulfamoyl group, a sulfo group, a tetrazolyl group, a C₁₋₆ alkyl group, a C₂₋₆ alkenyl group, a C₂₋₆ alkynyl group, a C₁₋₆ alkoxy group, a C₁₋₆ alkylthio group, a mono-C₁₋₆ alkyl amino group, or a di-C₁₋₆ alkyl amino group (the C₁₋₆ alkyl group, the C₂₋₆ alkenyl group, the C₂₋₆ alkynyl group, the C₁₋₆ alkoxy group, the C₁₋₆ alkylthio group, the mono-C₁₋₆ alkylamino group, and a di-C₁₋₆ alkylamino group are unsubstituted or substituted with one or more substituents independently selected from the substituent group V⁸);
L¹ and L² each independently mean a single bond, NR², O, S, SO, SO₂, or a C₁₋₆ alkylene group (the C₁₋₆ alkylene group is unsubstituted or substituted with one or more substituents independently selected from the substituent group V⁶ and a single methylene group in the C₁₋₆ alkylene group is optionally replaced by O, S, SO₂, C=O, C=S, or NR³);
B means a 3 to 13-membered heterocyclylene group (the 3 to 13-membered heterocyclylene group is unsubstituted or substituted with one or more substituents independently selected from the substituent group V⁶ and a single methylene group in the 3 to 13-membered heterocyclylene group is optionally replaced by a 1,1-C₃₋₇ cycloalkylene group);
A means a hydrogen atom, a C₃₋₁₁ cycloalkyl group, a C₃₋₁₁ cycloalkenyl group, a 3 to 13-membered heterocyclyl group, a C₆₋₁₄ aryl group, or a 5 to 10-membered heteroaryl group (the C₃₋₁₁ cycloalkyl group, the C₃₋₁₁ cycloalkenyl group, the 3 to 13-membered heterocyclyl group, the C₆₋₁₄ aryl group, and the 5 to 10-membered heteroaryl group are unsubstituted or substituted with one or more substituents independently selected from the substituent group V⁶);
L³ means a C₁₋₆ alkylene group (the C₁₋₆ alkylene group is unsubstituted or substituted with one or more substituents independently selected from the substituent group V⁸ and a single methylene group in the C₁₋₆ alkylene group is optionally replaced by C=O or C=S);
D means a 3 to 13-membered heterocyclyl group, a C₆₋₁₄ aryl group, or a 5 to 10-membered heteroaryl group (the 3 to 13-membered heterocyclyl group, the C₆₋₁₄ aryl group, and the 5 to 10-membered heteroaryl group are unsubstituted or substituted with one or more substituents independently selected from the substituent group V⁶);
R² and R³ each independently mean a hydrogen atom, a C₁₋₆ alkyl group, a C₂₋₆ alkenyl group, a C₂₋₆ alkynyl group (the C₁₋₆ alkyl group, the C₂₋₆ alkenyl group, and the C₂₋₆ alkynyl group are unsubstituted or substituted with one or more substituents independently selected from the substituent group V⁸), a C₃₋₁₁ cycloalkyl group, a 3 to 13-membered heterocyclyl group, a C₆₋₁₄ aryl group, or a 5 to 10-membered heteroaryl group (the C₃₋₁₁ cycloalkyl group, the 3 to 13-membered heterocyclyl group, the C₆₋₁₄ aryl group, and the 5 to 10-membered heteroaryl group are unsubstituted or substituted with one or more substituents independently selected from the substituent group V⁶)], a tautomer of the compound, a pharmaceutically acceptable salt thereof, or a solvate thereof.
2) The compound, the tautomer of the compound, the pharmaceutically acceptable salt thereof, or the solvate thereof according to 1), in which L³ is a C₁₋₃ alkylene group (the C₁₋₃ alkylene group is unsubstituted or substituted with one or more substituents independently selected from the substituent group V³).
3) The compound, the tautomer of the compound, the pharmaceutically acceptable salt thereof, or the solvate thereof according to 2), in which L³ is a C₁₋₃ alkylene group.
4) The compound, the tautomer of the compound, the pharmaceutically acceptable salt thereof, or the solvate thereof according to 3), in which L³ is a methylene group.
5) The compound, the tautomer of the compound, the pharmaceutically acceptable salt thereof, or the solvate thereof according to any one of 1) to 4), in which R⁴ is a hydrogen atom, a halogen atom, a nitro group, a alkoxy group, a mono-C₁₋₆ alkylamino group, a di-C₁₋₆ alkylamino group, or a C₁₋₆ alkyl group (the C₁₋₆ alkoxy group, the mono-C₁₋₆ alkylamino group, the di-C₁₋₆ alkylamino group, and the C₁₋₆ alkyl group are unsubstituted or substituted with one or more substituents independently selected from the substituent group V⁷).
6) The compound, the tautomer of the compound, the pharmaceutically acceptable salt thereof, or the solvate thereof according to 5), in which R⁴ is a hydrogen atom, a halogen atom, a nitro group, or a C₁₋₆ alkyl group (the C₁₋₆ alkyl group is unsubstituted or substituted with one or more substituents independently selected from the substituent group V³).
7) The compound, the tautomer of the compound, the pharmaceutically acceptable salt thereof, or the solvate thereof according to 6), in which R⁴ is a hydrogen atom.
8) The compound, the tautomer of the compound, the pharmaceutically acceptable salt thereof, or the solvate thereof according to any one of 1) to 7), in which D is a C₆₋₁₄ aryl group or a 5 to 10-membered heteroaryl group (the C₆₋₁₄ aryl group and the 5 to 10-membered heteroaryl group are unsubstituted or substituted with one or more substituents independently selected from the substituent group V⁵).
9) The compound, the tautomer of the compound, the pharmaceutically acceptable salt thereof, or the solvate thereof according to 8), in which D is a phenyl group (the phenyl group is unsubstituted or substituted with one or more substituents independently selected from the substituent group V⁴).
10) The compound, the tautomer of the compound, the pharmaceutically acceptable salt thereof, or the solvate thereof according to 9), in which D is a phenyl group (the phenyl group has one or more substituents independently selected from the group consisting of halogen atoms, a nitro group, C₁₋₆ alkyl groups, and C₁₋₆ alkoxy groups (the C₁₋₆ alkyl groups and the C₁₋₆ alkoxy groups are unsubstituted or substituted with one or more substituents independently selected from the group consisting of halogen atoms, C₁₋₆ alkoxy groups, C₁₋₃ haloalkoxy groups, and a phenyl group (the phenyl group is unsubstituted or substituted with one or more halogen atoms))).
11) The compound, the tautomer of the compound, the pharmaceutically acceptable salt thereof, or the solvate thereof according to 8), in which D is a 5 to 6-membered heteroaryl group (the 5 to 6-membered heteroaryl group is unsubstituted or substituted with one or more substituents independently selected from the substituent group V⁴).
12) The compound, the tautomer of the compound, the pharmaceutically acceptable salt thereof, or the solvate thereof according to 11), in which D is any one of Formulae (VIII-1) to (VIII-5) illustrated in (VIII); n is 0 to 3; R^{c} means a substituent selected from the substituent group V⁴; and when n is 2 or 3, R^{c}s may be the same as or different from each other. 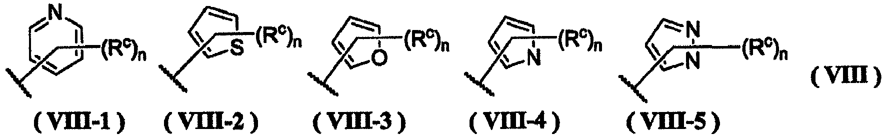
13) The compound, the tautomer of the compound, the pharmaceutically acceptable salt thereof, or the solvate thereof according to 12), in which n is 1; and R^{c} is a halogen atom, a C₁₋₆ alkyl group, a C₁₋₆ haloalkyl group, a C₁₋₆ alkoxy group, or a C₁₋₆ haloalkoxy group.
14) The compound, the tautomer of the compound, the pharmaceutically acceptable salt thereof, or the solvate thereof according to 11), in which D is any one of Formulae (V-1) to (V-4) illustrated in (V); n is 0 to 3; R^{c} means a substituent selected from the substituent group V⁴; and when n is 2 or 3, R^{c}s may be the same as or different from each other. 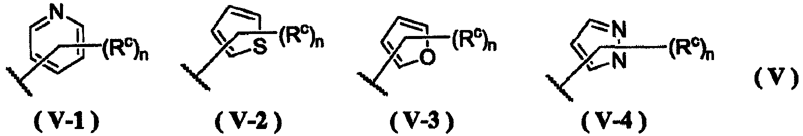
15) The compound, the tautomer of the compound, the pharmaceutically acceptable salt thereof, or the solvate thereof according to 14), in which n is 1 or 2; R^{c} means a halogen atom, a nitro group, a C₁₋₆ alkyl group, a C₁₋₆ haloalkyl group, a C₁₋₆ alkoxy group, or a C₁₋₆ haloalkoxy group; and when n is 2, R^{c}s may be the same as or different from each other.
16) The compound, the tautomer of the compound, the pharmaceutically acceptable salt thereof, or the solvate thereof according to 15), in which D is Formula (V-2) illustrated in (V); n is 1; and R^{c} is a halogen atom, a C₁₋₆ alkyl group, a C₁₋₆ haloalkyl group, a C₁₋₆ alkoxy group, or a C₁₋₆ haloalkoxy group. 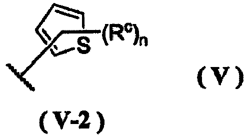
17) The compound, the tautomer of the compound, the pharmaceutically acceptable salt thereof, or the solvate thereof according to 16), in which R^{c} is a C₁₋₆ alkyl group or a C₁₋₆ haloalkyl group.
18) The compound, the tautomer of the compound, the pharmaceutically acceptable salt thereof, or the solvate thereof according to 17), in which R^{c} is a t-butyl group or a trifluoromethyl group.
19) The compound, the tautomer of the compound, the pharmaceutically acceptable salt thereof, or thea solvate thereof according to 11), in which D is either Formula (IX-1) or Formula (IX-2) illustrated in (IX); q is 0 to 2; R^{e} means a substituent selected from the substituent group V⁴; and when q is 2, R^{e}s may be the same as or different from each other. 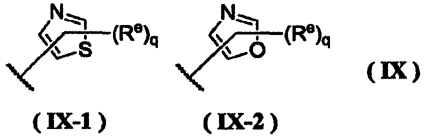
20) The compound, the tautomer of the compound, the pharmaceutically acceptable salt thereof, or the solvate thereof according to 19), in which q is 1; and R^{e} is a halogen atom, a C₁₋₆ alkyl group, a C₁₋₆ haloalkyl group, a C₁₋₆ alkoxy group, or a C₁₋₆ haloalkoxy group.
21) The compound, the tautomer of the compound, the pharmaceutically acceptable salt thereof, or the solvate thereof according to 11), in which D is Formula (VI-1) illustrated in (VI); q is 0 to 2; R^{e} means a substituent selected from the substituent group V⁴; and when q is 2, R^{e}s may be the same as or different from each other. 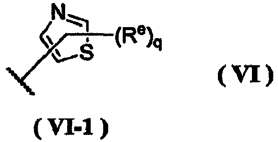
22) The compound, the tautomer of the compound, the pharmaceutically acceptable salt thereof, or the solvate thereof according to 21), in which q is 1 or 2; R^{e} means a halogen atom, a nitro group, a C₁₋₆ alkyl group, a C₁₋₆ haloalkyl group, a C₁₋₆ alkoxy group, or a C₁₋₆ haloalkoxy group; and when q is 2, R^{e}s may be the same as or different from each other.
23) The compound, the tautomer of the compound, the pharmaceutically acceptable salt thereof, or the solvate thereof according to 22), in which q is 1; and R^{e} is a C₁₋₆ alkyl group or a C₁₋₆ haloalkyl group.
24) The compound, the tautomer of the compound, the pharmaceutically acceptable salt thereof, or the solvate thereof according to 23), in which R^{e} is a trifluoromethyl group.
25) The compound, the tautomer of the compound, the pharmaceutically acceptable salt thereof, or the solvate thereof according to any one of 1) to 24), in which A is a C₆₋₁₄ aryl group or a 5 to 10-membered heteroaryl group (the C₆₋₁₄ aryl group and the 5 to 10-membered heteroaryl group are unsubstituted or substituted with one or more substituents independently selected from the substituent group V⁵).
26) The compound, the tautomer of the compound, the pharmaceutically acceptable salt thereof, or the solvate thereof according to 24), in which A is a phenyl group or a 5 to 6-membered heteroaryl group (the phenyl group and the 5 to 6-membered heteroaryl group are unsubstituted or substituted with one or more substituents independently selected from the substituent group V⁴).
27) The compound, the tautomer of the compound, the pharmaceutically acceptable salt thereof, or the solvate thereof according to 26), in which A is a phenyl group (the phenyl group has one or more substituents independently selected from the group consisting of halogen atoms, a nitro group, C₁₋₆ alkyl groups, C₁₋₆ haloalkyl groups, C₁₋₆ alkoxy groups, and C₁₋₆ haloalkoxy groups).
28) The compound, the tautomer of the compound, the pharmaceutically acceptable salt thereof, or the solvate thereof according to 26), in which A is a thienyl group, a pyridyl group, a pyrazolyl group, or a furanyl group (the thienyl group, the pyridyl group, the pyrazolyl group, or the furanyl group has one or more substituents independently selected from the group consisting of halogen atoms, C₁₋₆ alkyl groups, C₁₋₆ haloalkyl groups, C₁₋₆ alkoxy groups, and C₁₋₆ haloalkoxy groups).
29) The compound, the tautomer of the compound, the pharmaceutically acceptable salt thereof, or the solvate thereof according to 26), in which A is a thienyl group or a pyridyl group (the thienyl group and the pyridyl group have one or more substituents independently selected from the group consisting of halogen atoms, C₁₋₆ alkyl groups, C₁₋₆ haloalkyl groups, C₁₋₆ alkoxy groups, and C₁₋₆ haloalkoxy groups).
30) The compound, the tautomer of the compound, the pharmaceutically acceptable salt thereof, or the solvate thereof according to any one of 1) to 26), in which A is any one of Formulae (IV-1) to (IV-3) illustrated in (IV) (where p is 1 to 3; R^{a} means a substituent selected from the substituent group V⁴; and when p is 2 or 3, R^{a}s may be the same as or different from each other). 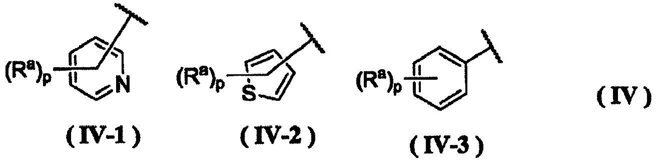
31) The compound, the tautomer of the compound, the pharmaceutically acceptable salt thereof, or the solvate thereof according to 30), in which p is 1; and R^{a} means a halogen atom, a nitro group, a C₁₋₆ alkyl group, a C₁₋₆ haloalkyl group, a C₁₋₆ alkoxy group, or a C₁₋₆ haloalkoxy group.
32) The compound, the tautomer of the compound, the pharmaceutically acceptable salt thereof, or the solvate thereof according to any one of 1) to 31), in which L¹ and L² are single bonds; and B is a 4 to 7-membered heterocyclylene group (the 4 to 7-membered heterocyclylene group is unsubstituted or substituted with one or more substituents independently selected from a substituent group V⁵ and further a single methylene group in the 4 to 7-membered heterocyclylene group is optionally replaced by a 1,1-C₃₋₇cycloalkylene group).
33) The compound, the tautomer of the compound, the pharmaceutically acceptable salt thereof, or the solvate thereof according to 32), in which B is a 6-membered heterocyclylene group (the 6-membered heterocyclylene group is unsubstituted or substituted with one or more substituents independently selected from the substituent group V³).
34) The compound, the tautomer of the compound, the pharmaceutically acceptable salt thereof, or the solvate thereof according to 33), in which B is any one of Formulae (III-1) to (III-3) illustrated in (III) (where m is 0 to 3; R^{b} means a substituent selected from the substituent group V³; and when m is 2 or 3, R^{b}s may be the same as or different from each other). 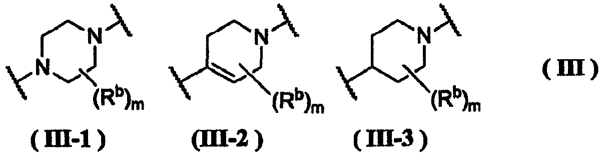
35) The compound, the tautomer of the compound, the pharmaceutically acceptable salt thereof, or the solvate thereof according to 34), in which m is 0 or 1; and R^{b} means a hydroxy group, a halogen atom, an amino group, a C₁₋₆ alkyl group, or a C₁₋₆ alkoxy group.
36) The compound, the tautomer of the compound, the pharmaceutically acceptable salt thereof, or the solvate thereof according to 34), in which m is 0 or 1; and R^{b} means a hydroxy group, a halogen atom, or an amino group.
37) The compound, the tautomer of the compound, the pharmaceutically acceptable salt thereof, or the solvate thereof according to any one of 1) to 24), in which L¹ and L² are single bonds; A is a hydrogen atom; and B is a tetrahydroisoquinoline-diyl group or a tetrahydrocarboline-diyl group (the tetrahydroisoquinoline-diyl group and the tetrahydrocarboline-diyl group are unsubstituted or substituted with one or more substituents independently selected from the substituent group V³, and further a single methylene group in the tetrahydroisoquinoline-diyl group and the tetrahydrocarboline-diyl group is optionally replaced by a 1,1-C₃₋₇ cycloalkylene group).
38) The compound, the tautomer of the compound, the pharmaceutically acceptable salt thereof, or the solvate thereof according to 37), in which A-L¹-B is either Formula (II-1) or Formula (II-2): 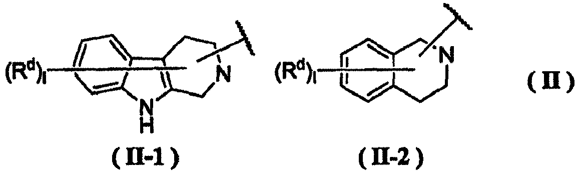 (wherein 1 is 1 to 3; R^{d} is optionally substituted for a tetrahydrocarboline ring or a tetrahydroisoquinoline ring at any positions; R^{d} means a hydroxy group, a halogen atom, an amino group, a C₁₋₆ alkyl group, a C₁₋₆ haloalkyl group, a C₁₋₆ alkoxy group, a C₁₋₆ haloalkoxy group, a mono-C₁₋₆ alkylamino group, a di-C₁₋₆ alkylamino group, or a C₁₋₆ alkylsulfonylamino group; and when 1 is 2 or 3, R^{d}s may be the same as or different from each other).
39) The compound, the tautomer of the compound, the pharmaceutically acceptable salt thereof, or the solvate thereof according to 38), in which A-L¹-B is either Formula (II-1) or Formula (II-2): 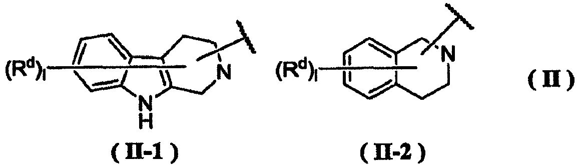 (wherein 1 is 1 or 2; R^{d} is optionally substituted for a tetrahydrocarboline ring or a tetrahydroisoquinoline ring at any positions; R^{d} means a hydroxy group, a halogen atom, a C₁₋₃ alkyl group, a C₁₋₃ haloalkyl group, a C₁₋₃ alkoxy group, or a C₁₋₃ haloalkoxy group; and when 1 is 2, R^{d}s may be the same as or different from each other).
40) A compound of Formula (I): 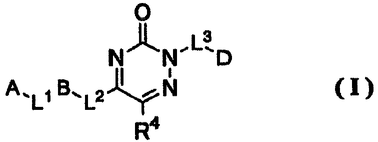 wherein R⁴ is a hydrogen atom; L¹ and L² are single bonds; L³ is a methylene group; and A, B, and D are a combination listed in Table 1 below, a tautomer of the compound, a pharmaceutically acceptable salt thereof, or a solvate thereof.
The symbols in Table 1 are the substituents shown below.

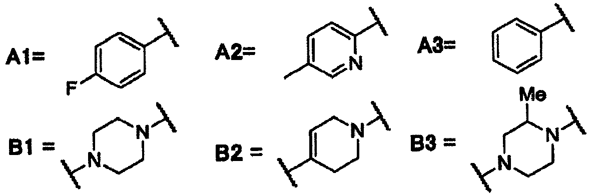

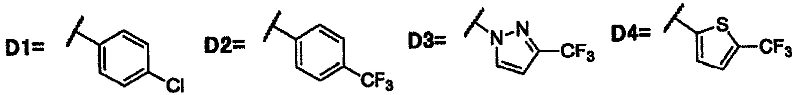

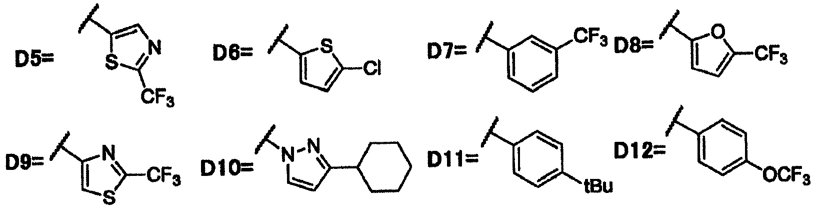

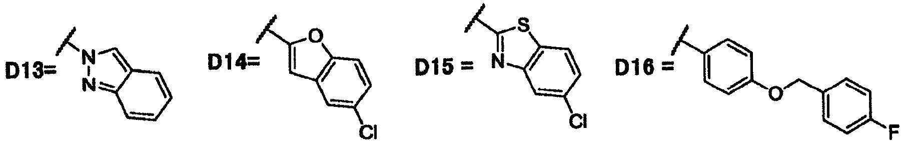

**Table 1**

| A | B | D | A | B | D | A | B | D |
|---|---|---|---|---|---|---|---|---|
| A 1 | B 1 | D 1 | A 1 | B 1 | D 2 | A 1 | B 1 | D 3 |
| A 1 | B 1 | D 4 | A 1 | B 1 | D 5 | A 1 | B 1 | D 6 |
| A 1 | B 1 | D 7 | A 1 | B 1 | D 8 | A 1 | B 1 | D 9 |
| A 1 | B 1 | D 1 0 | A 1 | B 1 | D 1 1 | A 1 | B 1 | D 1 2 |
| A 1 | B 1 | D 1 3 | A 1 | B 1 | D 1 4 | A 1 | B 1 | D 1 5 |
| A 1 | B 1 | D 1 6 | A 2 | B 1 | D 1 | A 2 | B 1 | D 2 |
| A 2 | B 1 | D 3 | A 2 | B 1 | D 4 | A 2 | B 1 | D 5 |
| A 2 | B 1 | D 6 | A 2 | B 1 | D 7 | A 2 | B 1 | D 8 |
| A 2 | B 1 | D 9 | A 2 | B 1 | D 1 0 | A 2 | B 1 | D 1 1 |
| A 2 | B 1 | D 1 2 | A 2 | B 1 | D 1 3 | A 2 | B 1 | D 1 4 |
| A 2 | B 1 | D 1 5 | A 2 | B 1 | D 1 6 | A 3 | B 1 | D 1 |
| A 3 | B 1 | D 2 | A 3 | B 1 | D 3 | A 3 | B 1 | D 4 |
| A 3 | B 1 | D 5 | A 3 | B 1 | D 6 | A 3 | B 1 | D 7 |
| A 3 | B 1 | D 8 | A 3 | B 1 | D 9 | A 3 | B 1 | D 1 0 |
| A 3 | B 1 | D 1 1 | A 3 | B 1 | D 1 2 | A 3 | B 1 | D 1 3 |
| A 3 | B 1 | D 1 4 | A 3 | B 1 | D 1 5 | A 3 | B 1 | D 1 6 |
| A 1 | B 2 | D 1 | A 1 | B 2 | D 2 | A 1 | B 2 | D 3 |
| A 1 | B 2 | D 4 | A 1 | B 2 | D 5 | A 1 | B 2 | D 6 |
| A 1 | B 2 | D 7 | A 1 | B 2 | D 8 | A 1 | B 2 | D 9 |
| A 1 | B 2 | D 1 0 | A 1 | B 2 | D 1 1 | A 1 | B 2 | D 1 2 |
| A 1 | B 2 | D 1 3 | A 1 | B 2 | D 1 4 | A 1 | B 2 | D 1 5 |
| A 1 | B 2 | D 1 6 | A 2 | B 2 | D 1 | A 2 | B 2 | D 2 |
| A 2 | B 2 | D 3 | A 2 | B 2 | D 4 | A 2 | B 2 | D 5 |
| A 2 | B 2 | D 6 | A 2 | B 2 | D 7 | A 2 | B 2 | D 8 |
| A 2 | B 2 | D 9 | A 2 | B 2 | D 1 0 | A 2 | B 2 | D 1 1 |
| A 2 | B 2 | D 1 2 | A 2 | B 2 | D 1 3 | A 2 | B 2 | D 1 4 |
| A 2 | B 2 | D 1 5 | A 2 | B 2 | D 1 6 | A 3 | B 2 | D 1 |
| A 3 | B 2 | D 2 | A 3 | B 2 | D 3 | A 3 | B 2 | D 4 |
| A 3 | B 2 | D 5 | A 3 | B 2 | D 6 | A 3 | B 2 | D 7 |
| A 3 | B 2 | D 8 | A 3 | B 2 | D 9 | A 3 | B 2 | D 1 0 |
| A 3 | B 2 | D 1 1 | A 3 | B 2 | D 1 2 | A 3 | B 2 | D 1 3 |
| A 3 | B 2 | D 1 4 | A 3 | B 2 | D 1 5 | A 3 | B 2 | D 1 6 |
| A 1 | B 3 | D 1 | A 1 | B 3 | D 2 | A 1 | B 3 | D 3 |
| A 1 | B 3 | D 4 | A 1 | B 3 | D 5 | A 1 | B 3 | D 6 |
| A 1 | B 3 | D 7 | A 1 | B 3 | D 8 | A 1 | B 3 | D 9 |
| A 1 | B 3 | D 1 0 | A 1 | B 3 | D 1 1 | A 1 | B 3 | D 1 2 |
| A 1 | B 3 | D 1 3 | A 1 | B 3 | D 1 4 | A 1 | B 3 | D 1 5 |
| A 1 | B 3 | D 1 6 | A 2 | B 3 | D 1 | A 2 | B 3 | D 2 |
| A 2 | B 3 | D 3 | A 2 | B 3 | D 4 | A 2 | B 3 | D 5 |
| A 2 | B 3 | D 6 | A 2 | B 3 | D 7 | A 2 | B 3 | D 8 |
| A 2 | B 3 | D 9 | A 2 | B 3 | D 1 0 | A 2 | B 3 | D 1 1 |
| A 2 | B 3 | D 1 2 | A 2 | B 3 | D 1 3 | A 2 | B 3 | D 1 4 |
| A 2 | B 3 | D 1 5 | A 2 | B 3 | D 1 6 | A 3 | B 3 | D 1 |
| A 3 | B 3 | D 2 | A 3 | B 3 | D 3 | A 3 | B 3 | D 4 |
| A 3 | B 3 | D 5 | A 3 | B 3 | D 6 | A 3 | B 3 | D 7 |
| A 3 | B 3 | D 8 | A 3 | B 3 | D 9 | A 3 | B 3 | D 1 0 |
| A 3 | B 3 | D 1 1 | A 3 | B 3 | D 1 2 | A 3 | B 3 | D 1 3 |
| A 3 | B 3 | D 1 4 | A 3 | B 3 | D 1 5 | A 3 | B 3 | D 1 6 |

41) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 41). 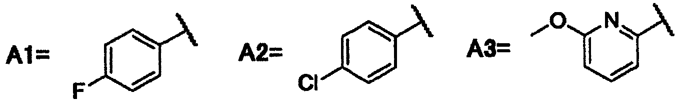 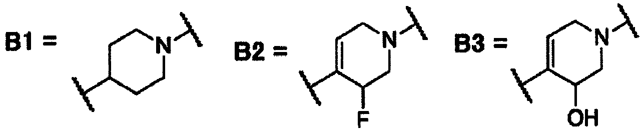 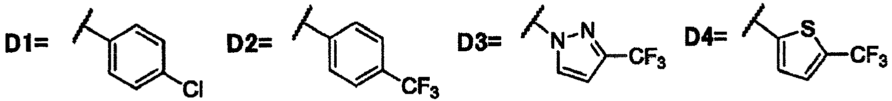 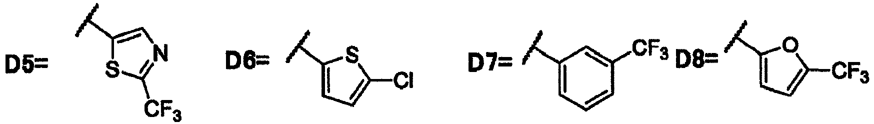 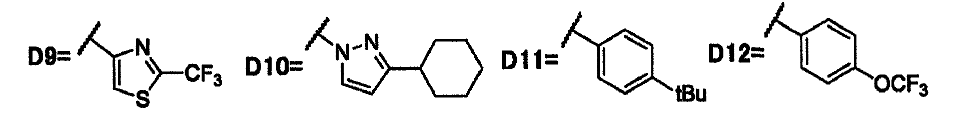 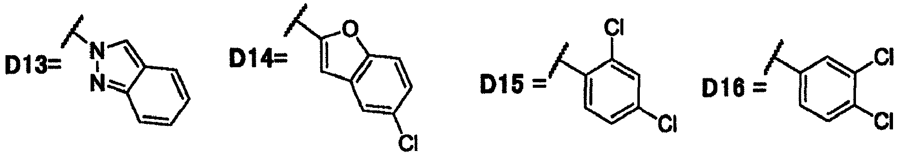
42) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 42). 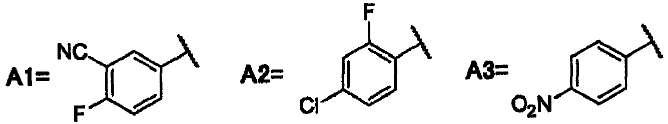 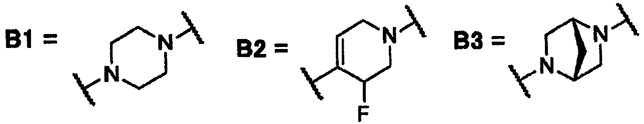 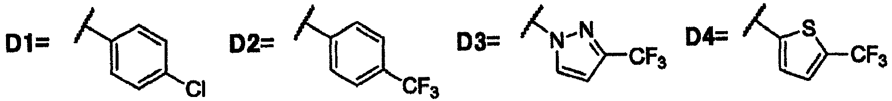 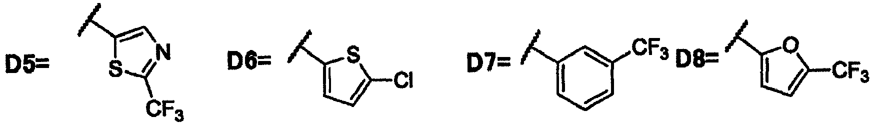 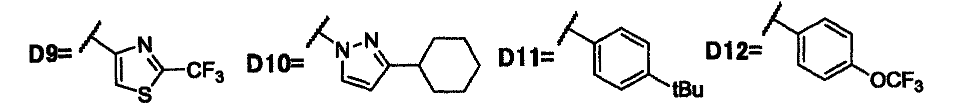 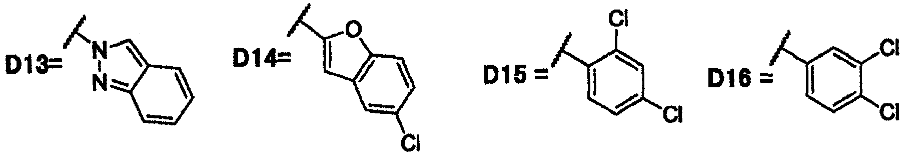
43) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 43). 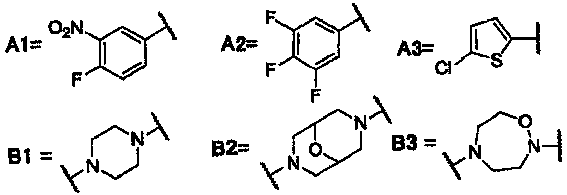 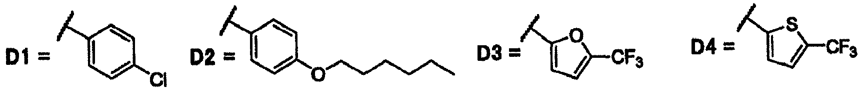 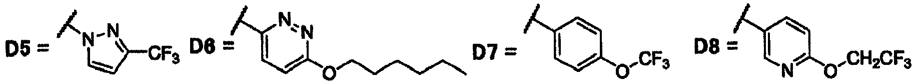 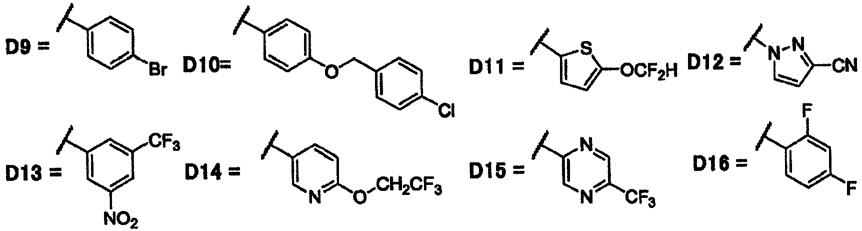
44) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 44). 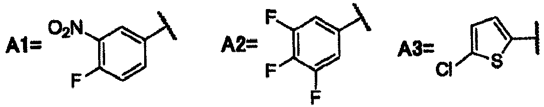 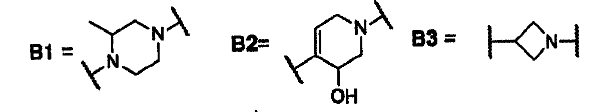 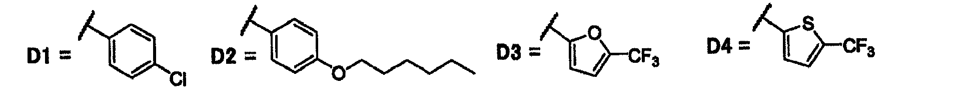 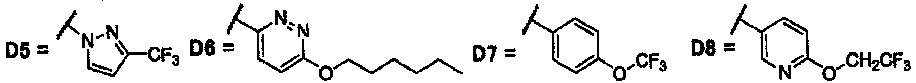 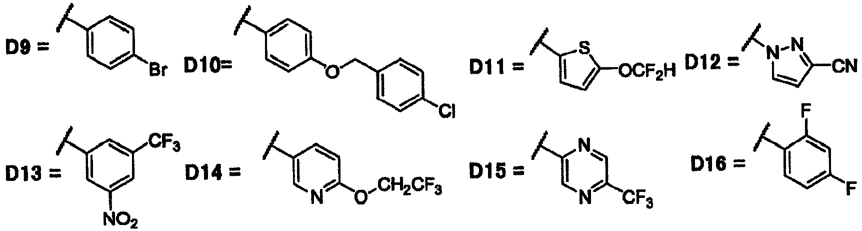
45) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 2 below.

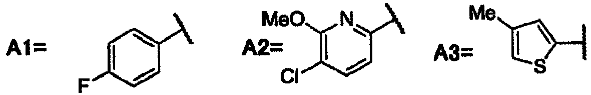

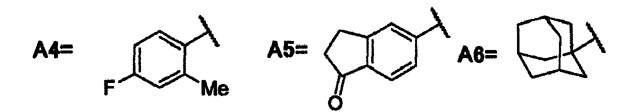

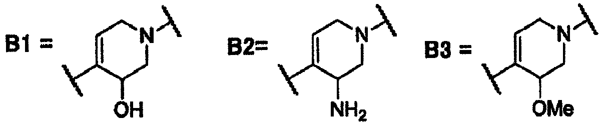

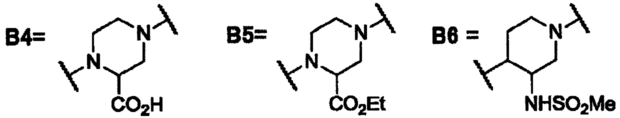

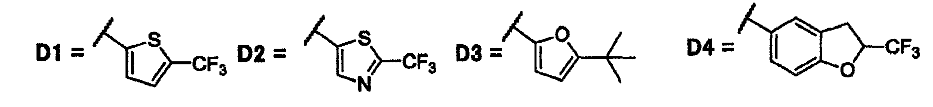

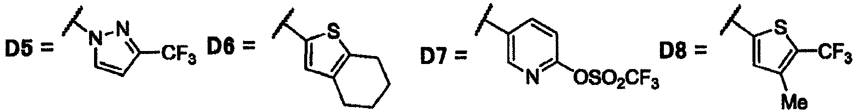

**Table 2**

| A | B | D | A | B | D | A | B | D |
|---|---|---|---|---|---|---|---|---|
| A 1 | B 1 | D 1 | A 1 | B 1 | D 2 | A 1 | B 1 | D 3 |
| A 1 | B 1 | D 4 | A 1 | B 1 | D 5 | A 1 | B 1 | D 6 |
| A 1 | B 1 | D 7 | A 1 | B 1 | D 8 | A 2 | B 1 | D 1 |
| A 2 | B 1 | D 2 | A 2 | B 1 | D 3 | A 2 | B 1 | D 4 |
| A 2 | B 1 | D 5 | A 2 | B 1 | D 6 | A 2 | B 1 | D 7 |
| A 2 | B 1 | D 8 | A 3 | B 1 | D 1 | A 3 | B 1 | D 2 |
| A 3 | B 1 | D 3 | A 3 | B 1 | D 4 | A 3 | B 1 | D 5 |
| A 3 | B 1 | D 6 | A 3 | B 1 | D 7 | A 3 | B 1 | D 8 |
| A 4 | B 1 | D 1 | A 4 | B 1 | D 2 | A 4 | B 1 | D 3 |
| A 4 | B 1 | D 4 | A 4 | B 1 | D 5 | A 4 | B 1 | D 6 |
| A 4 | B 1 | D 7 | A 4 | B 1 | D 8 | A 5 | B 1 | D 1 |
| A 5 | B 1 | D 2 | A 5 | B 1 | D 3 | A 5 | B 1 | D 4 |
| A 5 | B 1 | D 5 | A 5 | B 1 | D 6 | A 5 | B 1 | D 7 |
| A 5 | B 1 | D 8 | A 6 | B 1 | D 1 | A 6 | B 1 | D 2 |
| A 6 | B 1 | D 3 | A 6 | B 1 | D 4 | A 6 | B 1 | D 5 |
| A 6 | B 1 | D 6 | A 6 | B 1 | D 7 | A 6 | B 1 | D 8 |
| A 1 | B 2 | D 1 | A 1 | B 2 | D 2 | A 1 | B 2 | D 3 |
| A 1 | B 2 | D 4 | A 1 | B 2 | D 5 | A 1 | B 2 | D 6 |
| A 1 | B 2 | D 7 | A 1 | B 2 | D 8 | A 2 | B 2 | D 1 |
| A 2 | B 2 | D 2 | A 2 | B 2 | D 3 | A 2 | B 2 | D 4 |
| A 2 | B 2 | D 5 | A 2 | B 2 | D 6 | A 2 | B 2 | D 7 |
| A 2 | B 2 | D 8 | A 3 | B 2 | D 1 | A 3 | B 2 | D 2 |
| A 3 | B 2 | D 3 | A 3 | B 2 | D 4 | A 3 | B 2 | D 5 |
| A 3 | B 2 | D 6 | A 3 | B 2 | D 7 | A 3 | B 2 | D 8 |
| A 4 | B 2 | D 1 | A 4 | B 2 | D 2 | A 4 | B 2 | D 3 |
| A 4 | B 2 | D 4 | A 4 | B 2 | D 5 | A 4 | B 2 | D 6 |
| A 4 | B 2 | D 7 | A 4 | B 2 | D 8 | A 5 | B 2 | D 1 |
| A 5 | B 2 | D 2 | A 5 | B 2 | D 3 | A 5 | B 2 | D 4 |
| A 5 | B 2 | D 5 | A 5 | B 2 | D 6 | A 5 | B 2 | D 7 |
| A 5 | B 2 | D 8 | A 6 | B 2 | D 1 | A 6 | B 2 | D 2 |
| A 6 | B 2 | D 3 | A 6 | B 2 | D 4 | A 6 | B 2 | D 5 |
| A 6 | B 2 | D 6 | A 6 | B 2 | D 7 | A 6 | B 2 | D 8 |
| A 1 | B 3 | D 1 | A 1 | B 3 | D 2 | A 1 | B 3 | D 3 |
| A 1 | B 3 | D 4 | A 1 | B 3 | D 5 | A 1 | B 3 | D 6 |
| A 1 | B 3 | D 7 | A 1 | B 3 | D 8 | A 2 | B 3 | D 1 |
| A 2 | B 3 | D 2 | A 2 | B 3 | D 3 | A 2 | B 3 | D 4 |
| A 2 | B 3 | D 5 | A 2 | B 3 | D 6 | A 2 | B 3 | D 7 |
| A 2 | B 3 | D 8 | A 3 | B 3 | D 1 | A 3 | B 3 | D 2 |
| A 3 | B 3 | D 3 | A 3 | B 3 | D 4 | A 3 | B 3 | D 5 |
| A 3 | B 3 | D 6 | A 3 | B 3 | D 7 | A 3 | B 3 | D 8 |
| A 4 | B 3 | D 1 | A 4 | B 3 | D 2 | A 4 | B 3 | D 3 |
| A 4 | B 3 | D 4 | A 4 | B 3 | D 5 | A 4 | B 3 | D 6 |
| A 4 | B 3 | D 7 | A 4 | B 3 | D 8 | A 5 | B 3 | D 1 |
| A 5 | B 3 | D 2 | A 5 | B 3 | D 3 | A 5 | B 3 | D 4 |
| A 5 | B 3 | D 5 | A 5 | B 3 | D 6 | A 5 | B 3 | D 7 |
| A 5 | B 3 | D 8 | A 6 | B 3 | D 1 | A 6 | B 3 | D 2 |
| A 6 | B 3 | D 3 | A 6 | B 3 | D 4 | A 6 | B 3 | D 5 |
| A 6 | B 3 | D 6 | A 6 | B 3 | D 7 | A 6 | B 3 | D 8 |
| A 1 | B 4 | D 1 | A 1 | B 4 | D 2 | A 1 | B 4 | D 3 |
| A 1 | B 4 | D 4 | A 1 | B 4 | D 5 | A 1 | B 4 | D 6 |
| A 1 | B 4 | D 7 | A 1 | B 4 | D 8 | A 2 | B 4 | D 1 |
| A 2 | B 4 | D 2 | A 2 | B 4 | D 3 | A 2 | B 4 | D 4 |
| A 2 | B 4 | D 5 | A 2 | B 4 | D 6 | A 2 | B 4 | D 7 |
| A 2 | B 4 | D 8 | A 3 | B 4 | D 1 | A 3 | B 4 | D 2 |
| A 3 | B 4 | D 3 | A 3 | B 4 | D 4 | A 3 | B 4 | D 5 |
| A 3 | B 4 | D 6 | A 3 | B 4 | D 7 | A 3 | B 4 | D 8 |
| A 4 | B 4 | D 1 | A 4 | B 4 | D 2 | A 4 | B 4 | D 3 |
| A 4 | B 4 | D 4 | A 4 | B 4 | D 5 | A 4 | B 4 | D 6 |
| A 4 | B 4 | D 7 | A 4 | B 4 | D 8 | A 5 | B 4 | D 1 |
| A 5 | B 4 | D 2 | A 5 | B 4 | D 3 | A 5 | B 4 | D 4 |
| A 5 | B 4 | D 5 | A 5 | B 4 | D 6 | A 5 | B 4 | D 7 |
| A 5 | B 4 | D 8 | A 6 | B 4 | D 1 | A 6 | B 4 | D 2 |
| A 6 | B 4 | D 3 | A 6 | B 4 | D 4 | A 6 | B 4 | D 5 |
| A 6 | B 4 | D 6 | A 6 | B 4 | D 7 | A 6 | B 4 | D 8 |
| A 1 | B 5 | D 1 | A 1 | B 5 | D 2 | A 1 | B 5 | D 3 |
| A 1 | B 5 | D 4 | A 1 | B 5 | D 5 | A 1 | B 5 | D 6 |
| A 1 | B 5 | D 7 | A 1 | B 5 | D 8 | A 2 | B 5 | D 1 |
| A 2 | B 5 | D 2 | A 2 | B 5 | D 3 | A 2 | B 5 | D 4 |
| A 2 | B 5 | D 5 | A 2 | B 5 | D 6 | A 2 | B 5 | D 7 |
| A 2 | B 5 | D 8 | A 3 | B 5 | D 1 | A 3 | B 5 | D 2 |
| A 3 | B 5 | D 3 | A 3 | B 5 | D 4 | A 3 | B 5 | D 5 |
| A 3 | B 5 | D 6 | A 3 | B 5 | D 7 | A 3 | B 5 | D 8 |
| A 4 | B 5 | D 1 | A 4 | B 5 | D 2 | A 4 | B 5 | D 3 |
| A 4 | B 5 | D 4 | A 4 | B 5 | D 5 | A 4 | B 5 | D 6 |
| A 4 | B 5 | D 7 | A 4 | B 5 | D 8 | A 5 | B 5 | D 1 |
| A 5 | B 5 | D 2 | A 5 | B 5 | D 3 | A 5 | B 5 | D 4 |
| A 5 | B 5 | D 5 | A 5 | B 5 | D 6 | A 5 | B 5 | D 7 |
| A 5 | B 5 | D 8 | A 6 | B 5 | D 1 | A 6 | B 5 | D 2 |
| A 6 | B 5 | D 3 | A 6 | B 5 | D 4 | A 6 | B 5 | D 5 |
| A 6 | B 5 | D 6 | A 6 | B 5 | D 7 | A 6 | B 5 | D 8 |
| A 1 | B 6 | D 1 | A 1 | B 6 | D 2 | A 1 | B 6 | D 3 |
| A 1 | B 6 | D 4 | A 1 | B 6 | D 5 | A 1 | B 6 | D 6 |
| A 1 | B 6 | D 7 | A 1 | B 6 | D 8 | A 2 | B 6 | D 1 |
| A 2 | B 6 | D 2 | A 2 | B 6 | D 3 | A 2 | B 6 | D 4 |
| A 2 | B 6 | D 5 | A 2 | B 6 | D 6 | A 2 | B 6 | D 7 |
| A 2 | B 6 | D 8 | A 3 | B 6 | D 1 | A 3 | B 6 | D 2 |
| A 3 | B 6 | D 3 | A 3 | B 6 | D 4 | A 3 | B 6 | D 5 |
| A 3 | B 6 | D 6 | A 3 | B 6 | D 7 | A 3 | B 6 | D 8 |
| A 4 | B 6 | D 1 | A 4 | B 6 | D 2 | A 4 | B 6 | D 3 |
| A 4 | B 6 | D 4 | A 4 | B 6 | D 5 | A 4 | B 6 | D 6 |
| A 4 | B 6 | D 7 | A 4 | B 6 | D 8 | A 5 | B 6 | D 1 |
| A 5 | B 6 | D 2 | A 5 | B 6 | D 3 | A 5 | B 6 | D 4 |
| A 5 | B 6 | D 5 | A 5 | B 6 | D 6 | A 5 | B 6 | D 7 |
| A 5 | B 6 | D 8 | A 6 | B 6 | D 1 | A 6 | B 6 | D 2 |
| A 6 | B 6 | D 3 | A 6 | B 6 | D 4 | A 6 | B 6 | D 5 |
| A 6 | B 6 | D 6 | A 6 | B 6 | D 7 | A 6 | B 6 | D 8 |

46) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 2 (where A1 to A6, B1 to B6, and D1 to D8 in Table are the substituents shown below, in 46). 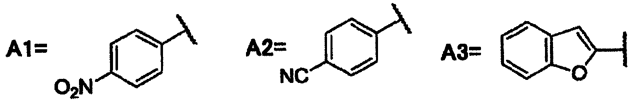 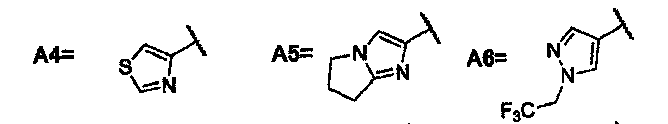 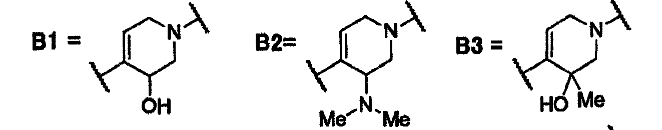 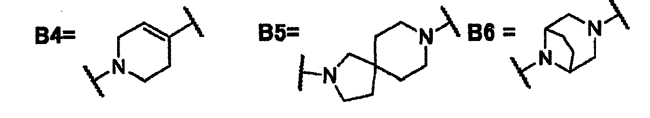 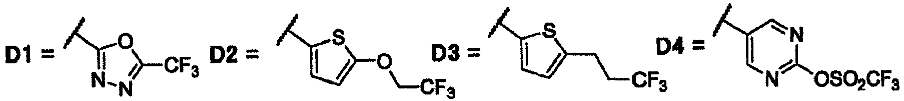 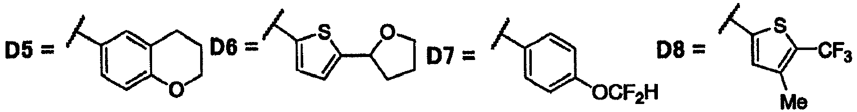
47) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 2 above (where A1 to A6, B1 to B6, and D1 to D8 in the Table are the substituents shown below, in 47). 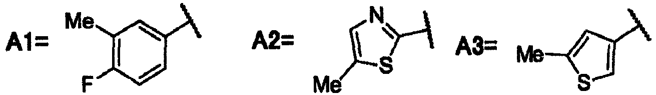 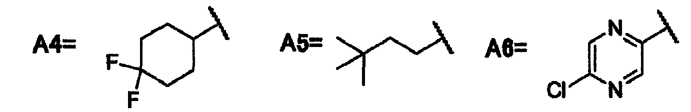 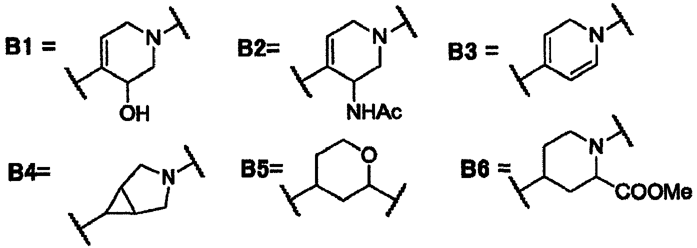 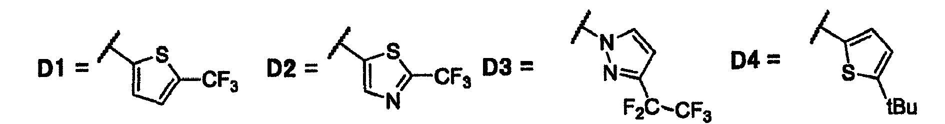 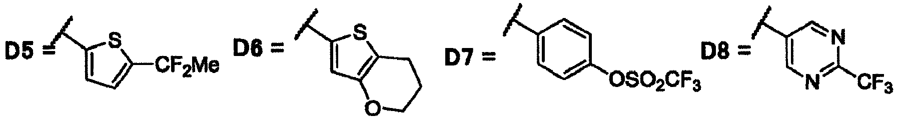
48) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 48). 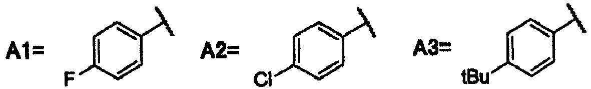 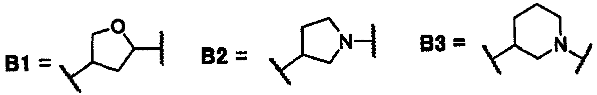 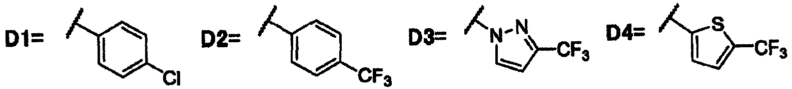 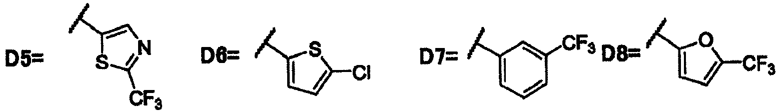 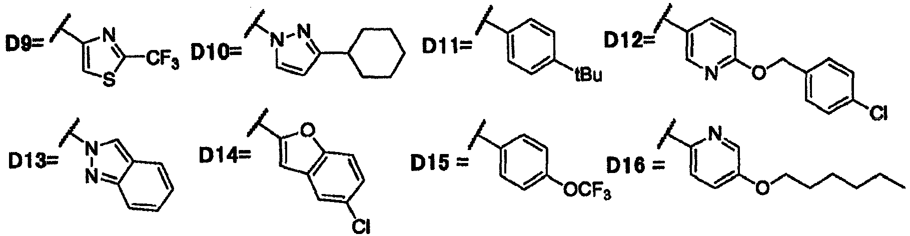
49) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D 16 in the Table are the substituents shown below, in 49). 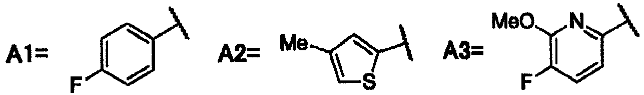 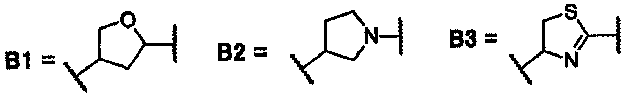 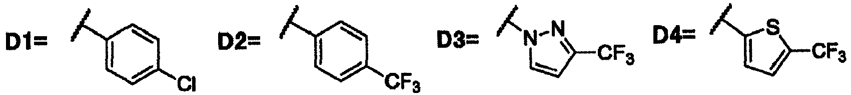 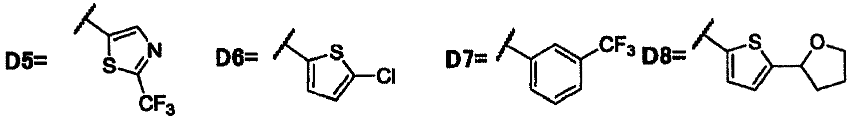 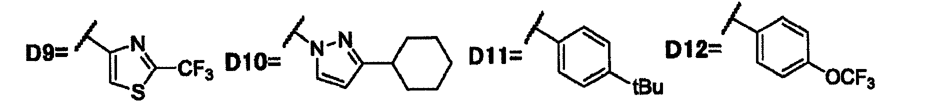 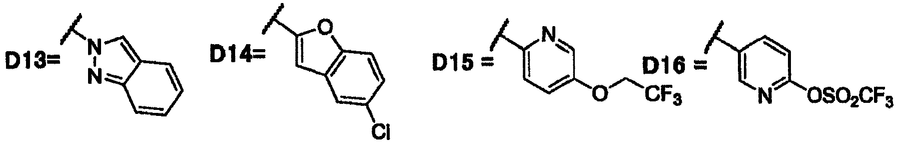
50) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 50). 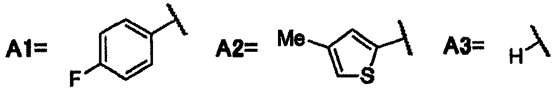 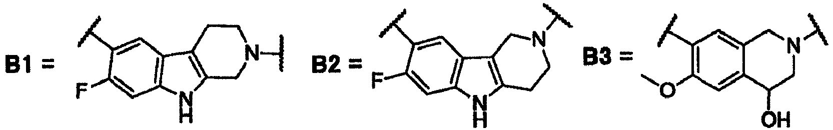 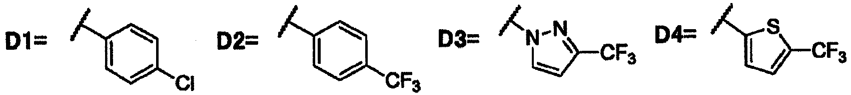 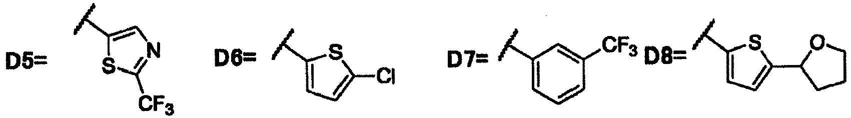 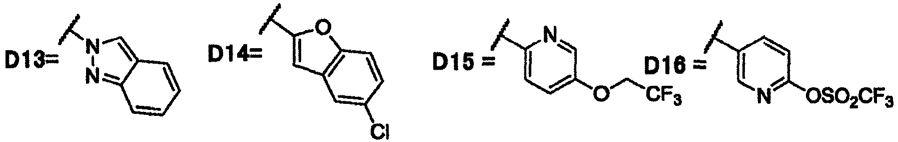
51) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 51). 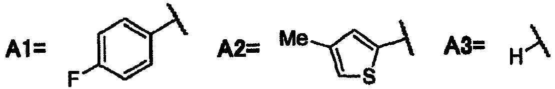 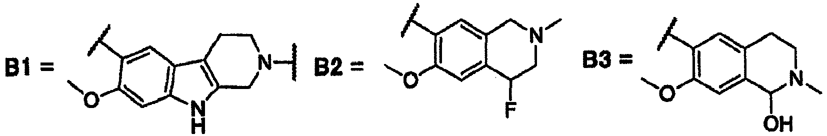 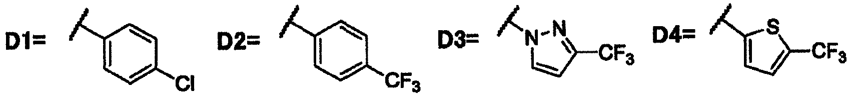 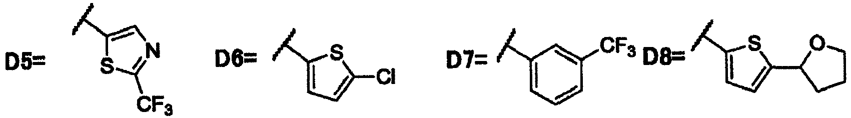 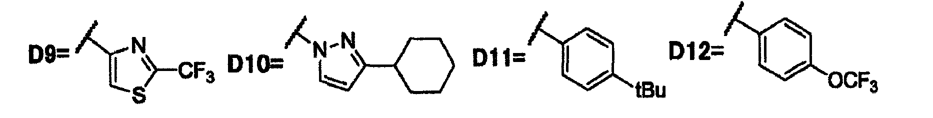 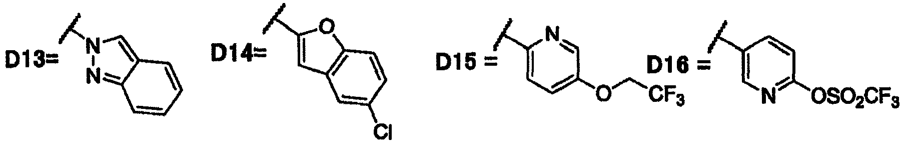
52) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 52). 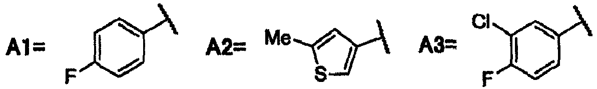 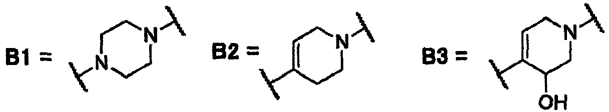 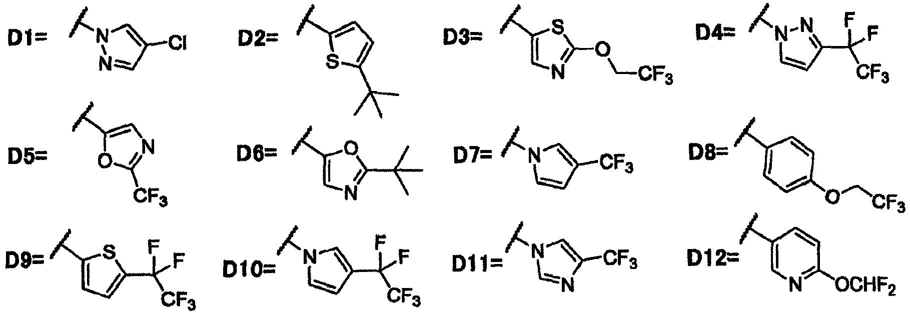 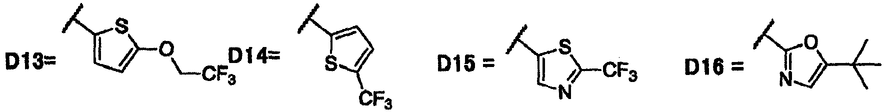
53) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 2 above (where A1 to A6, B1 to B6, and D1 to D8 in the Table are the substituents shown below, in 53). 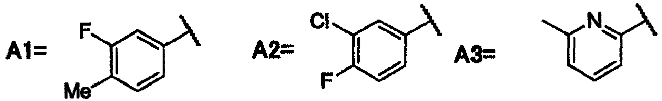 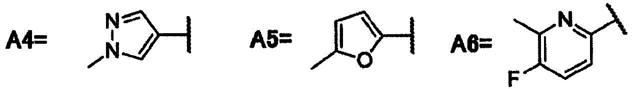 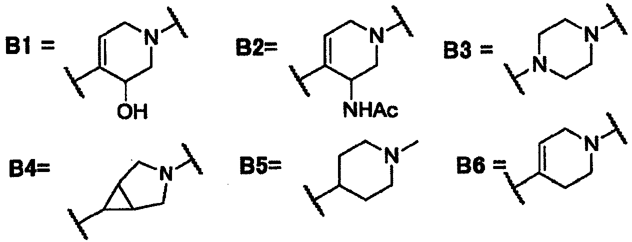 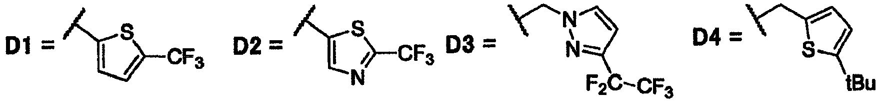 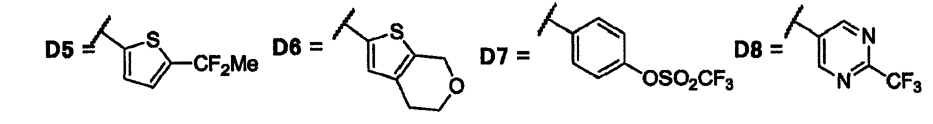
54) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 2 above (where A1 to A6, B1 to B6, and D1 to D8 in the Table are the substituents shown below, in 54). 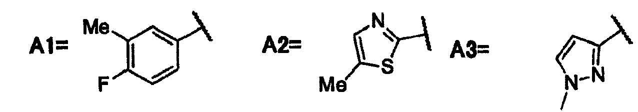 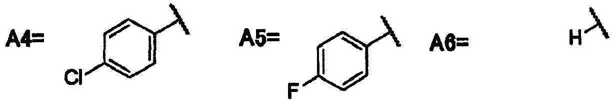 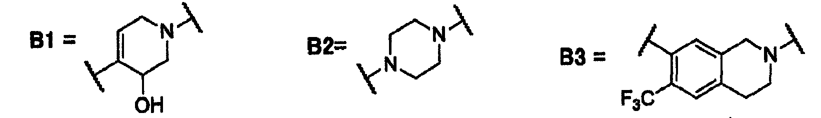 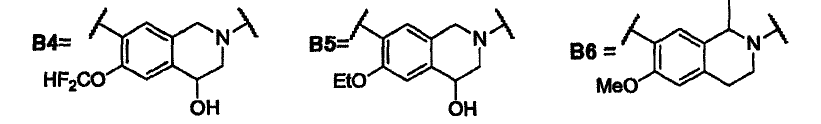 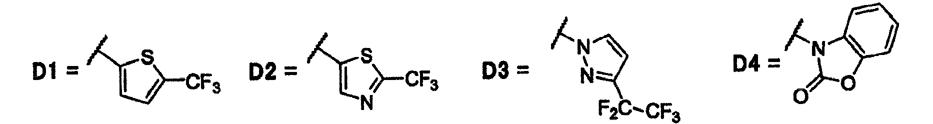 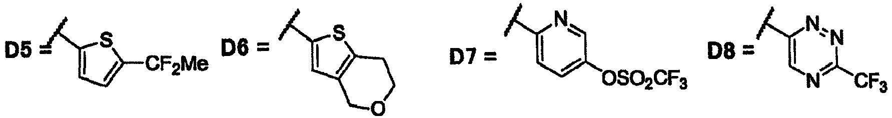
55) The compound of Formula (I), a tautomer of the compound, a pharmaceutically acceptable salt thereof, or a solvate thereof, in which R⁴ is a hydrogen atom; L¹ and L² are single bonds; L³ is a methylene group; and A, B, and D are combinations listed in Table 1 above (where A1 to A3, B1 to B3, and D1 to D16 in the Table are the substituents shown below, in 55). 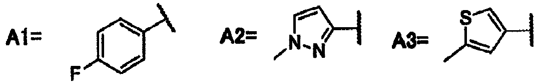 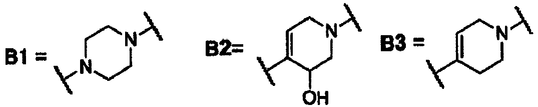 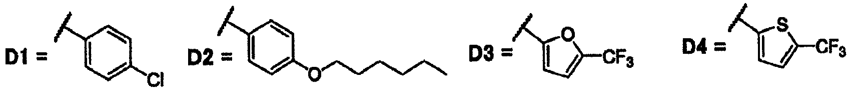 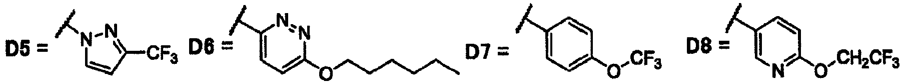 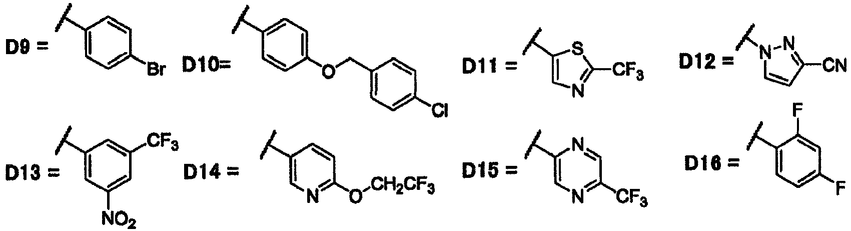
56) The compound including the combination according to 41), 44), 45), 47) to 49), or 53), a tautomer of the compound, a pharmaceutically acceptable salt thereof, or a solvate thereof, in which L¹ is NH.
57) The compound including the combination according to 41), 44), 45), 47) to 49), or 53), a tautomer of the compound, a pharmaceutically acceptable salt thereof, or a solvate thereof, in which L¹ is O.
58) The compound including the combination according to 40) to 49), 52) to 53), or 55), a tautomer of the compound, a pharmaceutically acceptable salt thereof, or a solvate thereof, in which L¹ is SO₂.
59) The compound including the combination according to 40) to 49), 52) to 53), or 55), a tautomer of the compound, a pharmaceutically acceptable salt thereof, or a solvate thereof, in which L¹ is CO.
60) The compound including the combination according to any one of 40) to 59), a tautomer of the compound, a pharmaceutically acceptable salt thereof, or a solvate thereof, in which L³ is an ethylene group.
61) The compound including the combination according to any one of 40) to 59), a tautomer of the compound, a pharmaceutically acceptable salt thereof, or a solvate thereof, in which L³ is a propane-1,3-diyl group.
62) A T-type calcium channel inhibitor including the compound as described in any one of 1) to 61), a tautomer of the compound, a pharmaceutically acceptable salt thereof, or a solvate thereof, as an active component.
63) A preventive agent, a therapeutic agent, and/or an improving agent for a disease treatable by a T-type calcium channel inhibitory activity including the T-type calcium channel inhibitor as described in 62) as an active component.
64) A therapeutic agent for neuropathic pain comprising the T-type calcium channel inhibitor as described in 62) as an active component.
65) A medicine including the compound as described in any one of 1) to 61), a tautomer of the compound, a pharmaceutically acceptable salt thereof, or a solvate thereof, as an active component.

The present invention also includes compounds obtained through, for example, tautomerization or geometric isomerization of the compound of Formula (I) of the present invention regardless of endocyclic or exocyclic isomerization, mixtures of them, and mixtures of respective isomers. If the compound has an asymmetric center or if an asymmetric center is generated by isomerization, the present invention includes respective optical isomers and mixtures of optical isomers at any ratio. If having two or more asymmetric centers, the compound further includes diastereomers due to optical isomerism of the respective asymmetric centers. The compound of the present invention includes mixtures containing all isomers at any ratio. For example, diastereomers can be separated by a method well-known to a person skilled in the art, such as fractional crystallization and column chromatography, and an optically active compound can be produced by an organic chemical technique well-known for the purpose.

The compound of Formula (I) of the present invention or pharmaceutically acceptable salts thereof can be present in any crystal form depending on production conditions and can be present as any hydrate. These crystal forms, hydrates, and mixtures of them are also included in the scope of the present invention. The compound may be present as a solvate containing an organic solvent such as acetone, ethanol, 1-propanol, and 2-propanol, and such solvates are also included in the scope of the present invention.

The present invention includes pharmaceutically acceptable salts of Formula (I) of the present invention.

The compound of Formula (I) of the present invention can be converted into a pharmaceutically acceptable salt, as necessary, or a free form of the compound can be converted from such a salt. Examples of the pharmaceutically acceptable salt of the present invention include salts of alkali metals (such as lithium, sodium, and potassium), alkaline earth metals (such as magnesium and calcium), ammonium, organic bases (such as triethylamine and trimethylamine), amino acids (such as glycine, lysine, and glutamic acid), inorganic acids (such as hydrochloric acid, hydrobromic acid, phosphoric acid, and sulfuric acid), and organic acids (such as acetic acid, citric acid, maleic acid, fumaric acid, tartaric acid, benzenesulfonic acid, methanesulfonic acid, and p-toluenesulfonic acid).

The present invention also includes prodrugs of the compound of Formula (I) of the present invention.

Prodrugs are derivatives that are derived from a pharmaceutical compound and have a chemically or metabolically degradable group and are compounds that are degraded by solvolysis or under physiological conditions in vivo into a pharmacologically active, pharmaceutical compound. Methods for selecting and producing an appropriate prodrug derivative are described in Design of Prodrugs (Elsevier, Amsterdam 1985), for example. In the case of the present invention, if having a hydroxy group, the compound is reacted with an appropriate acyl halide, an appropriate acid anhydride, or an appropriate halogenated alkyloxycarbonyl compound to produce a prodrug such as an acyloxy derivative, for example. Particularly preferred structures for the prodrug are exemplified by -O-COC₂H₅, -O-CO(t-Bu), -O-COC₁₅H₃₁, -O-CO(m-CO₂Na-Ph), -O-COCH₂CH₂CO₂Na, -OCOCH(NH₂)CH₃, -O-COCH₂N(CH₃)₂, or -O-CH₂OC(=O)CH₃. If the compound included in the present invention has an amino group, the compound having an amino group is reacted with an appropriate acid halide, an appropriate mixed acid anhydride, or an appropriate halogenated alkyloxycarbonyl compound to produce a prodrug, for example. Particularly preferred structures for the prodrug are exemplified by -N-CO(CH₂)₂₀OCH₃, -N-COCH(NH₂)CH₃, and -N-CH₂OC(=O)CH₃.

The preventive agent, the therapeutic agent, and/or the improving agent that are used for a disease treatable by a T-type calcium channel inhibitory activity and include the T-type calcium channel inhibitor of the present invention as an active component can be typically administered in oral administration forms such as tablets, capsules, powdered drugs, granules, pills, and syrups, rectal administration forms, transdermal absorption forms, or injection forms. The agent can be administered as a single therapeutic agent or as a mixture with other therapeutic agents. Such an agent can be singly administered but is typically administered in the form of a pharmaceutical composition. Such a formulation can be produced by adding pharmacologically, pharmaceutically acceptable additives in usual ways. In other words, the oral formulations may contain common additives such as diluents, lubricants, binders, disintegrants, wetting agents, plasticizers, and coating agents. Liquid formulations for oral administration may be aqueous suspensions, oily suspensions, solutions, emulsions, syrups, elixirs, or other forms, or may be produced as dry syrups, to which water or another appropriate solvent is added before use. The liquid formulations may contain common additives such as suspending agents, flavors, diluents, and emulsifiers. For rectal administration, the agent can be administered as suppositories. The suppositories can contain appropriate substances such as cacao butter, laurin butter, macrogol, glycerogelatin, Witepsol, sodium stearate, and mixtures of them as a base and, as necessary, emulsifiers, suspending agents, preservatives, and other additives. For the injections, pharmaceutical components including solvents or solubilizing agents such as distilled water for injection, physiological saline, 5% glucose solution, and propylene glycol, pH regulators, tonicity agents, and stabilizing agents may be used to form aqueous dosage forms or dosage forms that need dissolution before use.

The pharmaceutical composition of the present invention includes the compound (or a pharmaceutically acceptable salt of the compound) of the present invention as an active component, pharmaceutically acceptable carriers, and, in some cases, one or a plurality of additional therapeutic agents or adjuvants. Examples of such an additional therapeutic agent can include i) cannabinoid receptor agonists or antagonists, ii) narcotic analgesics (opioid analgesics), iii) serotonin (5-HT) receptor agonists or antagonists, iv) sodium channel blockers, v) NMDA receptor agonists or antagonists, vi) COX-2 selective inhibitors, vii) NK1 antagonists, viii) non-steroidal anti-inflammatory drugs ("NSAIDs"), ix) selective serotonin reuptake inhibitors ("SSRIs") and/or selective serotonin and norepinephrine reuptake inhibitors ("SSNRIs"), x) tricyclic antidepressants, xi) GABA receptor agonists, xii) lithium, xiii) valproates, xiv) Neurontin (gabapentin), xv) pregabalin, xvi) adrenergic receptor agonists or antagonists, xvii) neurotropin, xviii) capsaicin (TRPV1) receptor agonists or antagonists, xix) CGRP receptor antagonists, xx) steroids, xxi) bisphosphonates, and xxii) histamine receptor antagonists. The composition contains compositions suitable for oral, rectal, local, and parenteral (including subcutaneous, intramuscular, and intravenous) administrations. An optimum route for any administration is determined depending on a specific host and specific conditions and seriousness of the host to which the active component is to be administered. The pharmaceutical composition can be favorably administered in a single unit dosage form and can be prepared by any method well-known in the field of pharmaceutics.

To administer the medicinal agent of the present invention to a human, the dose is determined depending on the age and conditions of a patient. The dose for adults is typically about 0.1 mg/human/day to 1,000 mg/human/day through oral or rectal administration and about 0.05 mg/human/day to 500 mg/human/day through injection. These numerical values are merely illustrative values, and the dose is determined depending on the conditions of a patient.

The compound or the pharmaceutical composition of the present invention are intended to be used for all diseases to which T-type calcium channels relate.

The target disease includes pain.

With regard to pain, the target disease is specifically chronic pain and more specifically neuropathic pain.

In more detail, the pain is classified into chronic pains and acute pains including neuropathic pain, inflammatory pain, cancer pain, and visceral pain, primary diseases of which are exemplified by diabetic neuropathy, traumatic neurological disorder, nerve compression, strangulation, spinal cord injury, cerebral apoplexy, fibromyalgia syndrome, carpal tunnel syndrome, osteoarthritis, rheumatoid arthritis and multiple sclerosis, herpes zoster, herpes simplex, syphilis, nerve disorders induced by cancer chemotherapy, HIV, and HIV treatment, chronic joint pain, postherpetic neuralgia, neuroma pain, trigeminal neuralgia, phantom limb pain, postoperative pain, stump pain, tooth pain, plexus neuropathy, glossopharyngeal neuralgia, laryngeal neuralgia, migraine, carcinomatous neuropathy, polyneuropathy, causalgia, low back pain, complex regional pain syndrome (CRPS), and thalamic pain.

Pains derived from other pains except the above, however, are also included in the target diseases of the present invention.

Examples of other diseases except the pain include diseases associated with central nervous system (CNS) disorders, diseases associated with bladder function disorders, cerebral apoplexy, itching, atopic dermatitis, hypertension, hyperaldosteronemia, edema, ischemic heart diseases, age-related macular degeneration, cancer, diabetes mellitus, sterility, sexual dysfunction, arrhythmia, and kidney disease. Examples of the diseases associated with central nervous system (CNS) disorders include epilepsy, essential tremor, schizophrenia, Parkinson's disease, manic-depressive illness, bipolar disorder, depression, anxiety, dementia, drug dependence, Huntington's disease, and sleep disturbance. Examples of the diseases associated with bladder function disorder include overactive bladder.

The compound is also clinically used for the treatment of epilepsy and partial and generalized tonic seizure. The compound is also useful for neuroprotection under ischemic conditions caused by cerebral apoplexy or neurotrauma and is useful for the treatment of multiple sclerosis. The compound is useful for the treatment of tachyarrhythmia. The compound is useful for the treatment of depression, more specifically, depressive disorders, for example, sudden or recurrent major depressive disorder, and mood disorders such as dysthymic disorder and bipolar disorders, for example, bipolar I disorder, bipolar II disorder, and cyclothymic disorder; and neuropsychiatric disorders including panic disorder with or without agoraphobia, agoraphobia with no panic disorder history, specific phobias, for example, phobia specific to animals and anthropophobia, obsessive-compulsive disorder, stress disorders such as posttraumatic stress disorder and acute stress disorder, and anxiety disorders such as generalized anxiety disorder.

In addition to primates including human beings, various other mammals can be treated by the method of the present invention. Examples of the treatable mammals include, but are not limited to, rodents (for example, mice), cattle, sheep, goats, horses, dogs, and cats. The method can also be performed on other species such as birds (for example, chickens).

When specifically used for the treatment of depression or anxiety, the compound of the present invention can be used in combination with other antidepressants or antianxiety drugs such as norepinephrine reuptake inhibitors, selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), α-adrenergic receptor antagonists, atypical antidepressants, benzodiazepines, 5-HT1A agonists or antagonists, specifically, 5-HT1A partial agonists, neurokinin-1 receptor antagonists, corticotropin-releasing factor (CRF) antagonists, and pharmaceutically acceptable salts of them.

In addition, the compound of the present invention can be administered in order to prevent the conditions and the disorders described above and to prevent other conditions and disorders to which calcium channel activity relates, in a dose level effective in the prevention.

Creams, ointments, jellies, solutions, or suspensions containing the compound can be locally used. Mouthwashes and gargles are included within the local applications for the present invention.

The compound of the present invention can be synthesized by the methods shown below, but the production methods below are typical examples of the production method and are not intended to limit the production method.

In a typical method for producing the compound of the present invention, for smooth reactions, a reaction in the presence of an acid or a base may efficiently proceed, and a reaction under microwave irradiation may efficiently proceed. Among the typical production methods shown below, in schemes (1) to (5), (8) to (11), (13) to (15), (18) to (19), and (21) to (23), a reaction particularly in the presence of a base such as sodium hydroxide, potassium hydroxide, potassium tert-butoxide, sodium tert-butoxide, cesium carbonate, potassium carbonate, and triethylamine may be efficient for a smooth reaction.

In the typical production methods shown below, each of reagents and raw material compounds can be appropriately used in an equimolar amount or an excess molar amount relative to one compound of raw materials.

In the typical production methods of the compound of the present invention shown below, general formulae of intermediates and a final product are shown in each process, but the general formulae of these intermediates and final products also generally include derivatives protected with protective groups. A derivative protected with a protective group means a compound that can yield a target compound by hydrolysis, reduction, oxidation, dehydration, halogenation, alkylation, or a similar reaction, as necessary, and includes a compound protected with a protective group that is acceptable for organic synthetic chemistry, for example.

Protection and deprotection can be carried out with well-known protective groups through protection and deprotection reactions (see Protective Groups in Organic Synthesis, Fourth edition, by T.W. Greene, John Wiley & Sons Inc, 2006, for example).

Hydrolysis, reduction, oxidation, dehydration, and halogenation can be carried out by well-known transformation methods of functional groups (see Comprehensive Organic Transformations, Second Edition, by R.C. Larock, Wiley-VCH, 1999, for example).

(Symbols in typical production method)

In the typical production methods shown below, symbols in the drawings mean as follows unless otherwise noted:
In formulae, R², R⁴, L¹, L², L³, A, B, D, and the substituent group V⁶ mean the same as in General Formula (I) described above unless otherwise noted.

R^{L} is a leaving group such as a halogen atom, a methanesulfonyloxy group, and a p-toluenesulfonyloxy group.

X is a halogen atom.

R^{PR} is a hydrogen atom or a protective group such as a Boc group and a Cbz group.

Of the compounds of Formula (I), compound (1)-3 can be produced by the production method of scheme (1) below, for example.

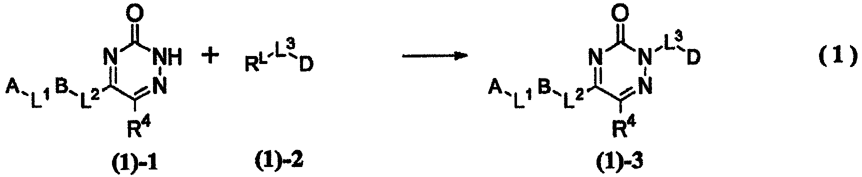

The compound (1)-3 can be synthesized using compound (1)-1 and an equal amount or excess amount of compound (1)-2 in an appropriate solvent or without solvent at from 0°C to a heat-reflux temperature.

Of the compounds of Formula (I), compound (2)-3 can be produced by the production method of scheme (2) below, for example (in the scheme, L¹ is a single bond, S, NR², or O; and B is a 3 to 13-membered heterocyclylene group containing NH or a 5 to 10-membered heteroarylene group containing NH when L¹ is a single bond).

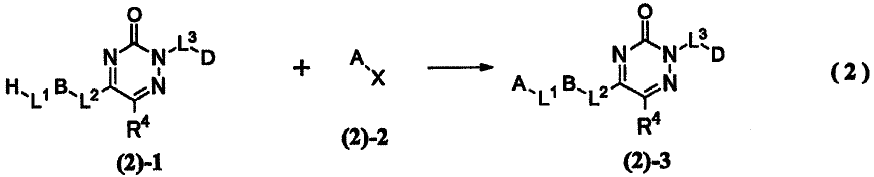

The compound (2)-3 can be synthesized using compound (2)-1 and an equal amount or excess amount of halogen derivative (2)-2 in the presence of copper powder or a copper salt in an appropriate solvent or without solvent at from 0°C to a heat-reflux temperature.

Of the compounds of Formula (I), compound (3)-3 can be produced by the production method of scheme (3) below, for example (in the scheme, L¹ is a single bond, S, NR², or O; and B is a 3 to 13-membered heterocyclylene group containing NH or a 5 to 10-membered heteroarylene group containing NH when L¹ is a single bond).

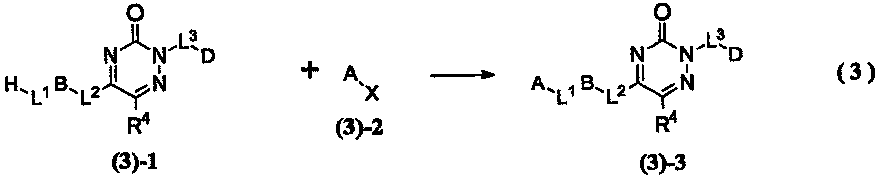

The compound (3)-3 can be synthesized using compound (3)-1 and an equal amount or excess amount of compound (3)-2 in the presence of a palladium catalyst such as tetrakis(triphenylphosphine)palladium (0), bis(triphenylphosphine)palladium (II) dichloride or bis(acetonitrile)palladium (II) dichloride in an appropriate solvent or without solvent at from 0°C to a heat-reflux temperature. Alternatively, the reaction can be carried out under reaction conditions used for Buchwald-Hartwig reaction (see Advanced Synthesis & Catalysis, 2004, 346, pp.1599-1626, for example). Although the reaction conditions are not paticularly limited, tris(dibenzylideneacetone)dipalladium (0), tetrakis(triphenylphosphine)palladium (0), palladium (II) acetate, or the like may be appropriately combined with 4,5-bis(diphenylphosphino)-9,9-dimethylxanthene (Xantphos), 2-dicyclohexylphosphino-2',6'-dimethoxybiphenyl (SPhos), 2-dicyclohexylphosphino-2',4',6'-triisopropylbiphenyl (XPhos), or the like.

Of the compounds of Formula (I), compound (4)-3 can be produced by the production method of scheme (4) below, for example (in the scheme, L² is a single bond, S, NR², or O; B is a 3 to 13-membered heterocyclylene group containing NH or a 5 to 10-membered heteroarylene group containing NH when L² is a single bond; and R^{A} is a hydrogen atom, a C₁₋₆ alkyl group (the C₁₋₆ alkyl group is unsubstituted or substituted with one or more substituents independently selected from the substituent group V⁶), or a dodecyl group).

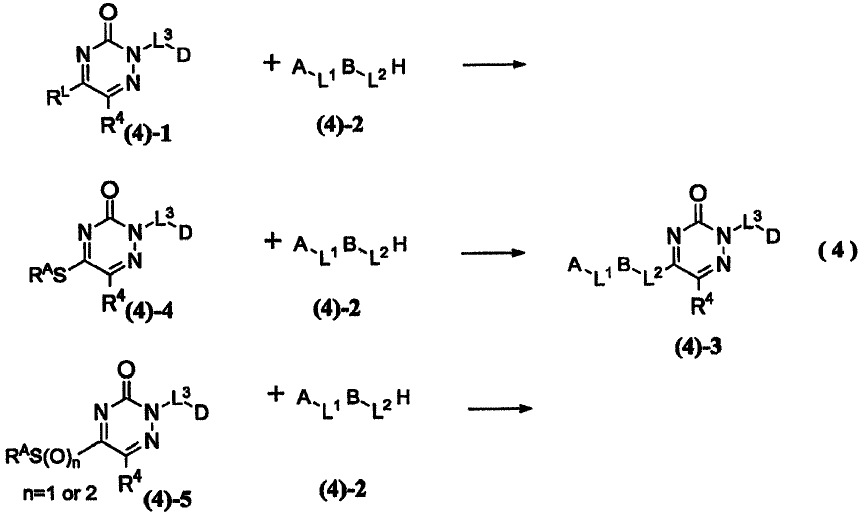

The compound (4)-3 can be synthesized using compound (4)-1, compound (4)-4, or compound (4)-5 and an equal amount or excess amount of compound (4)-2 in an appropriate solvent or without solvent at from 0°C to a heat-reflux temperature.

Of the compounds of Formula (I), compound (5)-3 can be produced by the production method of scheme (5) below, for example (in the scheme, B is a 3 to 13-membered heterocyclylene group containing NH or a 5 to 10-membered heteroarylene group containing NH).

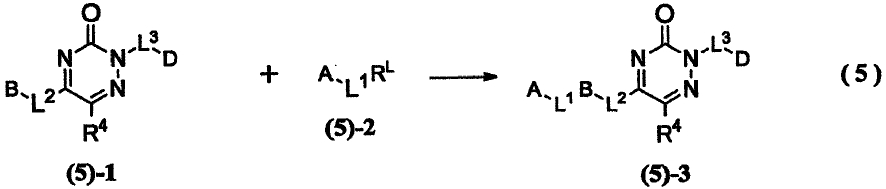

The compound (5)-3 can be synthesized using compound (5)-1 and an equal amount or excess amount of compound (5)-2 in an appropriate solvent or without solvent at from 0°C to a heat-reflux temperature.

Of the compounds of Formula (I), compound (6)-3 can be produced by the production method of scheme (6) below, for example.

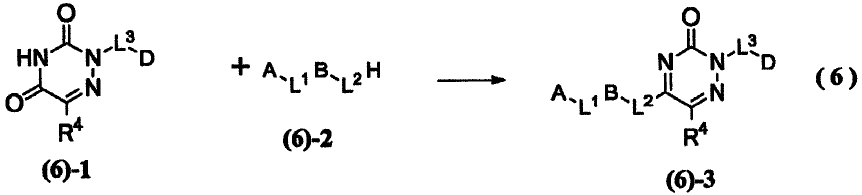

The compound (6)-3 can be synthesized using compound (6)-1 and an equal amount or excess amount of compound (6)-2 in the presence of an equal amount or excess amount of a silylating agent such as N,O-bis(trimethylsilyl)acetamide or hexamethyldisilazane or an equal amount or excess amount of a dehydration condensation agent such as O-(7-azabenzotriazol-1-yl)-N",N, N',N'-tetramethyluronium hexafluorophosphate or 1-ethyl-3-(3-dimethylaminopropyl)carbodiimide in an appropriate solvent or without solvent at from 0°C to a heat-reflux temperature.

Of the compounds of Formula (I), compound (7)-4 can be produced by the production method of scheme (7) below, for example.

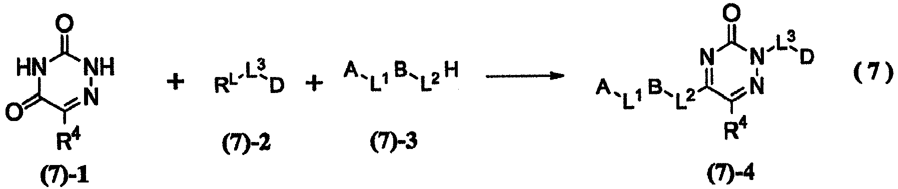

The compound (7)-4 can be synthesized by sequential reactions of compound (7)-1 with an equal amount or excess amount of compound (7)-2 and an equal amount or excess amount of compound (7)-3 in the presence of an equal amount or excess amount of a silylating agent such as N,O-bis(trimethylsilyl)acetamide or hexamethyldisilazane in an appropriate solvent or without solvent at from 0°C to a heat-reflux temperature.

Of the compounds of Formula (I), compound (8)-3 can be produced by the production method of scheme (8) below, for example.

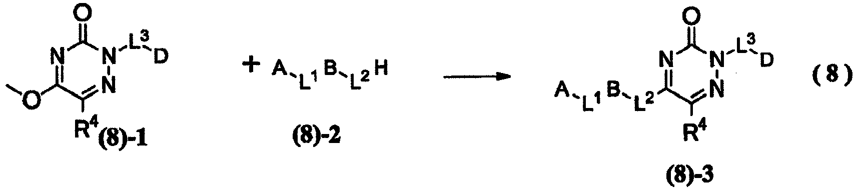

The compound (8)-3 can be synthesized using compound (8)-1 and an equal amount or excess amount of compound (8)-2 in an appropriate solvent or without solvent at from 0°C to a heat-reflux temperature.

### Raw material synthesis 1

Compound (9)-2 can be produced by the production method of scheme (9) below, for example (In the scheme, X is a halogen atom).

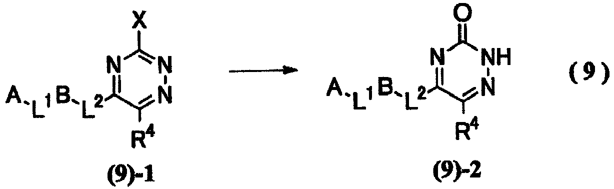

The compound (9)-2 can be synthesized by hydrolysis of compound (9)-1 with a base such as sodium hydroxide or an acid such as acetic acid in an appropriate solvent or without solvent at from 0°C to a heat-reflux temperature.

### Raw material synthesis 2

Compound (10)-3 can be produced by the production method of scheme (10) below, for example (in the scheme, X is a halogen atom, L² is a single bond, NR², O, S, SO, or SO₂; and B is a 3 to 11-membered heterocyclylene group containing NH or a 5 to 10-membered heteroarylene group containing NH when L² is a single bond).

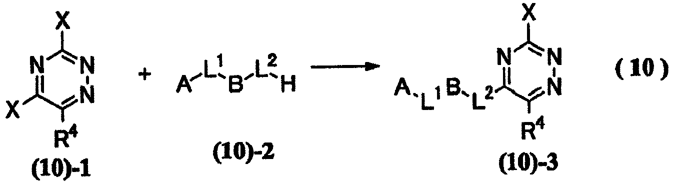

The compound (10)-3 can be synthesized using compound (10)-1 and an equal amount or excess amount of compound (10)-2 in an appropriate solvent or without solvent at from 0°C to a heat-reflux temperature.

### Raw material synthesis 3

Compound (11)-3 can be produced by the production method of scheme (11) below, for example.

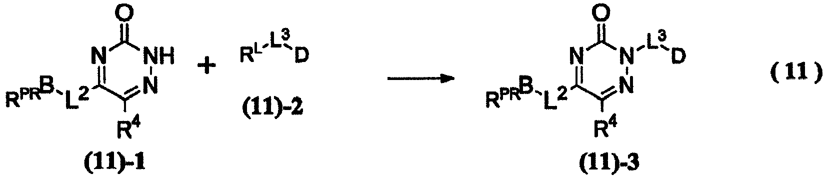

The compound (11)-3 can be obtained by reaction of compound (11)-1 and an equal amount or excess amount of compound (11)-2 in an appropriate solvent or without solvent at from 0°C to a heat-reflux temperature.

When R^{PR} is a protection group in the compound (11)-3, the compound in which R^{PR} is a hydrogen atom can be obtained by carrying out deprotecting reaction.

### Raw material synthesis 4

Compound (12)-3 can be produced by the production method of scheme (12) below, for example.

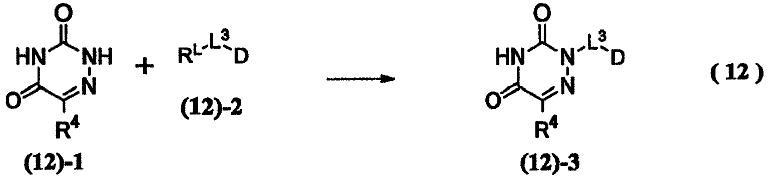

The compound (12)-3 can be obtained by reaction of compound (12)-1 and an equal amount or excess amount of compound (12)-2 in an appropriate solvent or without solvent at from 0°C to a heat-reflux temperature.

### Raw material synthesis 5

Compound (13)-2 can be produced by the production method of scheme (13) below, for example.

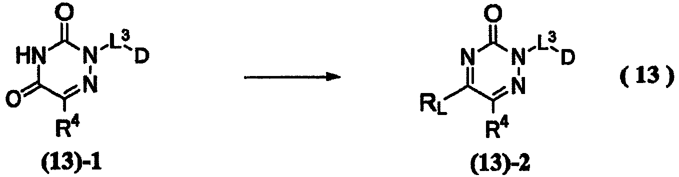

The compound (13)-2 can be synthesized from compound (13)-1 with an equal amount or excess amount of a halogenating agent such as thionyl chloride and phosphorus oxychloride in an appropriate solvent or without solvent at from 0°C to a heat-reflux temperature or synthesized from the compound (13)-1 with a sulfonyl chloride such as p-toluenesulfonyl chloride and methanesulfonyl chloride in an appropriate solvent or without solvent at from 0°C to a heat-reflux temperature.

### Raw material synthesis 6

Compound (14)-2 can be produced by the production method of scheme (14) below, for example (in the scheme, R^{A} is a hydrogen atom, a C₁₋₆ alkyl group (the C₁₋₆ alkyl group is unsubstituted or substituted with one or more substituents independently selected from the substituent group V⁶), or a dodecyl group).

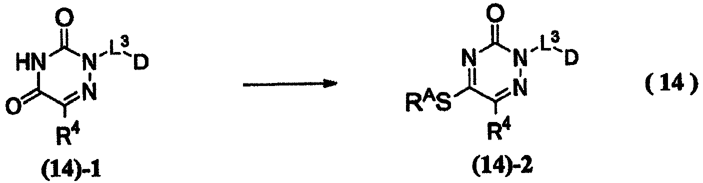

The compound (14)-2 can be obtained by reaction of compound (14)-1 and an equal amount or excess amount of a sulfur agent such as Lawesson's reagent in an appropriate solvent or without solvent at from 0°C to a heat-reflux temperature.

### Raw material synthesis 7

Compound (15)-2 can be produced by the production method of scheme (15) below, for example (in the scheme, R^{A} is a hydrogen atom, a C₁₋₆ alkyl group (the C₁₋₆ alkyl group is unsubstituted or substituted with one or more substituents independently selected from the substituent group V⁶), or a dodecyl group).

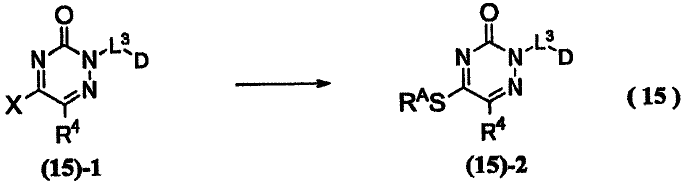

The compound (15)-2 can be obtained by reaction of compound (15)-1 and an equal amount or excess amount of a sulfur agent such as methanethiol and thiourea in an appropriate solvent or without solvent at from 0°C to a heat-reflux temperature.

### Raw material synthesis 8

Compound (16)-2 can be produced by the production method of scheme (16) below, for example (in the scheme, R^{A} is a hydrogen atom, a C₁₋₆ alkyl group (the C₁₋₆ alkyl group is unsubstituted or substituted with one or more substituents independently selected from the substituent group V⁶), or a dodecyl group; and n is 1 or 2).

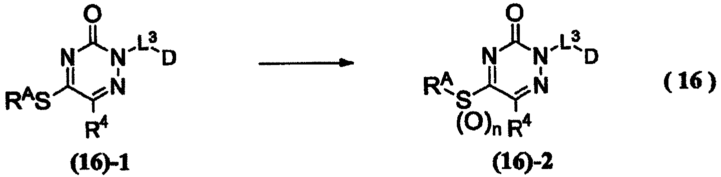

The compound (16)-2 can be obtained by reaction of compound (16)-1 and an equal amount or excess amount of an oxidizing agent such as meta-chloroperbenzoic acid and oxone in an appropriate solvent or without solvent at from 0°C to a heat-reflux temperature.

### Raw material synthesis 9

Compound (17)-3 can be produced by the production method of scheme (17) below, for example.

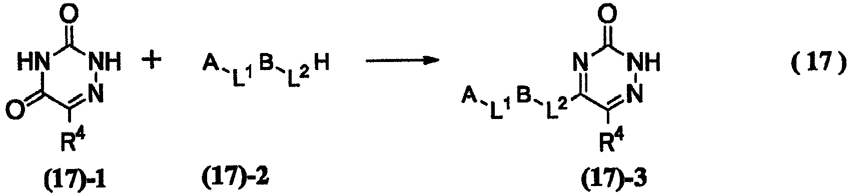

The compound (17)-3 can be obtained by reaction of compound (17)-1 and an equal amount or excess amount of compound (17)-2 in the presence of an equal amount or excess amount of a silylating agent such as N,O-bis(trimethylsilyl)acetamide or hexamethyldisilazane in an appropriate solvent or without solvent at from 0°C to a heat-reflux temperature.

### Raw material synthesis 10

Compound (18)-2 can be produced by the production method of scheme (18) below, for example.

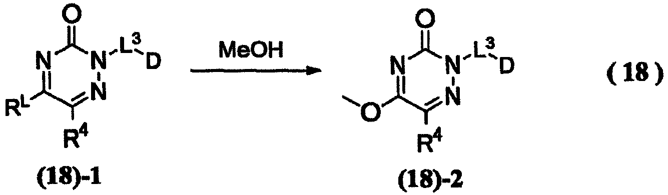

The compound (18)-2 can be obtained by reaction of compound (18)-1 and an equal amount or excess amount of methanol in an appropriate solvent or without solvent at from 0°C to a heat-reflux temperature.

### Raw material synthesis 11

Compound (19)-3 can be produced by the production method of scheme (19) below, for example.

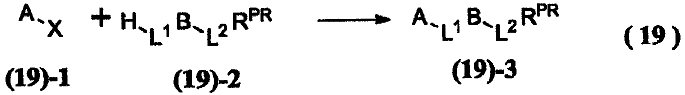

The compound (19)-3 can be obtained by reaction of compound (19)-1 and an equal amount or excess amount of compound (19)-2 in the presence of a palladium catalyst such as tetrakis(triphenylphosphine)palladium (0), bis(triphenylphosphine)palladium (II) dichloride or bis(acetonitrile)palladium (II) dichloride in an appropriate solvent or without solvent at from 0°C to a heat-reflux temperature. Alternatively, the reaction can be carried out under reaction conditions used for Buchwald-Hartwig reaction (see Advanced Synthesis & Catalysis, 2004,346, pp.1599-1626, for example). Although the reaction conditions are not particularly limited, tris(dibenzylideneacetone)dipalladium (0), tetrakis(triphenylphosphine)palladium (0), palladium (II) acetate or the like may be appropriately combined with 4,5-bis(diphenylphosphino)-9,9-dimethylxanthene (Xantphos), 2-dicyclohexylphosphino-2',6'-dimethoxybiphenyl (SPhos), 2-dicyclohexylphosphino-2',4',6'-triisopropylbiphenyl (XPhos), or the like.

When R^{PR} is a protection group in the compound (19)-3, the compound in which R^{PR} is a hydrogen atom can be obtained by carrying out deprotecting reaction.

### Raw material synthesis 12

Compound (20)-3 can be produced by the production method of scheme (20) below, for example (in the scheme, A is a 3 to 13-membered non-aromatic heterocycle containing NH or a 5 to 10-membered aromatic heterocycle containing NH).

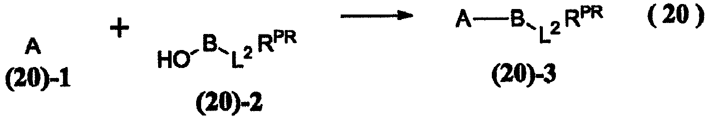

The compound (20)-3 can be synthesized by reaction of compound (20)-1 and an equal amount or excess amount of compound (20)-2 using Mitsunobu reagent and a phosphine reagent in an appropriate solvent or without solvent at from -78°C to a heat-reflux temperature. Examples of Mitsunobu reagent include diethyl azodicarboxylate and diisopropyl azodicarboxylate and examples of the phosphine reagent include triphenylphosphine and tributylphosphine.

When R^{PR} is a protection group in the compound (20)-3, the compound in which R^{PR} is a hydrogen atom can be obtained by carrying out deprotecting reaction.

### Raw material synthesis 13

Compound (21)-3 can be produced by the production method of scheme (21) below, for example (In the scheme, L¹ is S, NR², and O).

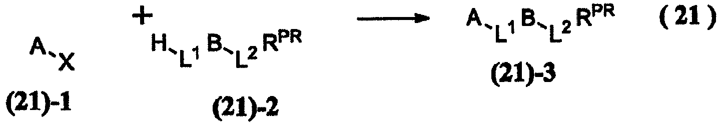

The compound (21)-3 can be synthesized by reaction of compound (21)-1 and compound (21)-2 with an equal amount or excess amount of a base such as sodium hydride and lithium hydride in an appropriate solvent or without solvent at from 0°C to a heat-reflux temperature.

When R^{PR} is a protection group in the compound (21)-3, the compound in which R^{PR} is a hydrogen atom can be obtained by carrying out deprotecting reaction.

### Raw material synthesis 14

Compound (22)-3 can be produced by the production method of scheme (22) below, for example (In the scheme, L¹ is S, NR², or O).

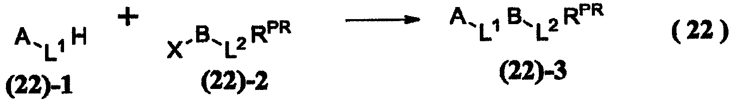

The compound (22)-3 can be obtained by reaction of compound (22)-1 and compound (22)-2.

The compound (22)-3 can be synthesized by reaction of the compound (22)-1 and the compound (22)-2 in an appropriate solvent or without solvent at from 0°C to a heat-reflux temperature.

When R^{PR} is a protection group in the compound (22)-3, the compound in which R^{PR} is a hydrogen atom can be obtained by carrying out deprotecting reaction.

### Raw material synthesis 15

Compound (23)-3 can be produced by the production method of scheme (23) below, for example. (In the scheme, E is a C₁₋₆ alkoxycarbonyl group or a carboxy group).

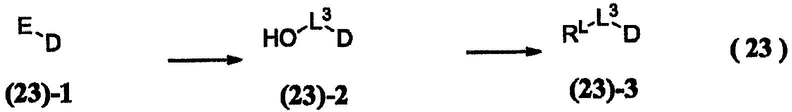

Compound (23)-2 can be synthesized from compound (23)-1 with an equal amount or excess amount of a reducing agent such as a borane-tetrahydrofuran complex, sodium borohydride, and lithium aluminum hydride in an appropriate solvent or without solvent at from 0°C to a heat-reflux temperature.

The compound (23)-3 can be synthesized from the compound (23)-2 with an equal amount or excess amount of a chlorinating agent such as thionyl chloride in an appropriate solvent or without solvent at from 0°C to a heat-reflux temperature or synthesized from the compound (23)-2 with a sulfonyl chloride such as p-toluenesulfonyl chloride and methanesulfonyl chloride in an appropriate solvent or without solvent at from 0°C to a heat-reflux temperature.

The production method is not limited to the above, and the compound of Formula (1) can be synthesized by a common method for synthesizing a triazine compound. The common method for synthesizing a triazine compound is described in the following document: Heterocyclic Compounds, New Edition, Applications (Kodansha Ltd., 2004) pp. 167 to 195.

### Synthesis method:

The compound of the present invention can be prepared in accordance with the schemes provided below and the procedures provided in Examples described below. The substituents are the same as the above unless otherwise defined or obvious to a person skilled in the art.

Novel compounds of the present invention can be easily synthesized through techniques known to a person skilled in the art, for example, described in Advanced Organic Chemistry, March, the fifth edition, John Wiley and Sons, New York, NY, 2001; Advanced Organic Chemistry, Carey and Sundberg, Vols. A and B, the third edition, Plenum Press, Inc., New York, NY, 1990; Protective groups in Organic Synthesis, Green and Wuts, the second edition, John Wiley and Sons, New York, NY, 1991; Comprehensive Organic Transformations, Larock, VCH Publishers, Inc., New York, NY, 1988; Handbook of Heterocyclic Chemistry, Katritzky and Pozharskii, the second edition, Pergamon, New York, NY, 2000; and reference documents cited therein. Other reference documents for the synthesis of novel compounds in the present invention include Buckwald et al., Tetrahedron, 2004, Vol. 60, pp. 7397-7403; Li et al., Tetrahedron Lett., 2004, Vol. 45, pp. 4257-4260; and Jean et al., J. Org. Chem., 2004, Vol. 69, pp. 8893-8902. Starting materials for the compounds can be prepared by standard synthetic conversions of chemical precursors that are easily available from commercial suppliers including Aldrich Chemical Co. (Milwaukee, WI); Sigma Chemical Co. (St. Louis, MO); Lancaster Synthesis (Windham, N.H.); Ryan Scientific (Columbia, S.C.); Maybridge (Cornwall, UK); Matrix Scientific (Columbia, S.C.); Arcos (Pittsburgh, PA); and Trans World Chemicals (Rockville, MD).

The procedures for synthesizing compounds described in the present specification can include one or a plurality of steps of protection and deprotection of functional groups and purification (for example, recrystallization, distillation, column chromatography, flash chromatography, medium-pressure column chromatography, thin-layer chromatography (TLC), radial chromatography, supercritical fluid chromatography (SFC), and high-pressure liquid chromatography (HPLC)). A product can be characterized by various techniques well-known in the chemical field, including proton and carbon-13 nuclear magnetic resonance (1H and 13C NMR), infrared and ultraviolet spectroscopy (IR and UV), X-ray crystallography, elemental analysis, and HPLC-mass analysis (HPLC-MS). The procedures for the protection and deprotection of functional groups and the methods of purification, structure identification, and quantitative determination are well-known to a person skilled in the chemical synthesis.

Solvents preferably used for the synthesis of the compound by the reactions at least partially dissolve one or all of reactants and do not disadvantageously react with any one of reactants or reaction products. Specific examples of the preferred solvent include aromatic hydrocarbons (for example, toluene and xylene), halogenated solvents (for example, methylene chloride, chloroform, carbon tetrachloride, and chlorobenzene), ethers (for example, diethyl ether, diisopropyl ether, tert-butyl methyl ether, diglyme, tetrahydrofuran, dioxane, and anisole), nitriles (for example, acetonitrile and propionitrile), ketones (for example, 2-butanone, diethyl ketone, and tert-butyl methyl ketone), alcohols (for example, methanol, ethanol, propanol, 2-propanol, n-butanol, and t-butanol), N,N-dimethylformamide (DMF), dimethyl sulfoxide (DMSO), and water. Mixtures of two or more of the solvents may also be used.

Examples of the base preferably used for the synthesis of the compound of the present invention typically include alkali metal hydrides and alkaline earth metal hydrides (for example, lithium hydride, sodium hydride, potassium hydride, and calcium hydride), alkali metal amides (for example, lithium diisopropylamide (LDA), lithium hexamethyldisilazide (LHMDS), potassium hexamethyldisilazide (KHMDS), lithium amide, sodium amide, and potassium amide), alkali metal carbonates and alkaline earth metal carbonates (for example, lithium carbonate, sodium carbonate, cesium carbonate, sodium hydrogen carbonate, and cesium hydrogen carbonate), alkali metal alkoxides and alkaline earth metal alkoxides (for example, sodium methoxide, sodium ethoxide, potassium tert-butoxide, and magnesium ethoxide), alkali metal alkyls (for example, methyllithium, n-butyllithium, sec-butyllithium, t-butyllithium, and phenyllithium), alkyl magnesium halides, organic bases (for example, trimethylamine, triethylamine, triisopropylamine, N,N-diisopropylethylamine, piperidine, N-methylpiperidine, morpholine, N-methylmorpholine, pyridine, collidine, lutidine, and 4-dimethylaminopyridine), and bicyclic amines (for example, 1,8-diazabicyclo[5.4.0]undec-7-ene (DBU) and 1,4-diazabicyclo[2.2.2]octane (DABCO)).

Functional groups of the compounds shown in the schemes below can be treated by a standard functional group conversion technique operable by a person skilled in the art, yielding an intended compound according to the present invention, if appropriate.

Other variations and modifications are obvious to a person skilled in the art and are included in the scope and teaching of the present invention. The present invention is not limited except the description in the claims below.

### [Examples]

The present invention will be described in detail with reference to Reference Synthesis Examples, Synthesis Examples, Test Examples, and Formulation Examples below. The present invention, however, is not limited to these Examples.

In Examples, NMR means a nuclear magnetic resonance spectrum, LC/MS means liquid chromatography/mass spectrometry, (v/v) means (volume/volume), and Rf and Ex described below mean Reference Synthesis Example and Synthesis Example, respectively.

### Morphology means a shape.

When ¹H-NMR data is described, the data is measured at 300 MHz (JNM-ECP300; manufactured by JEOL Ltd. or JNM-ECX300; manufactured by JEOL Ltd.) and indicates chemical shifts δ (unit: ppm) (split patterns and integral values) of signals determined by using tetramethylsilane as an internal standard. In Reference Synthesis Examples 12 to 15, however, the data is measured at 400 MHz (Avance 400 MHz; manufactured by Bruker Corporation) and indicates chemical shifts δ (unit: ppm) (split patterns and integral values) of signals determined by using tetramethylsilane as an internal standard. "s" means a singlet, "d" means a doublet, "t" means a triplet, "q" means a quartet, "quint" means a quintet, "sextet" means a sextet, "septet" means a septet, "dd" means a double doublet, "ddd" means a double double doublet, "m" means a multiplet, "br" means broad, "J" means a coupling constant, "CDCl₃" means deuterated chloroform, and "DMSO-d₆" means deuterated dimethyl sulfoxide.

As a micro-wave reactor, Initiator Sixty manufactured by Biotage Gb Ltd. was used.

In the purification by silica gel column chromatography, any one of Hi-Flash column manufactured by Yamazen Ltd., Silica gel 60 manufactured by Merck & Co., Inc., or PSQ60B manufactured by Fuji Silysia Chemical Ltd. was used, unless otherwise stated.

In the purification by silica gel (amino-based) column chromatography, Hi-Flash Amino Column manufactured by Yamazen Ltd. or DM1020 manufactured by Fuji Silysia Chemical Ltd. was used, unless otherwise stated.

In the purification by silica gel thin-layer chromatography, PLC Plate manufactured by Merck & Co., Inc. was used, unless otherwise stated.

In the purification by amino-based thin-layer chromatography, PLCP5 Plates NH manufactured by Fuji Silysia Chemical Ltd. was used, unless otherwise stated.

In the purification by preparative high-performance liquid chromatography, Shimadzu 6A Preparative High-performance Liquid Chromatography System was used, unless otherwise stated.

LC/MS was measured using an ESI (electrospray ionization) method under following conditions. "ESI⁺" means an ESI positive ion mode, "ESI⁻" means an ESI negative ion mode, "LC/MS: cond." means analysis conditions of LC/MS, and "RT" means a retention time.

LC/MS Conditions 1:
Apparatus: Waters Micromass ZQ
Column: Waters SunFire C18 (3.5 µm, 4.6x20 mm)
Column temperature: 40°C

### Solvents used

Solution A: 0.1% formic acid aqueous solution
Solution B: 0.1% formic acid - acetonitrile solution

### Elution conditions used:

The measurement was started at a flow rate of 0.4 mL/min and a mixing ratio of the solution A and the solution B of 90/10 (v/v), and then the mixing ratio of the solution A and the solution B was linearly changed to 15/85 (v/v) in 3 minutes.

Thereafter, the mixing ratio of the solution A and the solution B was fixed at 15/85 (v/v) for 2 minutes, and then the mixing ratio of the solution A and the solution B and the flow rate were linearly changed to 90/10 (v/v) and 0.5 mL/min, respectively, in 0.5 minutes. Thereafter these conditions were fixed for 2.5 minutes.

LC/MS Conditions 2
Apparatus: Thermo LTQ XL
Column: Waters AQUITY UPLC BEH C18 (1.7 µm, 2.1x50 mm)
Column temperature: 40°C

### Solvents used

Solution A: 0.1% formic acid aqueous solution
Solution B: 0.1% formic acid - acetonitrile solution

### Elution conditions used:

Conditions were fixed at a flow rate of 0.6 mL/min and a mixing ratio of the solution A and the solution B of 90/10 (v/v) and the measurement was started, and then, after 0.5 minutes, the mixing ratio of the solution A and the solution B was linearly changed to 10/90 (v/v) in 2.5 minutes.

Thereafter, the mixing ratio of the solution A and the solution B was fixed at 10/90 (v/v) for 0.7 minutes, and then the mixing ratio of the solution A and the solution B and the flow rate were linearly changed to 90/10 (v/v) and 0.8 mL/min, respectively, in 0.1 minutes, followed by fixing these conditions for 1 minute.

Thereafter, the mixing ratio of the solution A and the solution B was fixed at 90/10 (v/v) and the flow rate was linearly changed to 0.6 mL/min in 0.1 minutes.

### LC/MS Conditions 3

Apparatus: Waters Micromass ZQ 2000
Column: Waters SunFire C18 (3.5 µm, 2.1x20 mm)
Column temperature: 40°C

### Solvents used

Solution A: Acetonitrile solution
Solution A: 0.1% formic acid aqueous solution

### Elution conditions used:

Conditions were fixed at a flow rate of 0.4 mL/min and a mixing ratio of the solution A and the solution B of 15/85 (v/v) and the measurement was started, and then the mixing ratio of the solution A and the solution B was linearly changed to 85/15 (v/v) in 3 minutes.

Thereafter, the mixing ratio of the solution A and the solution B was fixed at 15/85 (v/v) for 2 minutes, and then the mixing ratio of the solution A and the solution B was linearly changed to 95/5 (v/v) in 0.5 minutes. Thereafter these conditions were fixed for 1.5 minutes.

### Reference Synthesis Example 1

### 5-Chloro-2-(4-chlorobenzyl)-1,2,4-triazin-3(2H)-one

To a toluene solution (2.0 mL) of 2-(4-chlorobenzyl)-1,2,4-triazine-3,5(2H, 4H)-dione (100 mg, 0.431 mmol), phosphorus oxychloride (196 µL, 2.10 mmol) and diisopropylethylamine (161 µL, 0.926 mmol) were added and the resultant reaction solution was stirred at 100°C for 7 hours. After completion of the reaction, the reaction solution was concentrated under reduced pressure to obtain the crude product of the title compound (108 mg, quantitative).

### Reference Synthesis Example 2

### 2-(4-methoxybenzyl)-1,2,4-triazine-3,5(2H,4H)-dione

To an acetonitrile solution (20 mL) of 1,2,4-triazine-3,5(2H,4H)-dione (1.00 g, 8.84 mmol), N,O-bis(trimethylsilyl)acetamide (4.33 mL, 17.7 mmol) was added and the resultant reaction solution was stirred at 80°C for 2 hours. To the reaction solution, 1-(chloromethyl)-4-methoxy-benzene (1.44 mL, 10.6 mmol) and sodium iodide (265 mg, 1.77 mmol) were added and the resultant reaction solution was stirred at 80°C for 8 hours. After completion of the reaction, water was added to the reaction solution and the resultant mixture was concentrated. The obtained residue was washed with ethyl acetate and diethyl ether to obtain the title compound (1.27 g, yield 61 %) as a yellow solid.

### Reference Synthesis Example 3

### 5-Chloro-2-(4-methoxybenzyl)-1,2,4-triazin-3(2H)-one

To a toluene solution (18 ml) of
2-(4-methoxybenzyl)-1,2,4-triazine-3,5(2H,4H)-dione (600 mg, 2.57 mmol) synthesized in Reference Synthesis Example 2, phosphorus oxychloride (528 µL, 5.66 mmol) and diisopropylethylamine (986 µL, 5.66 mmol) were added and the resultant reaction solution was stirred at 70°C for 4 hours. The reaction solution was concentrated under reduced pressure to obtain the crude product of the title compound. The crude product was used as it was in the next process.

### Reference Synthesis Example 4

### 5-[4-(4-Fluorophenyl)piperazin-1-yl]-2-(4-methoxybenzyl)-1,2,4-triazin-3(2H)-one

To a dimethylformamide solution (12 ml) of the crude product of 5-chloro-2-(4-methoxybenzyl)-1,2,4-triazin-3(2H)-one synthesized in Reference Synthesis Example 3, triethylamine (461 µL, 3.31 mmol) and 1-(4-fluorophenyl)piperazine (596 mg, 3.31 mmol) were added and resultant reaction solution was stirred at 80°C for 3 hours. After completion of the reaction, water was added to the reaction solution and the resultant mixture was extracted with chloroform. The organic layer was dried over anhydrous magnesium sulfate and concentrated under reduced pressure. The obtained residue was washed with a mixed liquid of isopropyl ether/2-propanol to obtain the title compound (815 mg, yield 80%) as a brown solid.

### Reference Synthesis Example 5

### 5-[4-(4-Fluorophenyl)piperazin-1-yl)-2-(4-hydroxybenzyl)-1,2,4-triazin-3(2H)-one

To a methylene chloride solution (9.3 mL) of
5-[4-(4-fluorophenyl)piperazin-1-yl]-2-(4-methoxybenzyl)-1,2,4-triazin-3(2H)-one (930 mg, 2.35 mmol) synthesized in Reference Synthesis Example 4, a methylene chloride solution of 17% boron tribromide (7.06 mL, 7.06 mmol) was added and the resultant reaction solution was stirred at 0°C for 4 hours. The reaction was terminated by adding methanol to the reaction solution. Thereafter, the resultant mixture was neutralized by adding 1 mol/L sodium hydroxide aqueous solution and the obtained mixture was extracted with chloroform. The organic layer was dried over anhydrous magnesium sulfate and concentrated under reduced pressure to obtain the title compound (881 mg, yield 98%) as a brown solid.

### Reference Synthesis Example 6

### 2-(Chloromethyl)-5-(trifluoromethyl)thiophene

To a methylene chloride solution (4.0 mL) of
[5-(trifluoromethyl)thiophene-2-yl]methanol (200 mg, 1.10 mmol), thionyl chloride (119 µL, 1.65 mmol) was added and the resultant reaction solution was stirred at room temperature for 6 hours. After completion of the reaction, the reaction solution was concentrated under reduced pressure to obtain the crude product (213 mg, yield 97%) of the title compound as a colorless oily product.

### Reference Synthesis Example 7

### 2-{[5-(Trifluoromethyl)thiophen-2-yl]methyl}-1,2,4-triazine-3,5(2H,4H)-dione

The crude product of 2-(chloromethyl)-5-(trifluoromethyl)thiophene (213 mg, 1.06 mmol) synthesized in Reference Synthesis Example 6 was used to obtain the title compound (106 mg, yield 43%) as a yellow solid by synthesis in a similar manner to Reference Synthesis Example 2.

### Reference Synthesis Example 8

### 5-Chloro-2-{[5-(trifluoromethyl)thiophen-2-yl]methyl}-1,2,4-triazin-3(2H)-one

2-([5-(Trifluoromethyl)thiophen-2-yl]methyl)-1,2,4-triazine-3,5(2H,4H)-dione (106 mg, 0.382 mmol) synthesized in Reference Synthesis Example 7 was used to obtain the title compound (113 mg, quantitative) as a brown oily product by synthesis in a similar manner to Reference Synthesis Example 3.

### Reference Synthesis Example 9

### 5-Mercapto-2-{[5-(trifluoromethyl)thiophen-2-yl]methyl}-1,2,4-triazin-3(2H)-one

To a 1,4-dioxane solution (1.1 mL) of
2-{[5-(trifluoromethyl)thiophen-2-yl]methyl}-1,2,4-triazine-3,5(2H,4H)-dione (111 mg, 0.400 mmol) synthesized in Reference Synthesis Example 7, Lawesson's reagent (162 mg, 0.400 mmol) was added and the resultant reaction solution was stirred for 40 minutes under reflux by heating. The reaction solution was concentrated under reduced pressure and the obtained residue was purified by silica gel column chromatography (hexane/ethyl acetate) to obtain the title compound (110 mg, yield 94%) as an orange color solid.

### Reference Synthesis Example 10

### 2-{[2-(Trifluoromethyl)thiazol-5-yl]methy}-1,2,4-triazine-3,5(2H,4H)-dione

5-(Bromomethyl)-2-(trifluoromethyl)thiazole (466 mg, 1.89 mmol) was used to obtain the title compound (153 mg, yield 44%) as a light yellow solid by synthesis in a similar manner to Reference Synthesis Example 2.

### Reference Synthesis Example 11

### 5-Mercapto-2-{[2-(trifluoromethyl)thiazol-5-yl]methyl}-1,2,4-triazin-3(2H)-one

2-{[2-(Trifluoromethyl)thiazol-5-yl]methyl}-1,2,4-triazine-3,5(2H,4H)-dione (153 mg, 0.556 mmol) synthesized in Reference Synthesis Example 10 was used to obtain the title compound (135 mg, yield 83%) as an orange color solid by synthesis in a similar manner to Reference Synthesis Example 9.

### Reference Synthesis Example 12

### 2-{[5-Trifluoromethyl)furan-2-yl]methyl}-1,2,4-triazine-3,5(2H,4H)-dione

2-(Bromomethyl)-5-(trifluoromethyl)furan (243 mg, 1.06 mmol) was used to obtain the title compound (100 mg, yield 43%) as a white solid by synthesis in a similar manner to Reference Synthesis Example 2.

### Reference Synthesis Example 13

### 5-Mercapto-2-{[5-trifluoromethyl)furan-2-yl]methyl}-1,2,4-triazin-3(2H)-one

2-{[5-(Trifluoromethyl)furan-2-yl]methyl}-1,2,4-triazine-3,5(2H,4H)-dione (100 mg, 0.382 mmol) synthesized in Reference Synthesis Example 12 was used to obtain the title compound (100 mg, yield 94%) as an orange color solid by synthesis in a similar manner to Reference Synthesis Example 9.

### Reference Synthesis Example 14

### 2-{[6-(Difluoromethoxy)pyridin-3-yl]methyl}-1,2,4-triazine-3,5(2H,4H)-dione

5-(Chloromethyl)-2-(difluoromethoxy)pyridine (1.50 g, 7.75 mmol) was used to obtain the title compound (500 mg, yield 24%) as a white solid by synthesis in a similar manner to Reference Synthesis Example 2.

### Reference Synthesis Example 15

### 2-{[6-(Difluoromethoxy)pyridin-3-yl]methyl}-5-mercapto-1,2,4-triazin-3(2H)-one

2-{[6-(Difluoromethoxy)pyridin-3-yl]methyl}-1,2,4-triazine-3,5(2H,4H)-dione (150 mg, 0.555 mmol) synthesized in Reference Synthesis Example 14 was used to obtain the title compound (120 mg, yield 76%) as a yellow solid by synthesis in a similar manner to Reference Synthesis Example 9.

### Reference Synthesis Example 16

### [2-(Tert-butyl)oxazol-5-yl]methanol

[2-(Tert-butyl)oxazol-5-yl]methyl acetate (303 mg, 1.54 mmol) was dissolved in methanol (3.0 mL) and potassium carbonate (425 mg, 3.08 mmol) was added thereto, followed by stirring the resultant reaction solution at room temperature for 3 days. The reaction solution was concentrated under reduced pressure and the resultant residue was extracted with ethyl acetate. The ethyl acetate layer was concentrated under reduced pressure to obtain the crude product (213 mg, yield 89%) of the title compound as a light orange color oily product.

### Reference Synthesis Example 17

### 2-(Tert-butyl)-5-(chloromethyl)oxazole

The roughly purified product of [2-(tert-butyl)oxazol-5-yl]methanol (239 mg, 1.54 mmol) synthesized in Reference Synthesis Example 16 was dissolved in methylene chloride (1.5 mL) and thionyl chloride (223 µL, 3.08 mmol) was added thereto, followed by stirring the resultant reaction solution at room temperature for 16 hours. Toluene was added to the reaction solution and the resultant mixture was concentrated under reduced pressure to obtain the crude product (206 mg, yield 77%) of the title compound as a light yellow oily product.

### Reference Synthesis Example 18

### (S)-Tert-butyl 4-(4-fluorophenyl)-5-hydroxy-5,6-dihydropyridine-1(2H)-carboxylate (S)-Tert-butyl

4-(4-Fluorophenyl)-5-(pivaloyloxy)-5,6-dihydropyridine-(2H)-carboxylate (69.5 g, 184 mmol) was dissolved in a mixed solvent of dioxane (350 g) and water (69 g) and lithium hydroxide monohydrate (23.2 g, 552 mmol) was added thereto, followed by stirring the resultant reaction solution at 90°C for 7 hours. The reaction solution was cooled to room temperature and ethyl acetate (360 g) was added thereto. The resultant mixture was sequentially washed with water (280 g) and brine (280 g) and dried over anhydrous magnesium sulfate. The magnesium sulfate was removed by filtration and the organic layer was concentrated under reduced pressure. The resultant residue was purified by silica gel column chromatography (hexane/ethyl acetate) to obtain the title compound (49.0 g, yield 91%) as a yellow solid.

### Reference Synthesis Example 19

### Tert-butyl 5-fluoro-4-(4-fluorophenyl)-5,6-dihydropyridine-1(2H)carboxylate

N,N-Diethylaminosulfur trifluoride (2.00 mL, 15.1 mmol) was dissolved in methylene chloride (20 mL) and trimethylsilylmorpholine (2.72 mL, 15.3 mmol) was added thereto at -78°C, followed by stirring the reaction solution for 2 hours while raising the temperature of the reaction solution to room temperature. The reaction solution was cooled to -78°C and (S)-tert-butyl
4-(4-fluorophenyl)-5-hydroxy-5,6-dihydropyridine-1(2H)-carboxylate (1.36 g, 4.64 mmol) synthesized in Reference Synthesis Example 18 was added thereto, followed by stirring the resultant reaction solution for 2 hours while raising the temperature of the reaction solution to room temperature. The reaction solution was cooled with ice and saturated sodium bicarbonate aqueous solution (14 mL) was added thereto, followed by extracting the resultant mixture with methylene chloride. The organic layer was concentrated under reduced pressure to obtain the roughly purified product (2.14 g, quantitative) of the title compound as an orange oily product.

### Reference Synthesis Example 20

### 3-Fluoro-4-(4-fluorophenyl)-1,2,3,6-tetrahydropyridine

Tert-butyl 5-fluoro-4-(4-fluorophenyl)-5,6-dihydropyridine-1(2H)-carboxylate (1.34 g, 4.56 mmol) synthesized in Reference Synthesis Example 19 was dissolved in dioxane (6.8 mL) and 4 N hydrochloric acid/dioxane solution (9.28 mL, 27.3 mmol) was added thereto under cooling with ice, followed by stirring the resultant reaction solution at 0°C for 2 hours. Sodium hydroxide solution was added dropwise to the reaction solution to adjust pH to 10 and the resultant mixture was extracted with ethyl acetate (30 mL) three times. The organic layer was concentrated under reduced pressure and the obtained residue was sequentially purified by silica gel (amino-based) column chromatography and silica gel column chromatography to obtain the title compound (663 mg, yield 73%) as a light yellow oily product.

### Reference Synthesis Example 21

### 6-Hydroxy-1-[(2-nitrophenyl)sulfonyl]-1,6-dihydropyridin-3(2H)-one

To a tetrahydrofuran solution (40 mL) of
N-(furan-2-ylmethyl)-2-nitrobenzenesulfonamide (8.02 g, 28.3 mmol), sodium acetate (2.32 g, 28.3 mmol), and water (16 g) were added at room temperature and the resultant reaction solution was cooled with ice. To the reaction solution, N-bromosuccinimide (5.05 g, 28.3 mmol) was added and the resultant reaction solution was stirred for 1 hour. To the reaction solution, sodium thiosulfate (2.24 g, 14.2 mmol), water (4.0 mL), and sodium chloride (1.20 g) were added and the organic layer was separated. The organic layer was washed with brine two times and dried over anhydrous magnesium sulfate. The magnesium sulfate was filtered and washed with methylene chloride (12 g), followed by concentrating the organic layer to 12.3 g under reduced pressure. To the residue, methylene chloride (42 g), sodium chloride (1.20 g), and water (18 g) were added and the organic layer was extracted. The organic layer was dried over anhydrous magnesium sulfate and the anhydrous magnesium sulfate was washed with methylene chloride (8.0 g), followed by concentrating the resultant organic layer under reduced pressure to obtain the title compound as a methylene chloride solution (34.3 g). The solution was used in the next process without further purification.

### Reference Synthesis Example 22

### 1-[(2-Nitropheny)sulfonyl]-1,6-dihydropyridin-3(2H)-one

To a methylene chloride solution (5.99 g) of
6-hydroxy-1-[(2-nitrophenyl)sulfonyl]-1,6-dihydropyridin-3(2H)-one synthesized in Reference Synthesis Example 21, triethylsilane (0.780 g, 6.72 mmol), boron trifluoride-ether complex (1.51 g, 10.6 mmol), and methylene chloride (17 g) were added and the resultant reaction solution was stirred at 0°C for 4 hours. To the reaction solution, water (6.3 g) was added at 0°C and the organic layer was extracted. The organic layer was washed with saturated sodium bicarbonate aqueous solution and brine and dried over anhydrous magnesium sulfate. The magnesium sulfate was removed by filtration and washed with methylene chloride (4.2 g). The filtrate was concentrated to 2.53 g under reduced pressure and hexane (11 g) was added thereto. The upper layer was removed by decantation. The operation was repeated three times and the methylene chloride layer was concentrated under reduced pressure to obtain the methylene chloride solution (1.45 g) of the title compound. Methylene chloride (7.2 g) was added to the solution and the resultant solution was used in the next process without further purification.

### Reference Synthesis Example 23

### 4-Bromo-1-[(2-nitrophenyl)sulfonyl]-1,6-dihydropyridin-3(2H)-one

To a methylene chloride solution (4.42 g) of
1-[(2-nitrophenyl)sulfonyl]-1,6-dihydropyridin-3(2H)-one synthesized in Reference Synthesis Example 22, methylene chloride (21 g) and bromine (2.52 g, 15.8 mmol) were added and the resultant reaction solution was stirred for 3 hours under cooling with ice. To the reaction solution, triethylamine (1.52 g, 15.1 mmol) was added and the resultant reaction solution was stirred for 3 hours under cooling with ice. To the reaction solution, water (21 g) was added and the organic layer was extracted. The organic layer was sequentially washed with sodium thiosulfate aqueous solution and water and silica gel (2.15 g) was added to the organic layer. The resultant mixture was stirred at room temperature for 1 hour and filtered. The filtrate was concentrated to 4.30 g under reduced pressure and acetone (21 g) was added to the residue. The solvent of the resultant mixture was distilled away under reduced pressure so that the weight of the mixture was reduced to 6.40 g. The concentrated liquid was heated to 40°C and then cooled to room temperature. Ethanol (21 g) was added to the solution and the resultant mixture was stirred -10°C for 1 hour. The generated solid was collected by filtration. The obtained solid was washed with ethanol (4.3 g) having a temperature of -10°C and dried under reduced pressure to obtain the title compound (0.797 g, yield 29%) as a light brown solid.

### Reference Synthesis Example 24

### (S)-4-Bromo-1-[(2-nitrophenyl)sulfonyl]-1,2,3,6-tetrahydropyridin-3-ol

(R)-Diphenyl(pyrrolidin-2-yl)methanol (112 mg, 0.442 mmol) was dissolved in tetrahydrofuran (32 mL) and trimethoxy boron (0.0520 mL, 0.544 mmol) was added thereto. The resultant reaction solution was stirred for 1 hour under cooling with ice, and thereafter the temperature of the reaction solution was raised to room temperature and the reaction solution was further stirred for 1 hour. To the reaction solution, N,N-diethylaniline borane (397 mg, 2.43 mmol) was added at 0°C. To the reaction solution, 4-bromo-1-[(2-nitrophenyl)sulfonyl]-1,6-dihydropyridin-3(2H)-one (800 mg, 2.22 mmol) synthesized in Reference Synthesis Example 23 was added and the resultant reaction solution was stirred overnight at room temperature. To the reaction solution, methanol (2.0 mL) was added and the resultant mixture was concentrated under reduced pressure. The obtained residue was extracted with chloroform and the organic layer was washed with 1N hydrochloric acid and brine and dried over anhydrous magnesium sulfate. The magnesium sulfate was filtered and the organic layer was concentrated under reduced pressure. The obtained residue was subjected to silica gel column chromatography to obtain the title compound (809 mg, quantitative) as a colorless amorphous product.

### Reference Synthesis Example 25

### (R)-4-(5-Methylfuran-2-yl)-1-[(2-nitrophenyl)sulfonyl)-1,2,3,6-tetrahydropyridin-3-ol

(S)-4-Bromo-1-[(2-nitrophenyl)sulfonyl]-1,2,3,6-tetrahydropyridin-3-ol (1.50 g, 4.13 mmol) synthesized in Reference Synthesis Example 24 was dissolved in a mixed solution of 2-methyltetrahydrofuran (3.0 g) and water (15 g) and 4,4,5,5-tetramethyl-2-(5-methylfuran-2-yl)-1,3,2-dioxaborolane (988 mg, 4.75 mmol), palladium acetate (46.4 mg, 0.210 mmol), 1,1'-bis(diphenylphosphino)ferrocene (126 mg, 0.230 mmol), and potassium carbonate (2.28 g, 16.5 mmol) were added thereto, followed by stirring the resultant reaction solution at 70°C for 2 hours. The reaction solution was cooled to room temperature and filtered with Celite, and the filtrate was extracted with 2-methyltetrahydrofuran. The solution was used as it was in the next process.

### Reference Synthesis Example 26

### (R)-4-(5-Methylfuran-2-yl)-1,2,3,6-tetrahydropyridin-3-ol

To a 2-methyltetrahydrofuran solution of
(R)-4-(5-methylfuran-2-yl)-1-[(2-nitrophenyl)sulfonyl]-1,2,3,6-tetrahydropyridin-3-ol synthesized in Reference Synthesis Example 25, lithium hydroxide monohydrate (693 mg, 16.5 mmol) and dodecanethiol (1.67 g, 8.26 mmol) were added and the resultant reaction solution was stirred at 60°C for 18 hours. To the reaction solution, 1N hydrochloric acid was added and the resultant mixture was washed with methylene chloride. The water layer was alkalinized with sodium hydroxide aqueous solution and the resultant mixture was extracted with methylene chloride four times. The organic layer was concentrated and the residue was subjected to silica gel (amino-based) column chromatography to obtain the title compound (505 mg, yield 68%) as a yellow solid.

### Reference Synthesis Example 27

### (R)-4-(1-Methyl-1H-pyrazol-4-yl)-1-[2-nitrophenyl)sulfonyl-1,2,3,6-tetrahydropyridin-3-ol

(S)-4-Bromo-1-[(2-nitrophenyl)sulfonyl]-1,2,3,6-tetrahydropyridin-3-ol (1.50 g, 4.13 mmol) synthesized in Reference Synthesis Example 24 was dissolved in a mixed solution of 2-methyltetrahydrofuran (3.0 g) and water (15 g) and 1-methyl-4-(4,4,5,5-tetramethyl-1,3,2-dioxaborolan-2-yl)-1H-pyrazole (988 mg, 4.75 mmol), palladium acetate (46.4 mg, 0.210 mmol), 1,1'-bis(diphenylphosphino)ferrocene (126 mg, 0.230 mmol), and potassium carbonate (2.28 g, 16.5 mmol) were added thereto, followed by stirring the resultant reaction solution at 70°C for 2 hours. The reaction solution was cooled to room temperature and filtered with Celite. The filtrate was extracted with 2-methyltetrahydrofuran and the organic layer was dried over anhydrous magnesium sulfate. The magnesium sulfate was removed by filtration and the organic layer was concentrated under reduced pressure. The resultant residue was subjected to silica gel (amino-based) column chromatography to obtain the title compound (883 mg, yield 59%) as a light brown amorphous product.

### Reference Synthesis Example 28

### (R)-4-(1-Methyl-1H-pyrazol-4-yl)1,2,3,6-tetrahydropyridin-3-ol

(R)-4-(1-Methyl-1H-pyrazol-4-yl)-1-[(2-nitrophenyl)sulfonyl]-1,2,3,6-tetrahydropyr idin-3-ol (883 mg, 2.42 mmol)) synthesized in Reference Synthesis Example 27 was dissolved in 2-methyltetrahydrofuran (27 g) and lithium hydroxide monohydrate (406 mg, 9.68 mmol) and dodecanethiol (980 mg, 4.84 mmol) were added thereto, followed by stirring the resultant reaction solution at 60°C for 18 hours. The reaction solution was filtered and the filtrate was concentrated. The resultant residue was subjected to silica gel (amine-based) column chromatography to obtain the title compound (297 mg, yield 68%) as a yellow-brown solid.

### Reference Synthesis Example 29

### 5-[4,6-Dihydroxy-3,4-dihydroisoquinolin-2(1H)-yl]-2-{[2-(trifluoromethyl)thiazol-5-yl] methyl}-1,2,4-triazin-3(2H)-one

5-Mercapto-2-{[2-(trifluoromethyl)thiazol-5-yl]methyl}-1,2,4-triazin-3(2H)-one (162 mg, 680 µmol) synthesized in Reference Synthesis Example 11, 1,2,3,4-tetrahydroisoquinoline-4,6-diol hydrochloride (200 mg, 680 µmol), and diisopropylethylamine (176 mg, 1.36 mmol) were dissolved in dioxane (5.0 mL) and the resultant reaction solution was stirred at 110°C for 16 hours. The reaction solution was concentrated under reduced pressure and water was added to the resultant residue. The resultant mixture was extracted with ethyl acetate. The organic layer was washed with brine, dried over sodium sulfate, and then concentrated under reduced pressure. To the resultant residue, a 1:1 mixed solution (20 mL) of t-butyl methyl ether and ethyl acetate was added and the generated solid was collected by filtration to obtain the title compound (140 mg, yield 48%) as a brown solid.

### Synthesis Example 1

### 2-(4-Chlorobenzyl)-5-[4-(4-fluorophenyl)piperazin-1-yl]-1,2,4-triazin-3(2H)-one

To a dimethylformamide solution (2.0 mL) of the crude product of 5-chloro-2-(4-chlorobenzyl)-1,2,4-triazin-3(2H)-one (107.6 mg, 0.420 mmol) synthesized in Reference Synthesis Example 1, 1-(4-fluoro-phenyl)piperazine (114 mg, 0.630 mmol) and triethylamine (88.0 µL, 0.630 mmol) were added and the resultant reaction solution was stirred at 80°C for 13 hours. After completion of the reaction, water and ethyl acetate were added to the reaction solution and the precipitated solid was collected by filtration to obtain the title compound (66.8 mg, yield 97%) as a light gray solid.

### Synthesis Example 2

### 5-[4-(4-Fluorophenyl)piperazin-1-yl]-2-[4-(hexyloxy)benzyl]-1,2,4-triazin-3(2H)-one

To a N,N-dimethylformamide solution (1.0 mL) of
5-[4-(4-fluorophenyl)piperazin-1-yl]-2-(4-hydroxybenzyl)-1,2,4-triazin-3(2H)-one (39.5 mg, 0.104 mmol) synthesized in Reference Synthesis Example 5, sodium hydride (6.80 mg, 0.155 mmol) and 1-iodohexane (22.9 µL, 0.155 mmol) were added and the resultant reaction solution was stirred at room temperature for 1 hour. After completion of the reaction, water was added to the reaction solution and the resultant mixture was extracted with ethyl acetate. The organic layer was dried over anhydrous magnesium sulfate and concentrated under reduced pressure. The obtained residue was washed with a mixed solvent of ethyl acetate/hexane to obtain the title compound (38.3 mg, yield 79%) as a cream color crystal.

### Synthesis Example 3

### 2-{4-[(4-Chlorobenzyl)oxy]benzyl}-5-[4-(4-fluorophenyl)piperazin-1-yl]-1,2,4-triazin-3( 2H)-one

To a N,N-dimethylformamide solution (0.60 mL) of
5-[4-(4-fluorophenyl)piperazin-1-yl]-2-(4-hydroxybenzyl)-1,2,4-triazin-3(2H)-one (30.0 mg, 0.0787 mmol) synthesized in Reference Synthesis Example 5,
1-chloro-4-(chloromethyl)benzene (15.2 mg, 0.0944 mmol) and potassium carbonate (13.0 mg, 0.0941 mmol) were added and the resultant reaction solution was stirred overnight at room temperature. After completion of the reaction, water was added to the reaction solution and the resultant mixture was extracted with ethyl acetate. The organic layer was dried over anhydrous magnesium sulfate and then concentrated under reduced pressure. The obtained residue was purified by silica gel column chromatography (chloroform) to obtain the title compound (12.8 mg, yield 32%) as a colorless crystal.

### Synthesis Example 4

### 2-{4-[(4-Fluorobenzyl)oxy]benzyl}-5-[4-(4-fluorophenyl)piperazin-1-yl]-1,2,4-triazin-3( 2H)-one

1-Fluoro-4-(chloromethyl)benzene (13.6 mg, 0.0941 mmol) was used to obtain the title compound (26.3 mg, yield 68%) as a colorless solid by synthesis in a similar manner to Example 3.

### Synthesis Example 5

### 5-[4-(4-Fluorophenyl)piperazin-1-yl]-2-{[5-(trifluoromethyl)thiophen-2-yl]methyl}-1,2,4 -triazin-3(2H)-one

To a dioxane solution (0.50 mL) of
5-mercapto-2-{[5-(trifluoromethyl)thiophen-2-yl]methyl}-1,2,4-triazin-3(2H)-one (110 mg, 0.375 mmol) synthesized in Reference Synthesis Example 9, 1-(4-fluoro-phenyl)piperazine (79.3 mg, 0.440 mmol) was added and the resultant reaction solution was stirred for 1 hour under reflux by heating. After completion of the reaction, the reaction solution was concentrated under reduced pressure and the resultant residue was purified by silica gel column chromatography (chloroform/ethyl acetate) to obtain the title compound (153 mg, yield 93%) as a light yellow solid.

### Synthesis Example 6

### 5-[4-(4-Fluorophenyl)-5,6-dihydropyridine-(2H)-yl]-2-{[5-(trifluoromethyl)thiophen-2-yl]methyl}-1,2,4-triazin-3(2H)-one

To a dimethylformamide solution (3.0 mL) of
5-chloro-2-{[5-(trifluoromethyl)thiophen-2-yl]methyl}-1,2,4-triazin-3(2H)-one (113 mg, 0.382 mmol) synthesized in Reference Synthesis Example 8, 4-(4-fluorophenyl)-1,2,3,6-tetrahydropyridine (98.0 mg, 0.459 mmol) and triethylamine (64.0 µL, 0.459 mmol) were added and the resultant reaction solution was stirred at room temperature for 4 days. After completion of the reaction, water was added to the reaction solution and the resultant mixture was extracted with ethyl acetate. The organic layer was dried over anhydrous magnesium sulfate and concentrated under reduced pressure. The obtained residue was purified by silica gel column chromatography (hexane/ethyl acetate). The obtained crude product was washed with a mixed solvent of ethyl acetate/diisopropyl ether to obtain the title compound (18.8 mg, yield 11%) as a colorless solid.

### Synthesis Example 7

### (R)-5-[4-Fluorophenyl)-5-hydroxy-5,6-dihydropyridin-1(2H)-yl]-2-{[5-trifluorometh yl)thiophene-2-yl]methyl}-1,2,4-triazin-3(2H)-one

(R)-4-(4-Fluorophenyl)-1,2,3,6-tetrahydropyridin-3-ol (50.0 mg, 0.259 mmol) was used to obtain the title compound (5.85 mg, yield 5%) as a light yellow solid by synthesis in a similar manner to Synthesis Example 6.

### Synthesis Example 8

### 5-[4-(5-methylpyridin-2-yl)piperazin-1-yl]-2-{[5-(trifluoromethyl)thiophene-2-yl]methyl }-1,2,4-triazin-3(2H)-one

1-(5-Methylpyridin-2-yl)piperazine (73.1 mg, 0.413 mmol) was used to obtain the title compound (140 mg, yield 86%) as a yellow solid by synthesis in a similar manner to Synthesis Example 5.

### Synthesis Example 9

### 5-(7-Fluoro-3,4-dihydro-1H-pyrido[3,4-b]indol-2(9H)-yl)-2-{[5-(trifluoromethyl)thiophe n-2-yl]methyl}-1,2,4-triazin-3(2H)-one

7-Fluoro-2,3,4,9-tetrahydro-1H-pyrido[3,4-b]indole (98.0 mg, 0.515 mmol) was used to obtain the title compound (166 mg, yield 72%) as a colorless solid by synthesis in a similar manner to Example 5.

### Synthesis Example 10 5-[4-(4-Fluorophenyl)piperazin-1-yl]-2-{[2-(trifluoromethyl)thiazol-5-yl]methyl}-1,2,4-tr iazin-3(2H)-one

To a 1,4-dioxane solution (0.60 mL) of
5-mercapto-2-{[2-(trifluoromethyl)thiazol-5-yl]methyl}-1,2,4-triazin-3(2H)-one (134 mg, 0.456 mmol) synthesized in Reference Synthesis Example 11,
1-(4-fluorophenyl)piperazine (90.4 mg, 0.502 mmol) was added and the resultant reaction solution was stirred for 3 hours under reflux by heating. The reaction solution was concentrated under reduced pressure and the obtained residue was purified by silica gel column chromatography (hexane/ethyl acetate) to obtain the title compound (145 mg, yield 72%) as a colorless solid.

### Synthesis Example 11

### (R)-5-[4-(4-Fluorophenyl)-5-hydroxy-5,6-dihydropyridin-1(2H)-yl]-2-{[2-(trifluorometh yl)thiazol-5-yl]methyl}-1,2,4-triazin-3(2H)-one

(R)-4-(4-Fluorophenyl)-1,2,3,6-tetrahydropyridin-3-ol (148 mg, 0.766 mmol) was used to obtain the title compound (160 mg, yield 69%) as a colorless solid by synthesis in a similar manner to Example 10.

### Synthesis Example 12

### (R)-5-[5-Hydroxy-4-(5-methylthiophen-3-yl)-5,6-dihydropyridin-1(2)-yl]-2-{[2-(trifluo romethyl)thiazol-5-yl]methyl}-1,2,4-triazin-3(2H)-one

(R)-4-(5-Methylthiophen-3-yl)-1,2,3,6-tetrahydropyridin-3-ol (88.0 mg, 0.451 mmol) was used to obtain the title compound (141 mg, yield 69%) as a brown solid by synthesis in a similar manner to Example 10.

### Synthesis Example 13

### 5-[4-(5-Methylpyridin-2-yl)piperazin-1-yl]-2-{[2-(trifluoromethyl)thiazol-5-yl]methyl}-1 ,2,4-triazine-3(2H)-one

1-(5-Methylpyridin-2-yl)piperazine (78.2 mg, 0.441 mmol) was used to obtain the title compound (150 mg, yield 86%) as a yellow solid by synthesis in a similar manner to Synthesis Example 10.

### Synthesis Example 14

### 5-(7-Fluoro-3,4-dihydro-1H-pyrido[3,4-b]indol-2(9H)-yl)-2-{[2-(trifluoromethyl)thiazol-5-yl]methyl}-1,2,4-triazin-3(2H)-one

7-Fluoro-2,3,4,9-tetrahydro-1H-pyrido[3,4-b]indole (80.0 mg, 0.418 mmol) was used to obtain the title compound (130 mg, yield 69%) as a colorless solid by synthesis in a similar manner to Example 10.

### Synthesis Example 15

### (R)5-[5-Hydroxy-4-(5-methylthiophen-3-yl)-5,6-dihydropyridin-1(2H)-yl]-2-{[5-(trifluo romethyl)thiophen-2-yl)methyl}-1,2,4-triazin-3(2H)-one

(R)-4-(5-Methylthiophen-3-yl)-1,2,3,6-tetrahydropyridin-3-ol (160 mg, 818 µmol) was used to obtain the title compound (23.0 mg, yield 7%) as a yellow solid by synthesis in a similar manner to Synthesis Example 5.

### Synthesis Example 16

### 5-[6-Ethoxy-4-hydroxy-3,4-dihydroisoquinolin-2(1H)-yl]-2-{[5-trifluoromethyl)thiophe n-2-yl]methyl}-1,2,4-triazin-3(2H)-one

6-Ethoxy-1,2,3,4-tetrahydroisoquinolin-4-ol (100 mg, 435 µmol) was used to obtain the title compound (78.0 mg, yield 33%) as a white solid by synthesis in a similar manner to Synthesis Example 5.

### Synthesis Example 17

### (R)-5-[5-Hydroxy-4-(4-methylthiophen-2-yl)-5,6-dihydropyridin-1(2H)-yl]-2-{[trifluo romethyl)thiazol-5-yl]methyl}-2,4-triazin-3(2H)-one

(R)-4-(4-Methylthiophen-2-yl)-1,2,3,6-tetrahydropyridin-3-ol (64.0 mg, 328 µmol) was used to obtain the title compound (17.0 mg, yield 14%) as a white solid by synthesis in a similar manner to Synthesis Example 10.

### Synthesis Example 18

### 5-[6-Ethoxy-4-hydroxy-3,4-dihydroisoquinolin-2(1H)-yl]-2-{[2-(trifluoromethyl)thiazol-5-yl]methyl}-1,2,4-triazin-3(2H)-one

6-Ethoxy-1,2,3,4-tetrahydroisoquinolin-4-ol (100 mg, 435 µmol) was used to obtain the title compound (95.0 mg, yield 48%) as a white solid by synthesis in a similar manner to Example 10.

### Synthesis Example 19

### 5-[6-Methoxy-1-methyl-3,4-dihydroisoquinolin-2(1H)-yl]-2-{[2-trifluoromethyl)thiazol-5-yl]methyl}-1,2,4-triazin-3(2H)-one

6-Methoxy-1-methyl-1,2,3,4-tetrahydroisoquinoline (18.1 mg, 0.102 mmol) was used to obtain the title compound (26.2 mg, yield 59%) as a light brown solid by synthesis in a similar manner to Synthesis Example 10.

### Synthesis Example 20

### 5-[4-(4-Chlorophenyl)piperazin-1-yl]-2-{[2-(trifluoromethyl)thiazol-5-yl]methyl}-1,2,4-t riazin-3(2H)-one

1-(4-Chlorophenyl)piperazine (20.1 mg, 0.102 mmol) was used to obtain the title compound (8.50 mg, yield 18%) as a light yellow solid by synthesis in a similar manner to Synthesis Example 10.

### Synthesis Example 21

### 5-[4-(3-Chloro-4-fluorophenyl)piperazin-1-yl]-2-{[2-(trifluoromethyl)thiazol-5-yl]methyl }-1,2,4-triazin-3(2H)-one

1-(3-Chloro-4-fluorophenyl)piperazine (22.0 mg, 0.102 mmol) was used to obtain the title compound (16.8 mg, yield 35%) as a light yellow solid by synthesis in a similar manner to Synthesis Example 10.

### Synthesis Example 22

### 5-[5-Fluoro-4-(4-fluorophenyl)-5,6-dihydropyridin-yl]-2-{[2-(trifluoromethyl)thia zol-5-yl]methyl-1,2,4-triazin-3(2H)-one

3-Fluoro-4-(4-fluorophenyl)-1,2,3,6-tetrahydropyridine (20.0 mg, 0.102 mmol) synthesized in Reference Synthesis Example 20 was used to obtain the title compound (10.2 mg, yield 22%) as a colorless solid by synthesis in a similar manner to Synthesis Example 10.

### Synthesis Example 23

### 5-[4-(5-Methylthiophen-3-yl)piperazin-1-yl]-2-{[2-(trifluoromethyl)thiazol-5-yl]methyl}-1,2,4-triazin-3(2H)-one

1-(5-Methylthiophen-3-yl)piperazine (18.6 mg, 0.102 mmol) was used to obtain the title compound (9.40 mg, yield 21%) as a light brown solid by synthesis in a similar manner to Synthesis Example 10.

### Synthesis Example 24

### (R)-5-[5-Hydroxy-4-(1-methyl-1H-pyrazol-4-yl)-5,6-dihydropyridin-1(2H)-yl]-2-{[2-(trif luoromethyl)thiazol-5-yl]methyl}-1,2,4-triazin-3(2H)-one

(R)-4-(1-Methyl-1H-pyrazol-4-yl)-1,2,3,6-tetrahydropyridin-3-ol (18.3 mg, 0.102 mmol) synthesized in Reference Synthesis Example 28 was used to obtain the title compound (32.2 mg, yield 72%) as a light yellow solid by synthesis in a similar manner to Synthesis Example 10.

### Synthesis Example 25

### 5-[4,6-Dihydroxy-3,4-dihydroisoquinolin-2(1H)-yl]-2-{[5-(trifluoromethyl)thiophen-2-yl ]methyl}-1,2,4-triazin-3(2H)-one

To 2-{[5-(trifluoromethyl)thiophen-2-yl]methyl}-1,2,4-triazine-3,5(2H,4H)-dione (548 mg, 1.98 mmol) synthesized in Reference Synthesis Example 7, thionyl chloride (1.48 mL, 20.5 mmol) and N,N-dimethylformamide (30.4 µL) were added and the resultant reaction solution was stirred at 80°C for 2 hours. The reaction solution was concentrated and dissolved in acetonitrile. The resultant solution was added dropwise to a N,N-dimethylformamide (5.6 mL) solution of 1,2,3,4-tetrahydroisoquinoline-4,6-diol hydrochloride (400 mg, 1.98 mmol) and triethylamine (1.72 mL, 23.4 mmol) under cooling with ice. The reaction solution was stirred overnight at room temperature. Ethyl acetate and water were added to the reaction solution to extract. The organic layer was dried over anhydrous magnesium sulfate and filtered, and the filtrate was concentrated under reduced pressure. The resultant residue was subjected to silica gel column chromatography to obtain the title compound (610 mg, yield 73%) as a brown solid.

### Synthesis Example 26

### 5-[6-(Difluoromethoxy)-4-hydroxy-3,4-dihydroxyisoquinolin-2(1H)-yl]-2-{[5-(trifluoro methyl)thiophen-2-yl]methyl}-1,2,4-triazin-3(2H)-one

To a N,N-dimethylformamide solution of 5-[4,6-dihydroxy-3,4-dihydroisoquinolin-2(1H)-yl]-2-{[5-(trifluoromethyl)thiophen-2-yl] methyl}-1,2,4-triazin-3(2H)-one (30.0 mg, 0.0707 mmol) synthesized in Synthesis Example 25, sodium 1,1,1-difluorochloroacetate (21.6 mg, 0.142 mmol) and cesium carbonate (46.1 mg, 0.141 mmol) were added and the resultant reaction solution was stirred at 80°C for 2 hours. Water was added to the reaction solution, the resultant mixture was extracted with ethyl acetate, and was dried over anhydrous magnesium sulfate and filtered. The filtrate was concentrated under reduced pressure and the resultant residue was purified by silica gel (amino-based) column chromatography to obtain the title compound (5.40 mg, yield 16%) as a white solid.

### Synthesis Example 27

### (R)-5-[4-(4-Fluorophenyl)-5-hydroxy-5,6-dihydropyridin-1(2H)-yl]-2-{[5-(trifluorometh yl)furan-2-yl]methyl}-1,2,4-triazin-3(2H)-one

To a 1,4-dioxane solution (3.0 mL) of
5-mercapto-2-{[5-(trifluoromethyl)furan-2-yl]methyl}-1,2,4-triazin-3(2H)-one (100 mg, 0.360 mmol) synthesized in Reference Synthesis Example 13,
(R)-4-(4-fluorophenyl)-1,2,3,6-tetrahydropyridin-3-ol (69.7 mg, 0.360 mmol) and diisopropylethylamine (93.2 mg, 0.721 mmol) were added and the resultant reaction solution was stirred at 110°C for 16 hours. The reaction solution was concentrated under reduced pressure and the obtained residue was purified by preparative HPLC to obtain the title compound (34.0 mg, yield 22%) as a white solid.

### Synthesis Example 28

### (R)-2-{[6-(Difluoromethoxy)pyridin-3-yl]methyl}-5-[4-(4-fluorophenyl)-5-hydroxy-5,6-dihydropyridin-1(2H)-yl]-1,2,4-triazin-3(2H)one

2-{[6-(Difluoromethoxy)pyridin-3-yl]methyl}-5-mercapto-1,2,4-triazin-3(2H)-one (120 mg, 419 µmol) synthesized in Reference Synthesis Example 15 was used to obtain the title compound (55.7 mg, yield 30%) as a white solid by synthesis in a similar manner to Synthesis Example 27.

### Synthesis Example 29

### (R)-5-[5-Chloro-5'-hydroxy-6-methoxy-5',6'-dihydro-(2.4'-bipyridine)-1'(2'H)-yl]-2-{[5-(t rifluoromethyl)thiophen-2-yl]methyl}-1,2,4-triazin-3(2H)-one

To 2-{[5-(trifluoromethyl)thiophen-2-yl]methyl}-1,2,4-triazine-3,5(2H,4H)-dione (5.80 mg, 0.0209 mmol) synthesized in Reference Synthesis Example 7, thionyl chloride (15.1 µL, 0.209 mmol) and N,N-dimethylformamide (0.300 µL, 0.00468 mmol) were added and the resultant reaction solution was heated to reflux for 2 hours. The reaction solution was concentrated under reduced pressure and the resultant residue was dissolved in acetonitrile. The resultant solution was added to a N,N-dimethylformamide solution (50 µL) of (R)-5-chloro-6-methoxy-1',2',3',6'-tetrahydro-(2,4'-bipyridin)-3'-ol (5.00 mg, 0.0209 mmol) and triethylamine (11.0 µL, 0.0836 mmol) and the resultant reaction solution was stirred at room temperature for 2 hours. The reaction solution was concentrated under reduced pressure and the obtained residue was purified by silica gel column chromatography (hexane/ethyl acetate) to obtain the title compound (1.53 mg, yield 15%) as a colorless solid.

### Synthesis Example 30

### (R)-5-[4-(4-Fluoro-2-methylphenyl)-5-hydroxy-5,6-dihydropyridin-1(2H)-yl]-2-{[5-(trifl uoromethyl)thiophen-2-yl]methyl}-1,2,4-triazin-3(2H)-one

(R)-4-(4-Fluoro-2-methylphenyl)-1,2,3,6-tetrahydropyridin-3-ol (11.2 mg, 0.0540 mmol) was used to obtain the title compound (2.60 mg, yield 3%) as a light purple solid by synthesis in a similar manner to Synthesis Example 29.

### Synthesis Example 31

### (R)-5-[5'-Hydroxy-6-methyl-5',6'-dihydro-(2,4'-bipyridin)-1'(2'H)-yl]-2-{[5-(trifluoromet hyl)thiophen-2-yl]methyl}-1,2,4-triazin-3(2H)-one

(R)-6-Methyl-1',2',3',6'-tetrahydro-(2,4'-bipyridin)-3'-ol (10.3 mg, 0.0540 mmol) was used to obtain the title compound (11.7 mg, yield 12%) as a light brown solid by synthesis in a similar manner to Synthesis Example 29.

### Synthesis Example 32

### (R)-5-[5'-Hydroxy-6-methoxy-5',6'-dihydro-(2,4'-bipyridin)-1'(2'H)-yl]-2-{[5-(trifluorom ethyl)thiophen-2-yl]methyl}-1,2,4-triazin-3(2H)-one

(R)-6-Methoxy-1',2',3',6'-tetrahydro-(2,4'-bipyridin)-3'-ol (11.1 mg, 0.0540 mmol) was used to obtain the title compound (13 mg, yield 13%) as a light brown solid by synthesis in a similar manner to Synthesis Example 29.

### Synthesis Example 33

### 5-[4-(5-Fluoro-6-methylpyridin-2-yl)piperazin-1-yl]-2-{[5-(trifluoromethyl)thiophen-2-yl ]methyl}-1,2,4-triazin-3(2H)-one

1-(5-Fluoro-6-methylpyridin-2-yl)piperazine (7.04 mg, 0.0361 mmol) was used to obtain the title compound (8.30 mg, yield 8%) as a light yellow solid by synthesis in a similar manner to Synthesis Example 29.

### Synthesis Example 34

### 5-[4-(3-Chloro-4-fluorophenyl)piperazin-1-yl)-2-{[5-(trifluoromethyl)thiophen-2-yl]meth yl}-1,2,4-triazin-3(2H)-one

1-(3-Chloro-4-fluorophenyl)piperazine (27.9 mg, 0.130 mmol) was used to obtain the title compound (27.1 mg, yield 53%) as a light yellow solid by synthesis in a similar manner to Synthesis Example 29.

### Synthesis Example 35

### 5-[5-Fluoro-4-(4-fluorophenyl)-5,6-dihydropyridin-1(2H)-yl]-2-{[5-trifluoromethyl)thio phen-2-yl]methyl}-1,2,4-triazin-3(2H)-one

3-Fluoro-4-(4-fluorophenyl)-1,2,3,6-tetrahydropyridine (25.4 mg, 0.130 mmol) synthesized in Reference Synthesis Example 20 was used to obtain the title compound (19.5 mg, yield 40%) as a light yellow solid by synthesis in a similar manner to Synthesis Example 29.

### Synthesis Example 36

### 5-[5-Amino-4-(4-fluorophenyl)-5,6-dihydropyridin-1(2H)-yl]-2-{[5-(trifluoromethyl)thio phen-2-yl]methyl}-1,2,4-triazin-3(2H)-one

4-(4-Fluorophenyl)-1,2,3,6-tetrahydropyridin-3-amine (25.0 mg, 0.130 mmol) was used to obtain the title compound (5.40 mg, yield 11 %) as a light yellow solid by synthesis in a similar manner to Synthesis Example 29.

### Synthesis Example 37

### (R)-5-[5-Hydroxy-4-(5-methylfuran-2-yl)-5,6-dihydropyridin-1(2H)-yl]-2-{[5-(trifluoro methy)thiophen-2-yl]methyl}-1,2,4-triazin-3(2H)-one

(R)-4-(5-Methylfuran-2-yl)-1,2,3,6-tetrahydropyridin-3-ol (23.3 mg, 0.130 mmol) synthesized in Reference Synthesis Example 26 was used to obtain the title compound (9.70 mg, yield 21 %) as a light yellow solid by synthesis in a similar manner to Synthesis Example 29.

### Synthesis Example 38

### (R)-5-[5-Hydroxy-4-(1-methyl-1H-pyrrazol-4-yl)-5,6-dihydropyridin-1(2H)-yl]-{[5-(triflu oromethyl)thiophen-2-yl]methyl}-1,2,4-triazin-3(2H)-one

(R)-4-(1-methyl-1H-pyrazol-4-yl)-1,2,3,6-tetrahydropyridin-3-ol (23.3 mg, 0.130 mmol) synthesized in Reference Synthesis Example 28 was used to obtain the title compound (15.2 mg, yield 32%) as a light yellow solid by synthesis in a similar manner to Synthesis Example 29.

### Synthesis Example 39

### 5-[4-(1-Methyl-1H-pyrazol-3-yl)-5,6-dihydropyridin-1(2H)-yl]-2-{5-(trifluoromethyl)thi ophen-2-yl]methyl}1,2,4-triazin-3(2H)-one

4-(1-Methyl-1H-pyrazol-3-yl)-1,2,3,6-tetrahydropyridine (21.2 mg, 0.130 mmol) was used to obtain the title compound (20.9 mg, yield 46%) as a colorless solid by synthesis in a similar manner to Synthesis Example 29.

### Synthesis Example 40

### 5-[4-(5-Methylthiophen-3-yl)piperazin-1-yl]-2-{[5-(trifluoromethyl)thiohen-2-yl]methyl }-1,2,4-triazin-3(2H)-one

1-(5-Methylthiophen-3-yl)piperazine (25.4 mg, 0.130 mmol) was used to obtain the title compound (28.9 mg, yield 59%) as a light yellow solid by synthesis in a similar manner to Synthesis Example 29.

### Synthesis Example 41

### 5-[4-(4-Chlorophenyl)piperazin-1-yl]-2-{[5-(trifluoromethyl)thiophen-2-yl]methyl}1,2,4 -triazin-3(2H)-one

1-(4-Chlorophenyl)piperazine (25.5 mg, 0.130 mmol) was used to obtain the title compound (33.9 mg, yield 69%) as a light yellow solid by synthesis in a similar manner to Synthesis Example 29.

### Synthesis Example 42

### 5-[6-Methoxy-1-methyl-3,4-dihydroisoquinolin-2(1H)-yl]-2-{[5-(trifluoromethyl)thiophe n-2-yl]methyl}-1,2,4-triazin-3(2H)-one

6-Methoxy-1-methyl-1,2,3,4-tetrahydroisoquinoline (23.0 mg, 0.130 mmol) was used to obtain the title compound (13.8 mg, yield 29%) as a colorless solid by synthesis in a similar manner to Synthesis Example 29.

### Synthesis Example 43

### (R)-5-[4-(3-Chloro-4-fluorophenyl)-5-hydroxy-5,6-dihydropyridin-1(2H)-yl]-2-{[2-triflu oromethyl)thiazol-5-yl]methyl}-1,2,4-triazin-3(2H)-one

To an acetonitrile solution (60 µL) of 1,2,4-triazine-3,5-dione (20.0 mg, 0.177 mmol), (R)-4-(3-chloro-4-fluorophenyl)-1,2,3,6-tetrahydropyridin-3-ol (48.3 mg, 0.212 mmol), N,O-bis(trimethylsilyl)acetamide (262 µL, 1.06 mmol), and lithium iodide (35.3 mg, 0.265 mmol) were added and the resultant reaction solution was stirred at 60°C for 12 hours. To the reaction solution, 2-trifluoromethyl-5-chloromethylthiazole (35.7 mg, 0.177 mmol) was added and the resultant reaction solution was stirred for 15 hours. The reaction solution was concentrated under reduced pressure and the obtained residue was purified by silica gel column chromatography (hexane/ethyl acetate) to obtain the title compound (22.1 mg, yield 26%) as a colorless solid.

### Synthesis Example 44

### 5-[6-(trifluoromethyl)-3,4-dihydroisoquinolin-2-(1H)-yl]-2-{[2-(trifluoromethyl)thiazol-5 -yl]methyl}-1,2,4-triazin-3(2H)one

6-(Trifluoromethyl)-1,2,3,4-tetrahydroisoquinoline (53.4 mg, 0.265 mmol) was used to obtain the title compound (6.40 mg, yield 5%) as a light brown solid by synthesis in a similar manner to Synthesis Example 43.

### Synthesis Example 45

### (R)-5-[4-(3-Fluoro-4-methylphenyl)-5-hydroxy-5,6-dihydropyridin-1(2H)-yl]-2-{[2-trifl uoromethyl)thiazol-5-yl]methyl}-1,2,4-triazin-3(2H)-one

(R)-4-(3-Fluoro-4-methylphenyl)-1,2,3,6-tetrahydropyridin-3-ol (55.0 mg, 0.265 mmol) was used to obtain the title compound (11.2 mg, yield 9%) as a light yellow solid by synthesis in a similar manner to Synthesis Example 43.

### Synthesis Example 46

### (R)-5-[4-(4-Fluorophenyl)-5-hydroxy-5,6-dihydropyridin-1(2H)-yl]-2-{[3-(trifluorometh yl)-1H-pyrazol-1-yl]methyl}-1,2,4-triazin-3(2H)-one

To an acetonitrile solution (60 µL) of 1,2,4-triazine-3,5-dione (20.0 mg, 0.177 mmol), (R)-4-(4-fluorophenyl)-1,2,3,6-tetrahydropyridin-3-ol (34.1 mg, 0.177 mmol), N,O-bis(trimethylsilyl)acetamide (700 µL, 2.82 mmol), and lithium iodide (35.5 mg, 0.265 mmol) were added and the resultant reaction solution was stirred at 60°C for 12 hours. To the reaction solution, 1-(chloromethyl)-3-(trifluoromethyl)-1H-pyrazole (32.6 mg, 0.177 mmol) was added and the resultant reaction solution was stirred for 15 hours. The reaction solution was concentrated under reduced pressure and the obtained residue was purified by silica gel column chromatography (hexane/ethyl acetate) to obtain the title compound (33.1 mg, yield 43%) as a colorless solid.

### Synthesis Example 47

### (R)-2-[(4-Chloro-1H-pyrazol-1-yl)methyl]-5-[4-(4-fluorophenyl)-5-hydroxy-5,6-dihydro pyridin-1(2H)-yl]-1,2,4-triazin-3(2H)-one

4-Chloro-1-(chloromethyl)-1H-pyrazole (44.1 mg, 0.292 mmol) was used to obtain the title compound (42.8 mg, yield 40%) as a light yellow solid by synthesis in a similar manner to Synthesis Example 46.

### Synthesis Example 48

### (R)-2-{[5-(Tert-butyl)thiophen-2-yl]methyl}-5-[4-(4-fluorophenyl)-5-hydroxy-5,6-dihydr opyridin-1(2H)yl]-1,2,4-triazin-3(2H)-one

2-(Tert-butyl)-5-(chloromethyl)thiophene (55.1 mg, 0.292 mmol) was used to obtain the title compound (37.7 mg, yield 32%) as a colorless solid by synthesis in a similar manner to Synthesis Example 46.

### Synthesis Example 49

### (R)-5-[4-(4-Fluorophenyl)-5-hydroxy-5,6-dihydropyridin-1(2H)-yl]-2-{[3-(perfluoroethyl )-1H-pyrazol-1-yl]methyl}-1,2,4-triazin-3(2H)-one

1-(chloromethyl)-3-(perfluoroethyl)-1H-pyrazole (68.5 mg, 0.292 mmol) was used to obtain the title compound (42.7 mg, yield 33%) as a colorless solid by synthesis in a similar manner to Synthesis Example 46.

### Synthesis Example 50

### (R)-2-{[2-(Tert-butyl)oxazol-5-yl]methyl}-5-[4-(4-fluorophenyl)-5-hydroxy-5,6-dihydro pyridin-1(2H)-yl]-1,2,4-triazin-3(2H)-one

2-(Tert-butyl)-5-(chloromethyl)oxazole (55.2 mg, 0.318 mmol) synthesized in Reference Synthesis Example 17 was used to obtain the title compound (8.30 mg, yield 7%) as a colorless solid by synthesis in a similar manner to Synthesis Example 46.

### Synthesis Example 51

### (R)-5-({5-[4-(4-Fluorophenyl)-5-hydroxy-5,6-dihydropyridin-1(2H)-yl]-3-oxo-1,2,4-triaz in-2(3H)-yl}methyl)pyridin-2-yl trifluoromethanesulfonate

5-(Chloromethyl)pyridin-2-yl trifluoromethanesulfonate (80.4 mg, 0.292 mmol) was used to obtain the title compound (26.7 mg, yield 19%) as a light yellow solid by synthesis in a similar manner to Synthesis Example 46.

### Synthesis Example 52

### (R)-5-[4-(3-Fluoro-4-methylphenyl)-5-hydroxy-5,6-dihydropyridin-1(2H)-yl]-2-{[5-(trifl uoromethyl)thiophen-2-yl]methyl}-1,2,4-triazin-3(2H)-one

To an acetonitrile solution (90 µL) of 1,2,4-triazine-3,5-dione (30.0 mg, 0.265 mmol), (R)-4-(3-fluoro-4-methylphenyl)-1,2,3,6-tetrahydropyridin-3-ol (55.0 mg, 0.263 mmol), N,O-bis(trimethylsilyl)acetamide (263 µL, 1.06 mmol), and lithium iodide (53.3 mg, 0.398 mmol) were added and the resultant reaction solution was stirred at 60°C for 12 hours. To the reaction solution, 2-trifluoromethyl-5-bromomethylthiophene (39.6 µL, 0.279 mmol) was added and the resultant reaction solution was stirred for 15 hours. The reaction solution was concentrated under reduced pressure and the obtained residue was purified by silica gel column chromatography (hexane/ethyl acetate) to obtain the title compound (25.8 mg, yield 21%) as a brown solid.

### Synthesis Example 53

### 5-[6-(trifluoromethyl)-3,4-dihydroisoquinolin-2(1H)-yl]-2-{[5-(trifluoromethyl)thiophen-2-yl]methyl}-1,2,4-triazin-3(2H)-one

6-(Trifluoromethyl)-1,2,3,4-tetrahydroisoquinoline (53.4 mg, 0.265 mmol) was used to obtain the title compound (21.8 mg, yield 18%) as a colorless solid by synthesis in a similar manner to Synthesis Example 52.

### Synthesis Example 54

### (R)-5-[4-(3-Chloro-4-fluorophenyl)-5-hydroxy-5,6-dihydropyridin-1(2H)-yl]-2-{[5-(triflu oromethyl)thiophen-2-yl]methyl}-1,2,4-triazin-3(2H)-one

(R)-4-(3-Chloro-4-fluorophenyl)-1,2,3,6-tetrahydropyridin-3-ol (60.4 mg, 0.265 mmol) was used to obtain the title compound (12.8 mg, yield 10%) as a light brown solid by synthesis in a similar manner to Synthesis Example 52.

### Synthesis Example 55

### 5-[6-(Difluoromethoxy)-4-hydroxy-3,4-dihydroxyisoquinolin-2(1H)-yl]-2-{[2-(trifluoro methyl)thiazol-5-yl]methyl)-1,2,4-triazin-3(2H)-one

5-[4,6-Dihydroxy-3,4-dihydroisoquinolin-2(1H)-yl]-2-{[2-(trifluoromethyl)thiazol-5 -yl]methyl}-1,2,4-triazin-3(2H)-one (95.0 mg, 0.223 mmol) synthesized in Synthesis Example 29 was used to obtain the title compound (18.0 mg, yield 17%) as a light yellow solid by synthesis in a similar manner to Synthesis Example 26.

The chemical structural formulae of the compounds synthesized in Reference Synthesis Examples and Synthesis Examples will be shown hereinafter. Tables 3 and 4 show the data of physical properties of the compounds synthesized in Reference Synthesis Examples, and Tables 5 to 7 show the data of physical properties of the compounds synthesized in Synthesis Examples. As mentioned earlier, Rf described below means Reference Synthesis Example, and Ex described below means Synthesis Example.

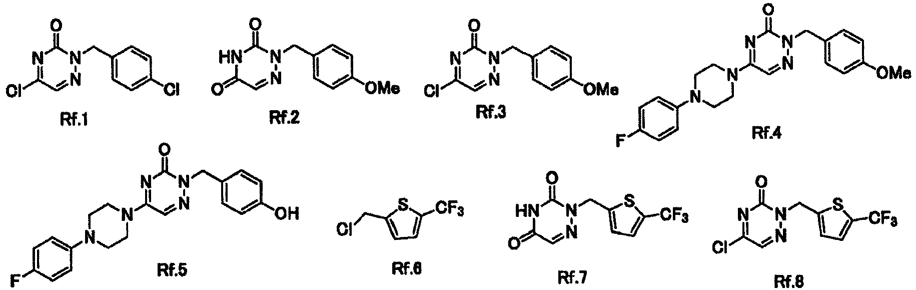

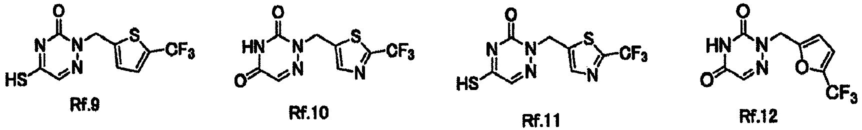

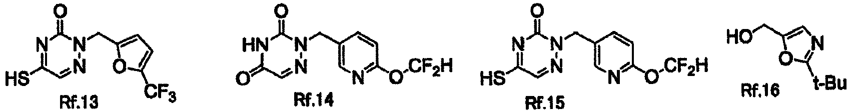

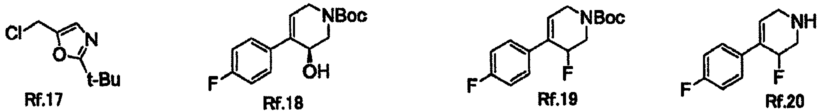

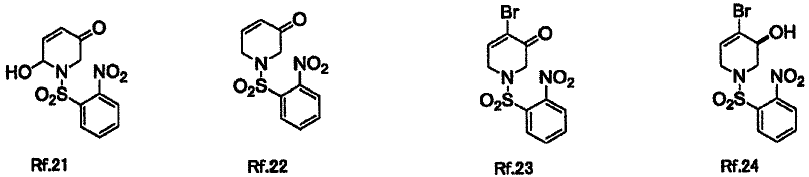

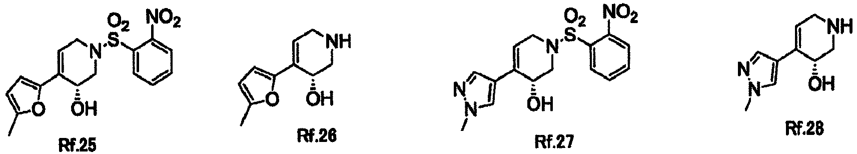

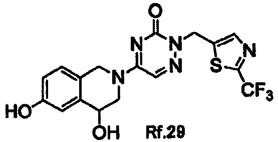

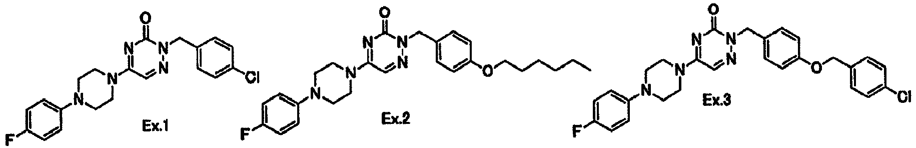

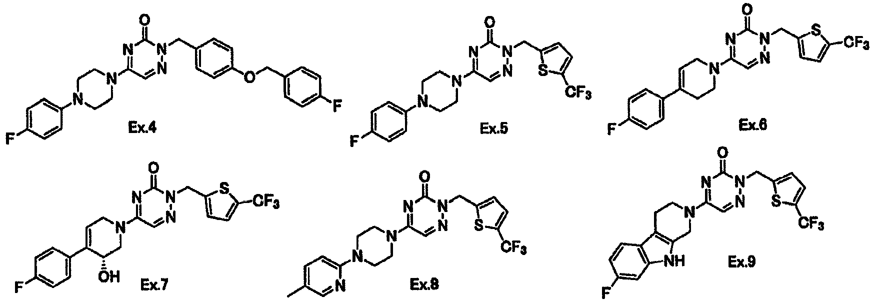

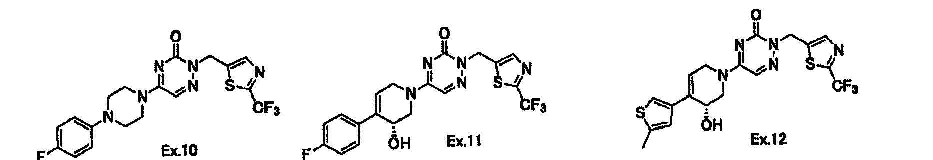

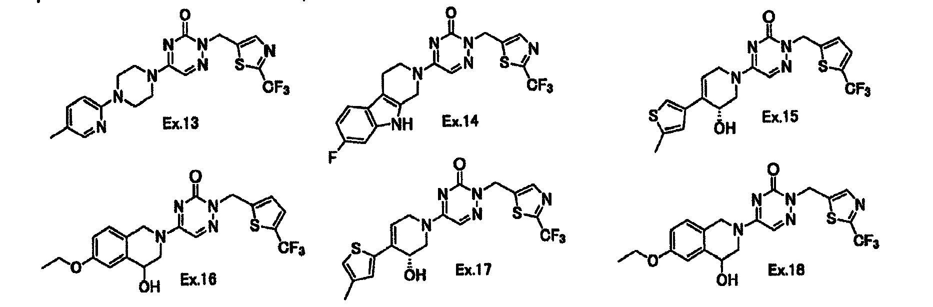

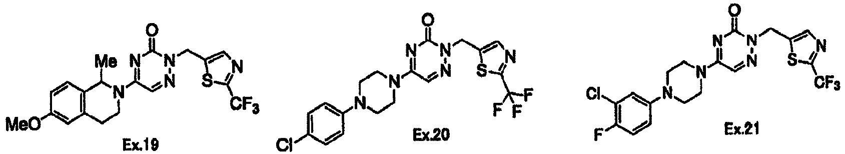

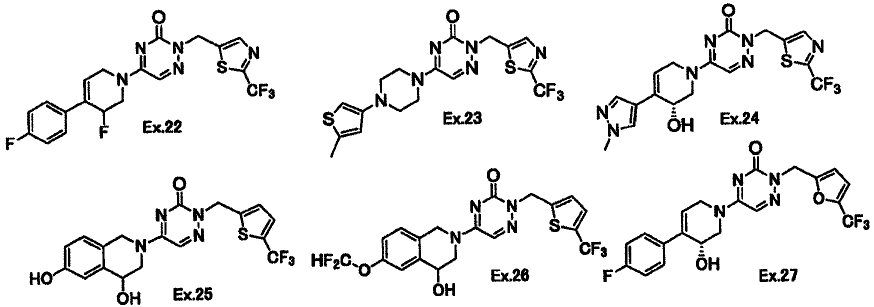

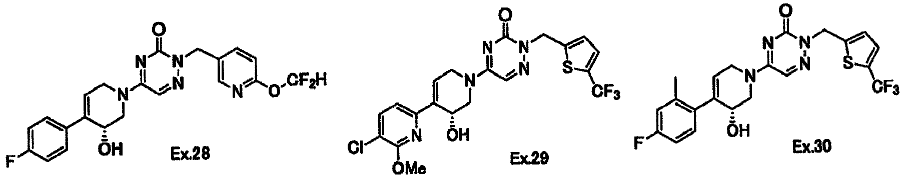

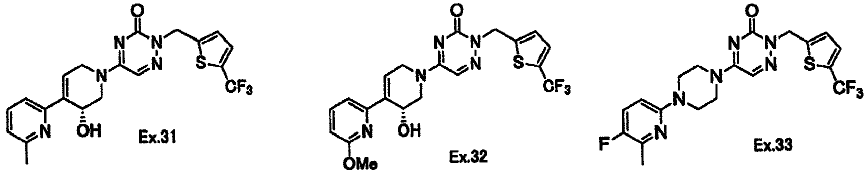

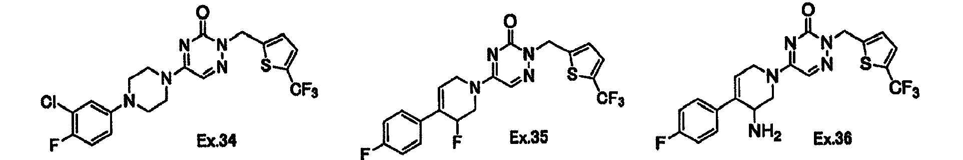

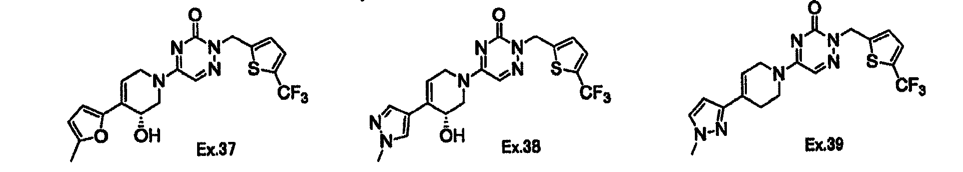

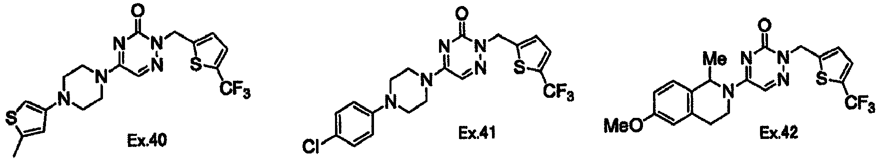

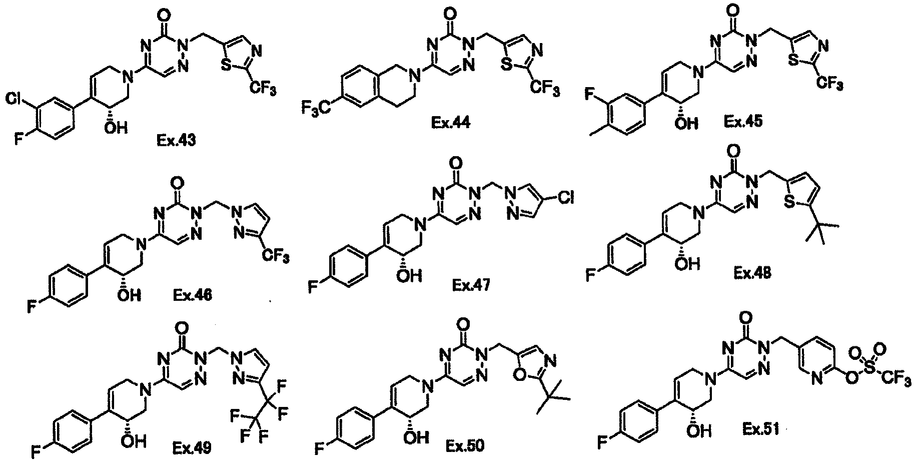

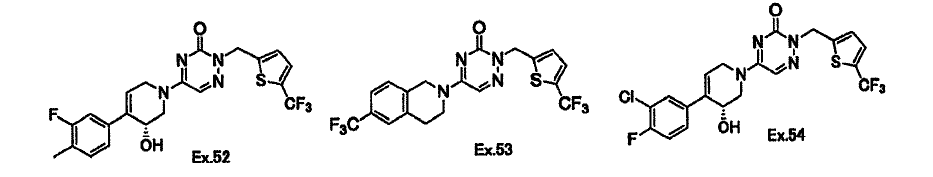

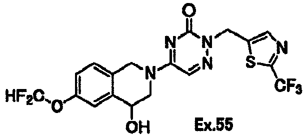

**Table 4**

| Rf | Data |
|---|---|
| 2 | ¹H-NMR (CDCl₃) *δ* : 3.80(s, 3H), 5.04(s, 2H), 6.87(d, J = 9.0 Hz, 2H), 7.34(d, J = 9.0 Hz, 2H), 7.38(s, 1H), 9.21(brs, 1H) |
| | LC/MS; cond. 1 RT 3.12 min |
| | LC/MS (ESI') m/z, 234 [M+H]⁺ |
| 4 | LC/MS: cond. 1 / RT 4.05 min |
| | LC/MS (ESI⁺) m/z; 396 [M+H]⁺ |
| 5 | ¹H-NMR (DMSO-d₆) *δ* **:** 3.16(m, 4H), 3. 84 (m, 4H), 4. 94 (s, 2H), 6.70(d. J = 8.4 Hz, 2H), 6.99-7.14(m, 6H), 8.09(s, 1H), 9.39(s, 1H) |
| | LC/MS: cond. 1 RT 3.70 min |
| | LC/MS (ESI⁺) m/z; 382 [M+H]⁺ LC/MS (ESI⁻) m/z : 380 [M-H]⁻ |
| 8 | LC/MS: cond. 1 RT 4.99 min |
| | LC/MS (ESI⁺) m/z; 325 [M+H]⁺ |
| 9 | LC/MS: cond. 2 RT 2.35 min |
| | LC/MS (ESI⁺) m/z; 294 [M+H]⁺ LC/MS (ESI⁻) m/z; 292 [M-H]⁻ |
| 10 | ¹H-NMR (DMSO-d₆) *δ* : 5.36(s, 2H), 7.52(s, 1H), 8. 16(s, 1H), 12.28(brs, 1H) |
| | LC/MS; cond. 2 RT 1.71 min |
| | LC/MS(ESI⁺) m/z, 279 [M+H]⁺ LC/MS (ESI⁻) m/z; 277 [M-H]⁻ |
| 11 | ¹H-NMR (DMSO-d₆) *δ* : 5.36(s, 2H), 7.74(s, 1H), 8.18(s, 1H), 13.74(brs, 1H) |
| | LC/MS; cond. 2 RT 2.09 min |
| | LC/MS(ESI⁺) m/z, 295 [M+H]⁺ LC/MS (ESI⁻) m/z; 293 [M-H]⁻ |
| 12 | ¹H-NMR(CDCl₃) *δ* : 5.15(s, 2H), 6.50(d, J = 3.2 Hz, 1H), 6.77 (d, = 2.4 Hz, 1H), 7.43(d, J = 2.0 Hz, 1H), 8.84(brs, 1H) |
| 13 | ¹H-NMR(CDCl₃) *δ* : 5.12(s, 2H), 6. 52 (d, J = 3.2 Hz, 1H), 6. 78 (d, J = 2.4 Hz, 1H), 7.63(d, J = 1.6 Hz, 1H), 9.85(brs, 1H) |
| 14 | ¹H-NMR(CDCl₃) *δ* : 5.08(s, 2H), 6.91(d, J = 8.4 Hz, 1H), 7.41(d, J = 2.4 Hz, 1H), 7.65(t, J = 72.8 Hz, 1H), 7.82(dd, J = 8.8 Hz, 2.4 Hz, 1H), 8.27(d, J = 2.4 Hz, 1H), 8.74 (brs, 1H) |
| 15 | ¹H-NMR(CDCl₃) *δ* : 5.05(s, 2H), 6. 92(d, J = 8.4 Hz, 1H), 7.47(t, J = 73.2 Hz, 1H), 7.61 (s, 1H), 7. 84 (dd, J = 8.8 Hz, 2.0 Hz, 1H), 8.29(d, J = 2.0 Hz, 1H), 9.81(brs, 1H) |
| 16 | LC/MS: cond.2 / RT 1.28 min |
| | LC/MS (ESI⁺) m/z; 156 [M+H]⁺ |
| 17 | LC/MS: cond.2 / RT 2.09 min |
| | LC/MS (ESI⁺) m/z; 174 [M+H]⁺ |
| 18 | LC/MS; cond. 2 RT 2.28 min |
| | LC/MS(ESI⁺) m/z, 293 [M+H]⁺ |
| 20 | LC/MS; cond. 2 RT 1.08 min |
| | LC/MS(ESI⁺) m/z, 196 [M+H]⁺ |
| 24 | ¹H-NMR(CDCl₃) *δ* : 2.43(d, J = 7.5 Hz, 1H), 3.55(dd, J = 14.4 Hz, 3.6 Hz, 1H), 3.68(dd, J = 13.2 Hz, 4.2 Hz, 1H), 3.73-3.85(m, 1H), 4.06(dd, J = 17.7Hz, 3.9Hz, 1H), 4.21-4.24(m, 1H), 6.23(dd, J = 4.2Hz, 3.0Hz, 1H), 7.64-7.77(m, 3H), 8.07(m, 1H) |
| 26 | LC/MS; cond. 2 RT 0.63 min |
| | LC/MS(ESI⁺) m/z, 180 [M+H]⁺ |
| 27 | LC/MS; cond. 2 RT 1.60 min |
| | LC/MS(ESI⁺) m/z, 365 [M+H]⁺ LC/MS (ESI⁻) m/z; 409 [M+HCO2]⁻ |
| 28 | LC/MS; cond. 1 RT 0.26 min |
| | LC/MS(ESI⁺) m/z, 180 [M+H]⁺ |
| 29 | LC/MS; cond. 2 RT 1.72 min |
| | LC/MS(ESI⁺) m/z, 426 [M+H]⁺ LC/MS (ESI⁻) m/z; 470 [M+HCO₂]⁻ |

**Table 5**

| Ex | Data |
|---|---|
| 1 | ¹H-NMR (CDCl₃) *δ* : 3. 10-3.20(m, 4H), 3. 67-4. 14(m, 4H), 5.16(s, 2H), 6. 84-6. 99 (m, 2H), 6.94-7.03(m, 2H), 7.26-7.30(m, 2H), 7. 34-7. 39 (m, 2H), 7.61(s, 1H) |
| | LC/MS: cond. 1 RT 4.28 min |
| | LC/MS (ESI⁺) m/z; 400 [M+H]⁺ |
| 2 | ¹H-NMR (CDCl₃) *δ* : 0.89(t, J = 6.9 Hz, 3H), 1.28-1.37(m, 4H), 1. 37-1. 52 (m, 2H), 1. 69-1. 82 (m, 2H), 3. 10-3. 19 (m, 4H), 3.65-4.14(m, 4H), 3.92(t, J = 6.6 Hz, 2H), 5.14(s, 2H), 6.80-7.02(m, 6H), 7.37(d, J = 8.7 Hz, 2H), 7.60(s, 1H). |
| | LC/MS: cond. 1 RT 4.92 min |
| | LC/MS (ESI⁺) m/z; 466 [M+H]⁺ |
| 3 | ¹H-NMR (CDCl₃) *δ* : 3. 12-3. 18 (m, 4H), 5.01(s, 2H), 5. 14 (s, 2H), 6. 84-7. 03 (m, 6H), 7.32-7.42(m, 6H), 7.61(s, 1H) |
| | LC/MS: cond. 1 RT 4.70 min |
| | LC/MS (ESI⁺) m/z; 506 [M+H]⁺ |
| 4 | ¹H-NMR (CDCl₃) *δ* : 3.12-3. 19(m, 4H), 3. 64-4. 19 (m, 4H), 5.00(s, 2H), 5.14(s, 2H), 6. 85-7.09(m, 8H), 7. 35-7.41(m, 4H), 7.60(s, 1H) |
| | LC/MS: cond. 1 RT 4.57 min |
| | LC/MS (ESI⁺) m/z; 490 [M+H]⁺ LC/MS (ESI⁻) m/z; 534 [M+HCO₂]⁻ |
| 5 | ¹H-NMR (CDCl₃) *δ* : 3.08-3.24 (m, 4H), 3. 68-4. 16 (m, 4H), 5.33( s, 2H), 6.80-7.04(m, 4H), 7.08-7. 13 (m, 1H), 7. 24-7. 30 (m, 1H), 7.65(s, 1H) |
| | LC/MS: cond. 2 RT 2.50 min |
| | LC/MS (ESI⁺) m/z; 440 [M+H]⁺ |
| 6 | ¹H-NMR (CDCl₃) *δ* : 2.53-2.67 (m, 2H), 3.92(d, J = 5.7 Hz, 1H), 3.94(d, J = 5.7 Hz, 1H), 4.26-4.40 (m, 2H), 5. 29 (s, 2H), 6. 15-6. 22 (m, 1H), 7. 13-7. 24 (m, 3H), 7. 46-7. 54 (m, 2H), 7. 57 (dd, J = 1.2 Hz, 3.9 Hz, 1H), 8.02-8.23 (m, 1H) |
| | LC/MS: cond. 1 RT 4.39 min |
| | LC/MS (ESI⁺) m/z; 437 [M+H]⁺ LC/MS (ESI⁻) m/z; 481 [M-H]⁻ |
| 7 | LC/MS: cond. 2 RT 2.30 min LC/MS (ESI⁺) m/z; 453 [M+H]⁺ LC/MS (ESI⁻) m/z; 497 [M+HCO₂]⁻ |
| 8 | ¹H-NMR (CDCl₃) *δ* : 2.22(s, 3H), 3.46-4.10(m, 8H), 5.34(s, 2H), 6.60(d, J = 8.7 Hz, 1H), 7.12 (d J = 3.3 Hz, 1H), 7.29(d, J = 2.7 Hz, 1H), 7.36(dd, J = 8.4 Hz, 2.4 Hz, 1H), 7.64(s, 1H), 8.03(d, J = 2.7 Hz, 1H) |
| | LC/MS: cond. 2 RT 1.82 min |
| | LC/MS (ESI⁻) m/z; 437 [M+H]⁺ |
| 9 | ¹H-NMR (DMSO-d₆) *δ* : 2. 70-2.88(m, 2H), 3.99-4. 12(m, 2H), 4.90(s, 2H), 5. 29(s, 2H), 6. 78-6. 89 (m, 1H), 7. 13(d, J = 10.5 Hz, 1H), 7.21(d, J = 3Hz, 1H), 7.32-7.46 (m, 1H), 7. 56 (d, J = 3 Hz, 1H), 8.30(s, 1H), 11.04(s, 1H) |
| | LC/MS: cond. 2 RT 2.57 min |
| | LC/MS (ESI⁻) m/z; 450 [M+H]⁺ LC/MS (ESI⁻) m/z; 494 [M+HCO₂]⁻ |
| 10 | ¹H-NMR (CDCl₃) *δ* : 3.08-3.28(m, 4H), 3.68-4.16(m, 4H), 5.39(s, 2H), 6.83-7.04(m, 4H), 7.64(s, H), 7.96(s, 1H) |
| | LC/MS: cond. 2 RT 2.31 min |
| | LC/MS (ESI⁺) m/z; 441 [M+H]⁺ |

**Table 6**

| Ex | Data |
|---|---|
| 11 | LC/MS: cond. 2 RT 2.14 min |
| | LC/MS (ESI⁺) m/z; 454 [M+H]⁺ LC/MS (ESI⁻) m/z; 498 [M+HCO₂]⁻ |
| 12 | LC/MS: cond. 2 RT 2.19 min |
| | LC/MS (ESI⁺) m/z; 456 [M+H]⁺ LC/MS (ESI⁻) m/z; 500 [M+HCO₂]⁻ |
| 13 | ¹H-NMR (CDCl₃) *δ* : 2.21(s, 3H), 3.44-4.10(m, 8H), 5.39(s, 2H), 6.60(d J = 8.2 Hz, 1H), 7.36(dd, J = 8.4 Hz, J = 1.8 Hz, 1H), 7.64(s, 1H), 7. 96 (s, 1H), 8.02(s, 1H) |
| | LC/MS: cond. 2 RT 1.82 min |
| | LC/MS (ESI⁺) m/z; 437 [M+H]⁺ |
| 14 | ¹H-NMR (DMSO-d₆) *δ* : 2. 69-2.88 (m, 2H), 3.99-4.16(m, 2H), 4.90(s, 2H), 5.39(s, 2H), 6.75-6.88 (m, 1H), 7.13(d J = 10.2 Hz, 1H), 7. 37-7. 43 (m, 1H), 8.16(s, 1H), 8.31 (s, 1H), 11.04(s, 1H) |
| | LC/MS: cond. 2 RT 2.41 min |
| | LC/MS (ESI⁺) m/z; 451 [M+H]⁺ LC/MS (ESI⁻) m/z; 495 [M+HCO₂]⁻ |
| 15 | LC/MS: cond. 2 RT 2.30 min |
| | LC/MS (ESI⁻) m/z; 455 [M+H]⁺ LC/MS (ESI⁻) m/z; 499 [M+HCO₂]⁻ |
| 16 | LC/MS: cond. 2 RT 2.25 min |
| | LC/MS (ESI⁻) m/z; 453 [M+H]⁺ LC/MS (ESI⁻) m/z; 497 [M+HCO₂]⁻ |
| 17 | LC/MS: cond. 2 RT 2.14 min |
| | LC/MS (ESI⁺) m/z; 456 [M+H]⁺ LC/MS (ESI⁻) m/z; 500 [M+HCO₂]⁻ |
| 18 | LC/MS: cond. 2 RT 2.07 min |
| | LC/MS (ESI⁺) m/z; 454 [M+H]⁺ LC/MS (ESI⁻) m/z; 498 [M+HCO₂]⁻ |
| 19 | LC/MS: cond. 2 RT 2.33 min |
| | LC/MS (ESI⁺) m/z; 438 [M+H]⁺ |
| 20 | LC/MS: cond. 2 RT 2.44 min |
| | LC/MS (ESI⁺) m/z; 457 [M+H]⁺ |
| 21 | LC/MS: cond. 2 RT 2.46 min |
| | LC/MS (ESI⁺) m/z; 475 [M+H]⁺ |
| 22 | LC/MS: cond. 2 RT 2.33 min |
| | LC/MS (ESI⁺) m/z; 456 [M+H]⁺ |
| 23 | LC/MS: cond. 2 RT 2.26 min |
| | LC/MS (ESI⁺) m/z; 443 [M+H]⁺ |
| 24 | LC/MS: cond. 2 RT 1.65 min |
| | LC/MS (ESI⁺) m/z; 440 [M+H]⁺ LC/MS (ESI⁻) m/z; 484 [M+HCO₂]⁻ |
| 25 | LC/MS; cond. 2 RT 1.93 min |
| | LC/MS (ESI⁺) m/z, 425 [M+H]⁺ LC/MS (ESI⁻) m/z; 469 [M+HCO₂]⁻ |
| 26 | LC/MS; cond. 2 RT 2.25 min |
| | LC/MS (ESI⁺) m/z, 475 [M+H]⁺ LC/MS (ESI⁻) m/z; 519 [M+HCO₂]⁻ |
| 27 | LC/MS: cond. 2 RT 2.15 min |
| | LC/MS (ESI⁺) m/z; 437 [M+H]⁺ LC/MS (ESI⁻) m/z; 481 [M+HCO₂]⁻ |
| 28 | LC/MS: cond. 2 RT 2.06 min |
| | LC/MS (ESI⁺) m/z; 446 [M+H]⁺ LC/MS (ESI⁻) m/z; 490 [M+HCO₂]⁻ |
| 29 | LC/MS: cond. 2 RT 2.38 min |
| | LC/MS (ESI⁺) m/z; 500 [M+H]⁺ LC/MS (ESI⁻) m/z; 544 [M+HCO₂]⁻ |
| 30 | LC/MS: cond. 2 RT 2.35 min |
| | LC/MS (ESI⁺) m/z; 467 [M+H]⁺ LC/MS (ESI⁻) m/z; 511 [M+HCO₂]⁻ |

**Table 7**

| Ex | Data |
|---|---|
| 31 | LC/MS: cond. 2 RT 1.71 min |
| | LC/MS (ESI⁺) m/z; 450 [M+H]⁺ LC/MS (ESI⁻) m/z; 494 [M+HCO₂]⁻ |
| 32 | LC/MS: cond. 2 RT 2.22 min |
| | LC/MS (ESI⁺) m/z; 466 [M+H]⁺ LC/MS (ESI⁻) m/z; 510 [M+HCO₂]⁻ |
| 33 | LC/MS: cond. 2 RT 2.45 min |
| | LC/MS (ESI⁺) m/z; 455 [M+H]⁺ |
| 34 | LC/MS: cond. 2 RT 2.61 min |
| | LC/MS (ESI⁺) m/z; 474 [M+H]⁺ |
| 35 | LC/MS: cond. 2 RT 2.49 min |
| | LC/MS (ESI⁺) m/z; 455 [M+H]⁺ |
| 36 | LC/MS: cond. 2 RT 1.87 min |
| | LC/MS (ESI⁺) m/z; 452 [M+H]⁺ |
| 37 | LC/MS: cond. 2 RT 2.23 min |
| | LC/MS (ESI⁺) m/z; 439 [M+H]⁺ LC/MS (ESI⁻) m/z; 483 [M+HCO₂]⁻ |
| 38 | LC/MS: cond. 2 RT 1.87 min |
| | LC/MS (ESI⁺) m/z; 439 [M+H]⁺ |
| 39 | LC/MS: cond. 2 RT 2.12 min |
| | LC/MS (ESI⁺) m/z; 423 [M+H]⁺ |
| 40 | LC/MS: cond. 2 RT 2.44 min |
| | LC/MS (ESI⁺) m/z; 442 [M+H]⁺ |
| 41 | LC/MS: cond. 2 RT 2.59 min |
| | LC/MS (ESI⁺) m/z; 456 [M+H]⁺ |
| 42 | LC/MS: cond. 2 RT 2.50 min |
| | LC/MS (ESI⁺) m/z; 437 [M+H]⁺ |
| 43 | LC/MS: cond. 2 RT 2.24 min |
| | LC/MS (ESI⁺) m/z; 488 [M+H]⁺ |
| 44 | LC/MS: cond. 2 RT 2.47 min |
| | LC/MS (ESI⁺) m/z; 462 [M+H]⁺ LC/MS (ESI⁻) m/z; 506 [M+HCO₂]⁻ |
| 45 | LC/MS: cond. 2 RT 2.22 min |
| | LC/MS (ESI⁺) m/z; 468 [M+H]⁺ LC/MS (ESI⁻) m/z; 512 [M+HCO₂]⁻ |
| 46 | LC/MS: cond. 2 RT 2.05 min |
| | LC/MS (ESI⁺) m/z; 437 [M+H]⁺ LC/MS (ESI⁻) m/z; 481 [M+HCO₂]⁻ |
| 47 | LC/MS: cond. 2 RT 1.86 min |
| | LC/MS (ESI⁺) m/z; 403 [M+H]⁺ LC/MS (ESI⁻) m/z; 447 [M+HCO₂]⁻ |
| 48 | LC/MS: cond. 2 RT 2.41 min |
| | LC/MS (ESI⁺) m/z; 441 [M+H]⁺ LC/MS (ESI⁻) m/z; 485 [M+HCO₂]⁻ |
| 49 | LC/MS: cond. 2 RT 2.23 min |
| | LC/MS (ESI⁺) m/z; 487 [M+H]⁺ LC/MS (ESI⁻) m/z; 531 [M+HCO₂]⁻ |
| 50 | LC/MS: cond. 2 RT 1.95 min |
| | LC/MS (ESI⁺) m/z; 426 [M+H]⁺ LC/MS (ESI⁻) m/z; 470 [M+HCO₂]⁻ |
| 51 | LC/MS: cond. 2 RT 2.26 min |
| | LC/MS (ESI⁺) m/z; 528 [M+H]⁺ LC/MS (ESI⁻) m/z; 572 [M+HCO₂]⁻ |
| 52 | LC/MS: cond. 2 RT 2.37 min |
| | LC/MS (ESI⁺) m/z; 467 [M+H]⁺ LC/MS (ESI⁻) m/z; 511 [M+HCO₂]⁻ |
| 53 | LC/MS: cond. 2 RT 2.62 min |
| | LC/MS (ESI⁺) m/z; 461 [M+H]⁺ LC/MS (ESI⁻) m/z; 505 [M+HCO₂]⁻ |
| 54 | LC/MS: cond. 2 RT 2.39 min |
| | LC/MS (ESI⁺) m/z; 487 [M+H]⁺ |
| 55 | LC/MS; cond. 2 RT 2.08 min |
| | LC/MS (ESI⁺) m/z, 476 [M+H]⁺ LC/MS (ESI⁻) m/z; 520 [M+HCO₂]⁻ |

### Pharmacological analysis

The pharmacological analysis on the compound of the present invention will be described below.

### 1. Calcium influx inhibition assay

Human T-type calcium channel (Cav 3.2) expressing HEK293 cells were introduced from Prof. Edward Perez-Reyes, University of Virginia, USA. The calcium-sensitive fluorescent dye used was FLIPR Calcium 4 Assay Kit (Molecular Devices). To a black 96-well plate with a clear bottom coated with type I collagen, the human T-type calcium channel (Cav 3.2) expressing HEK293 cells were seeded and cultured overnight, and the culture medium was removed. A solution of the calcium-sensitive fluorescent dye was added, and the resultant plate was incubated at 37°C in an atmosphere of 5% carbon dioxide for 30 minutes. To the plate, a diluted solution of a compound was added, and subjected to further incubation at 37°C in an atmosphere of 5% carbon dioxide for 30 minutes. While fluorescence was continuously analyzed from the bottom with a FlexStation 3 (Molecular Devices), a calcium solution was added. The increase in the fluorescence due to calcium influx caused by the stimulus was observed for 70 seconds. Based on the rise value in the fluorescence from the base line, the inhibition percentage was calculated. The logarithms of compound concentrations were plotted with respect to the inhibition activities to obtain an IC₅₀ value.

The IC₅₀ values of all the compounds of Synthesis Examples showed 10 µM or less.

Table 8 shows the resulting T-type calcium channel inhibition concentrations of the compounds of Synthesis Examples.

**Table 8**

| Synthesis Example Number | IC₅₀ (*µ*M) |
|---|---|
| 1 | 0.22 |
| 2 | 0.038 |
| 3 | 0.20 |
| 4 | 0.035 |
| 5 | 0.027 |
| 6 | 0.024 |
| 7 | 0.0060 |
| 8 | 0.21 |
| 9 | 0.017 |
| 10 | 0.49 |
| 11 | 0.030 |
| 12 | 0.026 |
| 13 | 6.1 |
| 14 | 0.039 |
| 15 | 0.0010 |
| 16 | 0.18 |
| 17 | 0.20 |
| 18 | 2.9 |
| 19 | 2.2 |
| 20 | 0.19 |
| 21 | 0.23 |
| 22 | 0.12 |
| 23 | 0.67 |
| 24 | 4.9 |
| 25 | 1.8 |
| 26 | 0.064 |
| 27 | 0.13 |
| 28 | 0.094 |
| 29 | 0.012 |
| 30 | 0.072 |
| 31 | 0.12 |
| 32 | 0.048 |
| 33 | 0.032 |
| 34 | 0.061 |
| 35 | 0.031 |
| 36 | 0.0034 |
| 37 | 0.0048 |
| 38 | 0.18 |
| 39 | 1.3 |
| 40 | 0.069 |
| 41 | 0.029 |
| 42 | 0.60 |
| 43 | 0.054 |
| 44 | 0.57 |
| 45 | 0.13 |
| 46 | 0.11 |
| 47 | 2.6 |
| 48 | 0.0064 |
| 49 | 0.025 |
| 50 | 0.062 |
| 51 | 0.0056 |
| 52 | 0.0079 |
| 53 | 0.17 |
| 54 | 0.0031 |
| 55 | 1.0 |

### Formulation Example 1

Granules containing the following components are produced.

| | | |
|---|---|---|
| Component | Compound of Formula (I) | 10 mg |
| | Lactose | 700 mg |
| | Cornstarch | 274 mg |
| | HPC-L | 16 mg |
| | Total | 1,000 mg |

The compound of Formula (I) and lactose are sieved through a 60-mesh sieve. Cornstarch is sieved through a 120-mesh sieve. The sieved materials are mixed in a V-type mixer. To the mixed powder, an aqueous solution of low-viscosity hydroxypropylcellulose (HPC-L) is added, then the resultant mixture is kneaded and granulated (extruding granulation, a pore size of 0.5 mm to 1 mm), and the granules are dried. The resulting dried granules are sieved through a vibrating screen (12/60 mesh) to obtain granules.

### Formulation Example 2

Powders that are to be filled into capsules and contain the following components are produced.

| | | |
|---|---|---|
| Component | Compound of Formula (I) | 10 mg |
| | Lactose | 79 mg |
| | Cornstarch | 10 mg |
| | Magnesium stearate | 1 mg |
| | Total | 100 mg |

The compound of Formula (I) and lactose are sieved through a 60-mesh sieve. Cornstarch is sieved through a 120-mesh sieve. The sieved materials and magnesium stearate are mixed in a V-type mixer. Into a No. 5 hard gelatin capsule, 100 mg of the 10% mixture is filled.

### Formulation Example 3

Granules that are to be filled into capsules and contain the following components are produced.

| | | |
|---|---|---|
| Component | Compound of Formula (I) | 15 mg |
| | Lactose | 90 mg |
| | Cornstarch | 42 mg |
| | HPC-L | 3 mg |
| | Total | 150 mg |

The compound of Formula (I) and lactose are sieved through a 60-mesh sieve. Cornstarch is sieved through a 120-mesh sieve. The sieved materials are mixed in a V-type mixer. To the mixed powder, an aqueous solution of low-viscosity hydroxypropylcellulose (HPC-L) is added, then the resultant mixture is kneaded and granulated, and the granules are dried. The resulting dried granules are sieved through a vibrating screen (12/60 mesh), and 150 mg of the sieved granules are filled into a No. 4 hard gelatin capsule.

### Formulation Example 4

Tablets containing the following components are produced.

| | | |
|---|---|---|
| Component | Compound of Formula (I) | 10 mg |
| | Lactose | 90 mg |
| | Microcrystalline cellulose | 30 mg |
| | Magnesium stearate | 5 mg |
| | CMC-Na | 15 mg |
| | Total | 150 mg |

The compound of Formula (I), lactose, microcrystalline cellulose, and CMC-Na (carboxymethylcellulose sodium salt) are sieved through a 60-mesh sieve and mixed. To the mixed powder, magnesium stearate is added to obtain a mixed powder for formulation. The mixed powder is directly compressed to form 150-mg tablets.

### Formulation Example 5

An intravenous formulation is prepared as follows:

| | |
|---|---|
| Compound of Formula (I) | 100 mg |
| Saturated fatty acid glyceride | 1,000 mL |

Usually, the formulated solution is intravenously administered to a patient at a speed of 1 mL/min.

### INDUSTRIAL APPLICABILITY

The compound of the present invention has an excellent T-type calcium channel inhibitory activity and is specifically useful for prevention or treatment of pains, such as chronic pains and acute pains including neuropathic pain, inflammatory pain, cancer pain, and visceral pain, which are caused by diabetic neuropathy, postherpetic neuralgia, trigeminal neuralgia, phantom limb pain, postoperative pain, stump pain, traumatic neurological disorder, carpal tunnel syndrome, plexus neuropathy, glossopharyngeal neuralgia, laryngeal neuralgia, migraine, fibromyalgia syndrome, rheumatoid arthritis, multiple sclerosis, HIV, herpes simplex, syphilis, carcinomatous neuropathy, polyneuropathy, causalgia, low back pain, complex regional pain syndrome (CRPS), thalamic pain, osteoarthritis, spinal cord injury, and cerebral apoplexy.

## Claims

1. A compound of Formula (I): 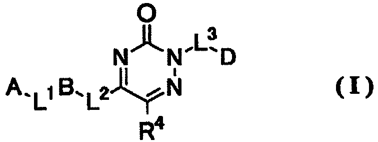 [wherein
R⁴ means a hydrogen atom, a halogen atom, a cyano group, a nitro group, a carboxy group, a carbamoyl group, a sulfamoyl group, a sulfo group, a tetrazolyl group, a C₁₋₆ alkyl group, a C₂₋₆ alkenyl group, a C₂₋₆ alkynyl group, a C₁₋₆ alkoxy group, a C₁₋₆ alkylthio group, a mono-C₁₋₆ alkyl amino group, or a di-C₁₋₆ alkyl amino group (the C₁₋₆ alkyl group, the C₂₋₆ alkenyl group, the C₂₋₆ alkynyl group, the C₁₋₆ alkoxy group, the C₁₋₆ alkylthio group, the mono-C₁₋₆ alkylamino group, and the di-C₁₋₆ alkylamino group are unsubstituted or substituted with one or more substituents independently selected from a substituent group V⁸);
L¹ and L² each independently mean a single bond, NR², O, S, SO, SO₂, or a C₁₋₆ alkylene group (the C₁₋₆ alkylene group is unsubstituted or substituted with one or more substituents independently selected from the substituent group V⁸ and a single methylene group in the C₁₋₆ alkylene group is optionally replaced by O, S, SO₂, C=O, C=S, or NR³);
B means a 3 to 13-membered heterocyclylene group (the 3 to 13-membered heterocyclylene group is unsubstituted or substituted with one or more substituents independently selected from a substituent group V⁶ and a single methylene group in the 3 to 13-membered heterocyclylene group is optionally replaced by a 1,1-C₃₋₇ cycloalkylene group);
A means a hydrogen atom, a C₃₋₁₁ cycloalkyl group, a C₃₋₁₁ cycloalkenyl group, a 3 to 13-membered heterocyclyl group, a C₆₋₁₄ aryl group, or a 5 to 10-membered heteroaryl group (the C₃₋₁₁ cycloalkyl group, the C₃₋₁₁ cycloalkenyl group, the 3 to 13-membered heterocyclyl group, the C₆₋₁₄ aryl group, and the 5 to 10-membered heteroaryl group are unsubstituted or substituted with one or more substituents independently selected from the substituent group V⁶);
L³ means a C₁₋₆ alkylene group (the C₁₋₆ alkylene group is unsubstituted or substituted with one or more substituents independently selected from the substituent group V⁸ and a single methylene group in the C₁₋₆ alkylene group is optionally replaced by C=O or C=S);
D means a 3 to 13-membered heterocyclyl group, a C₆₋₁₄ aryl group, or a 5 to 10-membered heteroaryl group (the 3 to 13-membered heterocyclyl group, the C₆₋₁₄ aryl group, and the 5 to 10-membered heteroaryl group are unsubstituted or substituted with one or more substituents independently selected from the substituent group V⁶);
R² and R³ each independently mean a hydrogen atom, a C₁₋₆ alkyl group, a C₂₋₆ alkenyl group, a C₂₋₆ alkynyl group (the C₁₋₆ alkyl group, the C₂₋₆ alkenyl group, and the C₂₋₆ alkynyl group are unsubstituted or substituted with one or more substituents independently selected from the substituent group V⁸), a C₃₋₁₁ cycloalkyl group, a 3 to 13-membered heterocyclyl group, a C₆₋₁₄ aryl group, or a 5 to 10-membered heteroaryl group (the C₃₋₁₁ cycloalkyl group, the 3 to 13-membered heterocyclyl group, the C₆₋₁₄ aryl group, and the 5 to 10-membered heteroaryl group are unsubstituted or substituted with one or more substituents independently selected from the substituent group V⁶);
the substituent group V⁶ means a substituent group consisting of substituents constituting the substituent group V⁸, C₁₋₆ alkyl groups, C₂₋₆ alkenyl groups, and C₂₋₆ alkynyl groups (the C₁₋₆ alkyl groups, the C₂₋₆ alkenyl groups, and the C₂₋₆ alkynyl groups are unsubstituted or substituted with one or more substituents independently selected from a substituent group V¹);
the substituent group V⁸ means a substituent group consisting of substituents constituting a substituent group V^{a}, C₁₋₁₀ alkoxy groups, C₁₋₆ alkylthio groups, C₁₋₆ alkylcarbonyl groups, C₁₋₆ alkylsulfonyl groups, C₁₋₆ alkoxycarbonyl groups, mono-C₁₋₆ alkylamino groups, di-C₁₋₆ alkylamino groups, mono-C₁₋₆ alkylaminocarbonyl groups, di-C₁₋₆ alkylaminocarbonyl groups, mono-C₁₋₆ alkylaminosulfonyl groups, di-C₁₋₆ alkylaminosulfonyl groups, C₁₋₆ alkylcarbonylamino groups, C₁₋₆ alkylcarbonyloxy groups, C₁₋₆ alkylsulfonylamino groups, C₁₋₆ alkylsulfonyloxy groups (the C₁₋₁₀ alkoxy groups, the C₁₋₆ alkylthio groups, the C₁₋₆ alkylcarbonyl groups, the C₁₋₆ alkylsulfonyl groups, the C₁₋₆ alkoxycarbonyl groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the mono-C₁₋₆ alkylaminosulfonyl groups, the di-C₁₋₆ alkylaminosulfonyl groups, the C₁₋₆ alkylcarbonylamino groups, the C₁₋₆ alkylcarbonyloxy groups, the C₁₋₆ alkylsulfonylamino groups, and the C₁₋₆ alkylsulfonyloxy groups are unsubstituted or substituted with one or more substituents independently selected from the substituent group V¹), C₃₋₆ cycloalkoxy groups, C₆₋₁₄ aryloxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylsulfonylamino groups, C₃₋₆ cycloalkylsulfonyloxy groups, C₃₋₆ cycloalkylthio groups, C₃₋₁₁ cycloalkyl groups, 3 to 13-membered heterocyclyl groups, C₆₋₁₄ aryl groups, and 5 to 10-membered heteroaryl groups (the C₃₋₆ cycloalkoxy groups, the C₆₋₁₄ aryloxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylsulfonylamino groups, the C₃₋₆ cycloalkylsulfonyloxy groups, the C₃₋₆ cycloalkylthio groups, the C₃₋₁₁ cycloalkyl groups, the 3 to 13-membered heterocyclyl groups, the C₆₋₁₄ aryl groups, and the 5 to 10-membered heteroaryl groups are unsubstituted, or substituted with one or more substituents independently selected from a substituent group V²);
the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group;
the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups, (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di C₁₋₆ alkylaminocarbonyl groups, C₁₋₆ alkylcarbonylamino groups, C₁₋₆ alkylthio groups, and C₁₋₆ alkylsulfonyl groups); and
the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups)], a tautomer of the compound, a pharmaceutically acceptable salt of the compound, or a solvate of the compound, the tautomer, or the pharmaceutically acceptable salt.

2. The compound, the tautomer of the compound, the pharmaceutically acceptable salt of the compound, or the solvate of the compound, the tautomer, or the pharmaceutically acceptable salt according to claim 1, in which L³ is a C₁₋₃ alkylene group.

3. The compound, the tautomer of the compound, the pharmaceutically acceptable salt of the compound, or the solvate of the compound, the tautomer, or the pharmaceutically acceptable salt according to claim 2, in which L³ is a methylene group.

4. The compound, the tautomer of the compound, the pharmaceutically acceptable salt of the compound, or the solvate of the compound, the tautomer, or the pharmaceutically acceptable salt according to any one of claims 1 to 3, in which R⁴ is a hydrogen atom.

5. The compound, the tautomer of the compound, the pharmaceutically acceptable salt of the compound, or the solvate of the compound, the tautomer, or the pharmaceutically acceptable salt according to any one of claims 1 to 4, in which L¹ and L² are single bonds;
B is a 3 to 13-membered heterocyclylene group (the 3 to 13-membered heterocyclylene group is unsubstituted or substituted with one or more substituents independently selected from a substituent group V⁵ and a single methylene group in the 3 to 13-membered heterocyclylene group is optionally replaced by a 1,1-C₃₋₇ cycloalkylene group);
the substituent group V⁵ means a substituent group consisting of a hydroxy group, halogen atoms, a cyano group, a nitro group, an amino group, C₁₋₆ alkyl groups, C₂₋₆ alkenyl groups, C₂₋₆ alkynyl groups, C₁₋₆ alkoxy groups, C₁₋₆ alkylthio groups, C₁₋₆ alkoxycarbonyl groups, mono-C₁₋₆ alkylamino groups, di-C₁₋₆ alkylamino groups, C₁₋₆ alkylsulfonylamino groups, C₁₋₆ alkylsulfonyloxy groups (the C₁₋₆ alkyl groups, the C₂₋₆ alkenyl groups, the C₂₋₆ alkynyl groups, the C₁₋₆ alkoxy groups, the C₁₋₆ alkylthio groups, the C₁₋₆ alkoxycarbonyl groups, the mono-C₁₋₆ alkylamino groups, the di-C₁₋₆ alkylamino groups, the C₁₋₆ alkylsulfonylamino groups, and the C₁₋₆ alkylsulfonyloxy groups are unsubstituted, or substituted with one or more substituents independently selected from a substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents independently selected from the substituent group V²);
the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups); and
the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group.

6. The compound, the tautomer of the compound, the pharmaceutically acceptable salt of the compound, or the solvate of the compound, the tautomer, or the pharmaceutically acceptable salt according to claim 5, in which B is a 4 to 7-membered heterocyclylene group (the 4 to 7-membered heterocyclylene group is unsubstituted or substituted with one or more substituents independently selected from a substituent group V³ and a single methylene group in the 4 to 7-membered heterocyclylene group is optionally replaced by a 1,1-C₃₋₇ cycloalkylene group); and
the substituent group V³ means a substituent group consisting of a hydroxy group, halogen atoms, a cyano group, a nitro group, an amino group, C₁₋₆ alkyl groups, C₁₋₆ haloalkyl groups, C₃₋₆ cycloalkyl groups, C₁₋₆ alkoxy groups, C₁₋₆ haloalkoxy groups, mono-C₁₋₆ alkylamino groups, di-C₁₋₆ alkylamino groups, and C₁₋₆ alkylsulfonylamino groups.

7. The compound, the tautomer of the compound, the pharmaceutically acceptable salt of the compound, or the solvate of the compound, the tautomer, or the pharmaceutically acceptable salt according to claim 6, in which B is a 6-membered heterocyclylene group (the 6-membered heterocyclylene group is unsubstituted or substituted with one or more substituents independently selected from the group consisting of a hydroxy group, halogen atoms, and an amino group).

8. The compound, the tautomer of the compound, the pharmaceutically acceptable salt of the compound, or the solvate of the compound, the tautomer, or the pharmaceutically acceptable salt according to claim 5, in which A is a hydrogen atom;
B is a tetrahydroisoquinoline-diyl group or a tetrahydrocarboline-diyl group (the tetrahydroisoquinoline-diyl group and the tetrahydrocarboline-diyl group are unsubstituted, or substituted with one or more substituents independently selected from a substituent group V³, and further a single methylene group in the tetrahydroisoquinoline-diyl group and the tetrahydrocarboline-diyl group is optionally replaced by a 1,1-C₃₋₇ cycloalkylene group); and
the substituent group V³ means a substituent group consisting of a hydroxy group, halogen atoms, a cyano group, a nitro group, an amino group, C₁₋₆ alkyl groups, C₁₋₆ haloalkyl groups, C₃₋₆ cycloalkyl groups, C₁₋₆ alkoxy groups, C₁₋₆ haloalkoxy groups, mono-C₁₋₆ alkylamino groups, di-C₁₋₆ alkylamino groups, and C₁₋₆ alkylsulfonylamino groups.

9. The compound, the tautomer of the compound, the pharmaceutically acceptable salt of the compound, or the solvate of the compound, the tautomer, or the pharmaceutically acceptable salt according to claim 8, in which A-L¹-B is either a structure of Formula (II-1) or a structure of Formula (II-2): 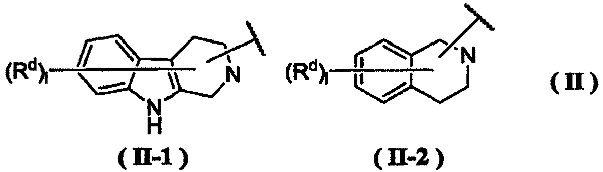 (wherein 1 is 1 to 3; R^{d} is optionally substituted for a tetrahydrocarboline ring or a tetrahydroisoquinoline ring at any positions; R^{d} means a hydroxy group, a halogen atom, an amino group, a C₁₋₆ alkyl group, a C₁₋₆ haloalkyl group, a C₁₋₆ alkoxy group, a C₁₋₆ haloalkoxy group, a mono-C₁₋₆ alkylamino group, a di-C₁₋₆ alkylamino group, or a C₁₋₆ alkylsulfonylamino group; and when 1 is 2 or 3, R^{d}s are the same as or different from each other).

10. The compound, the tautomer of the compound, the pharmaceutically acceptable salt of the compound, or the solvate of the compound, the tautomer, or the pharmaceutically acceptable salt according to claims 1 to 7, in which A is a C₆₋₁₄ aryl group or a 5 to 10-membered heteroaryl group (the C₆₋₁₄ aryl group and the 5 to 10-membered heteroaryl group are unsubstituted or substituted with one or more substituents independently selected from a substituent group V⁵);
the substituent group V⁵ means a substituent group consisting of a hydroxy group, halogen atoms, a cyano group, a nitro group, an amino group, C₁₋₆ alkyl groups, C₂₋₆ alkenyl groups, C₂₋₆ alkynyl groups, C₁₋₆ alkoxy groups, C₁₋₆ alkylthio groups, C₁₋₆ alkoxycarbonyl groups, mono-C₁₋₆ alkylamino groups, di-C₁₋₆ alkylamino groups, C₁₋₆ alkylsulfonylamino groups, C₁₋₆ alkylsulfonyloxy groups (the C₁₋₆ alkyl groups, the C₂₋₆ alkenyl groups, the C₂₋₆ alkynyl groups, the C₁₋₆ alkoxy groups, the C₁₋₆ alkylthio groups, the C₁₋₆ alkoxycarbonyl groups, the mono-C₁₋₆ alkylamino groups, the di-C₁₋₆ alkylamino groups, the C₁₋₆ alkylsulfonylamino groups, and the C₁₋₆ alkylsulfonyloxy groups are unsubstituted, or substituted with one or more substituents independently selected from a substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents independently selected from a substituent group V²);
the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino group, C₁₋₆ alkylthio groups, and C₁₋₆ alkylsulfonyl groups); and
the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group.

11. The compound, the tautomer of the compound, the pharmaceutically acceptable salt of the compound, or the solvate of the compound, the tautomer, or the pharmaceutically acceptable salt according to claim 10, in which A is a phenyl group or a 5 to 6-membered heteroaryl group (the phenyl group and the 5 to 6-membered heteroaryl group are unsubstituted or substituted with one or more substituents independently selected from a substituent group V⁴); and
the substituent group V⁴ means a substituent group consisting of a hydroxy group, halogen atoms, a cyano group, a nitro group, an amino group, C₁₋₆ alkyl groups, C₁₋₆ alkoxy groups, mono-C₁₋₆ alkylamino groups, di-C₁₋₆ alkylamino groups, C₁₋₆ alkylsulfonylamino groups, C₁₋₆ alkylsulfonyloxy groups, C₃₋₆ cycloalkyl groups, C₃₋₆ cycloalkoxy groups, mono-C₃₋₆ cycloalkylamino groups, di-C₃₋₆ cycloalkylamino groups, and C₃₋₆ cycloalkylthio groups (the C₁₋₆ alkyl groups, the C₁₋₆ alkoxy groups, the mono-C₁₋₆ alkylamino groups, the di-C₁₋₆ alkylamino groups, the C₁₋₆ alkylsulfonylamino groups, the C₁₋₆ alkylsulfonyloxy groups, the C₃₋₆ cycloalkyl groups, the C₃₋₆ cycloalkoxy groups, the mono-C₃₋₆ cycloalkylamino groups, the di-C₃₋₆ cycloalkylamino groups, and the C₃₋₆ cycloalkylthio groups are unsubstituted or substituted with one or more substituents independently selected from the group consisting of a hydroxy group, halogen atoms, an amino group, a nitro group, a cyano group, C₁₋₆ alkoxy groups, C₁₋₃ haloalkoxy groups, 3 to 13-membered heterocyclyl groups, C₆₋₁₄ aryl groups, and 5 to 10-membered heteroaryl groups (the 3 to 13-membered heterocyclyl groups, the C₆₋₁₄ aryl groups, and the 5 to 10-membered heteroaryl groups are unsubstituted or substituted with one or more substituents independently selected from the group consisting of a carboxy group, a carbamoyl group, a sulfamoyl group, a phosphono group, a sulfo group, a tetrazolyl group, a formyl group, a nitro group, a cyano group, halogen atoms, a hydroxy group, an amino group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups)).

12. The compound, the tautomer of the compound, the pharmaceutically acceptable salt of the compound, or the solvate of the compound, the tautomer, or the pharmaceutically acceptable salt according to claim 11, in which A is phenyl group (the phenyl group has one or more substituents independently selected from the group consisting of halogen atoms, a nitro group, C₁₋₆ alkyl groups, C₁₋₆ haloalkyl groups, C₁₋₆ alkoxy groups, and C₁₋₆ haloalkoxy groups.

13. The compound, the tautomer of the compound, the pharmaceutically acceptable salt of the compound, or the solvate of the compound, the tautomer, or the pharmaceutically acceptable salt according to claim 11, in which A is a thienyl group, a pyridyl group, a pyrazolyl group, or a furanyl group (the thienyl group, the pyridyl group, the pyrazolyl group, and the furanyl group have one or more substituents independently selected from the group consisting of halogen atoms, C₁₋₆ alkyl groups, C₁₋₆ haloalkyl groups, C₁₋₆ alkoxy groups, and C₁₋₆ haloalkoxy groups).

14. The compound, the tautomer of the compound, the pharmaceutically acceptable salt of the compound, or the solvate of the compound, the tautomer, or the pharmaceutically acceptable salt according to claim 11, in which A is a thienyl group or a pyridyl group (the thienyl group and the pyridyl group have one or more substituents independently selected from the group consisting of halogen atoms, C₁₋₆ alkyl groups, C₁₋₆ haloalkyl groups, C₁₋₆ alkoxy groups, and C₁₋₆ haloalkoxy groups).

15. The compound, the tautomer of the compound, the pharmaceutically acceptable salt of the compound, or the solvate of the compound, the tautomer, or the pharmaceutically acceptable salt according to any one of claims 1 to 14, in which D is a C₆₋₁₄ aryl group or a 5 to 10-membered heteroaryl group (the C₆₋₁₄ aryl group and the 5 to 10-membered heteroaryl group are unsubstituted or substituted with one or more substituents independently selected from a substituent group V⁵);
the substituent group V⁵ means a substituent group consisting of a hydroxy group, halogen atoms, a cyano group, a nitro group, an amino group, C₁₋₆ alkyl groups, C₂₋₆ alkenyl groups, C₂₋₆ alkynyl groups, C₁₋₆ alkoxy groups, C₁₋₆ alkylthio groups, C₁₋₆ alkoxycarbonyl groups, mono-C₁₋₆ alkylamino groups, di-C₁₋₆ alkylamino groups, C₁₋₆ alkylsulfonylamino groups, C₁₋₆ alkylsulfonyloxy groups (the C₁₋₆alkyl groups, the C₂₋₆ alkenyl groups, the C₂₋₆ alkynyl groups, the C₁₋₆ alkoxy groups, the C₁₋₆ alkylthio groups, the C₁₋₆ alkoxycarbonyl groups, the mono-C₁₋₆ alkylamino groups, the di-C₁₋₆ alkylamino groups, the C₁₋₆ alkylsulfonylamino groups, and the C₁₋₆ alkylsulfonyloxy groups are unsubstituted, or substituted with one or more substituents independently selected from a substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents independently selected from a substituent group V²);
the substituent group V¹ means a substituent group consisting of substituents constituting the substituent group V^{a}, C₁₋₆ alkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 13-membered heterocyclyl groups, C₆₋₁₄ aryl groups, and 5 to 10-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 13-membered heterocyclyl groups, the C₆₋₁₄ aryl groups, and 5 to 10-membered heteroaryl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
the substituent group V² means a substituent group consisting of substituents constituting the substituent group V¹, C₁₋₆ alkyl groups, C₂₋₆ alkenyl groups, and C₂₋₆ alkynyl groups (the C₁₋₆ alkyl groups, the C₂₋₆ alkenyl groups, and C₂₋₆ alkynyl groups are unsubstituted or substituted with one or more substituents independently selected from the group consisting of a hydroxy group, halogen atoms, a cyano group, a nitro group, an amino group, a carboxy group, a carbamoyl group, a sulfamoyl group, a phosphono group, a phosphinoyl group, a sulfo group, a sulfino group, a tetrazolyl group, a formyl group, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups);
the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group.

16. The compound, the tautomer of the compound, the pharmaceutically acceptable salt of the compound, or the solvate of the compound, the tautomer, or the pharmaceutically acceptable salt according to claim 15, in which D is phenyl group (the phenyl group is unsubstituted or substituted with one or more substituents independently selected from a substituent group V⁴); and
the substituent group V⁴ means a substituent group consisting of a hydroxy group, halogen atoms, a cyano group, a nitro group, an amino group, C₁₋₆ alkyl groups, C₁₋₆ alkoxy groups, mono-C₁₋₆ alkylamino groups, di-C₁₋₆ alkylamino groups, C₁₋₆ alkylsulfonylamino groups, C₁₋₆ alkylsulfonyloxy groups, C₃₋₆ cycloalkyl groups, C₃₋₆ cycloalkoxy groups, mono-C₃₋₆ cycloalkylamino groups, di-C₃₋₆ cycloalkylamino groups, and C₃₋₆ cycloalkylthio groups (the C₁₋₆ alkyl groups, the C₁₋₆ alkoxy groups, the mono-C₁₋₆ alkylamino groups, the di-C₁₋₆ alkylamino groups, the C₁₋₆ alkylsulfonylamino groups, the C₁₋₆ alkylsulfonyloxy groups, the C₃₋₆ cycloalkyl groups, the C₃₋₆ cycloalkoxy groups, the mono-C₃₋₆ cycloalkylamino groups, the di-C₃₋₆ cycloalkylamino groups, and the C₃₋₆ cycloalkylthio groups are unsubstituted or substituted with one or more substituents independently selected from the group consisting of a hydroxy group, halogen atoms, an amino group, a nitro group, a cyano group, C₁₋₆ alkoxy groups, C₁₋₃ haloalkoxy groups, 3 to 13-membered heterocyclyl groups, C₆₋₁₄ aryl groups, or 5 to 10-membered heteroaryl groups (the 3 to 13-membered heterocyclyl groups, the C₆₋₁₄ aryl groups, and the 5 to 10-membered heteroaryl groups are unsubstituted or substituted with one or more substituents independently selected from the group consisting of a carboxy group, a carbamoyl group, a sulfamoyl group, a phosphono group, a sulfo group, a tetrazolyl group, a formyl group, a nitro group, a cyano group, halogen atoms, a hydroxy group, an amino group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups)).

17. The compound, the tautomer of the compound, the pharmaceutically acceptable salt of the compound, or the solvate of the compound, the tautomer, or the pharmaceutically acceptable salt according to claim 16, in which D is a phenyl group (the phenyl group has one or more substituents independently selected from the group consisting of halogen atoms, a nitro group, C₁₋₆ alkyl groups, and C₁₋₆ alkoxy groups (the C₁₋₆ alkyl groups and the C₁₋₆ alkoxy groups are unsubstituted or substituted with one or more substituents independently selected from the group consisting of halogen atoms, C₁₋₆ alkoxy groups, C₁₋₃ haloalkoxy groups, and a phenyl group (the phenyl group is unsubstituted or substituted with one or more halogen atoms))).

18. The compound, the tautomer of the compound, the pharmaceutically acceptable salt of the compound, or the solvate of the compound, the tautomer, or the pharmaceutically acceptable salt according to claim 15, in which D is a 5 to 6-membered heteroaryl group (the 5 to 6-membered heteroaryl group is unsubstituted or substituted with one or more substituents independently selected from a substituent group V⁴); and
the substituent group V⁴ means a substituent group consisting of a hydroxy group, halogen atoms, a cyano group, a nitro group, an amino group, C₁₋₆ alkyl groups, C₁₋₆ alkoxy groups, mono-C₁₋₆ alkylamino groups, di-C₁₋₆ alkylamino groups, C₁₋₆ alkylsulfonylamino groups, C₁₋₆ alkylsulfonyloxy groups, C₃₋₆ cycloalkyl groups, C₃₋₆ cycloalkoxy groups, mono-C₃₋₆ cycloalkylamino groups, di-C₃₋₆ cycloalkylamino groups, and C₃₋₆ cycloalkylthio groups (the C₁₋₆ alkyl groups, the C₁₋₆ alkoxy groups, the mono-C₁₋₆ alkylamino groups, the di-C₁₋₆ alkylamino groups, the C₁₋₆ alkylsulfonylamino groups, the C₁₋₆ alkylsulfonyloxy groups, the C₃₋₆ cycloalkyl groups, the C₃₋₆ cycloalkoxy groups, the mono-C₃₋₆ cycloalkylamino groups, the di-C₃₋₆ cycloalkylamino groups, and the C₃₋₆ cycloalkylthio groups are unsubstituted or substituted with one or more substituents independently selected from the group consisting of a hydroxy group, halogen atoms, an amino group, a nitro group, a cyano group, C₁₋₆ alkoxy groups, C₁₋₃ haloalkoxy groups, 3 to 13-membered heterocyclyl groups, C₆₋₁₄ aryl groups, and 5 to 10-membered heteroaryl groups (the 3 to 13-membered heterocyclyl groups, the C₆₋₁₄ aryl groups, and the 5 to 10-membered heteroaryl groups are unsubstituted or substituted with one or more substituents independently selected from the group consisting of a carboxy group, a carbamoyl group, a sulfamoyl group, a phosphono group, a sulfo group, a tetrazolyl group, a formyl group, a nitro group, a cyano group, halogen atoms, a hydroxy group, an amino group, C₁₋₆ alkyl groups, C₁₋₃ haloalkyl groups, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, and C₁₋₆ alkylsulfonyl groups)).

19. The compound, the tautomer of the compound, the pharmaceutically acceptable salt of the compound, or the solvate of the compound, the tautomer, or the pharmaceutically acceptable salt according to claim 18, in which D is a 5 to 6-membered heteroaryl group (the 5 to 6-membered heteroaryl group is unsubstituted or substituted with one or more substituents independently selected from the group consisting of halogen atoms, a nitro group, C₁₋₆ alkyl groups, C₁₋₆ haloalkyl groups, C₁₋₆ alkoxy groups, C₁₋₆ haloalkoxy groups (the C₁₋₆ alkyl groups, the C₁₋₆ haloalkyl groups, the C₁₋₆ alkoxy groups, and the C₁₋₆ haloalkoxy groups are unsubstituted or substituted with one or more substituents independently selected from the group consisting of a nitro group, C₁₋₆ alkoxy groups, C₁₋₃ haloalkoxy groups, and C₆₋₁₄ aryl groups (the C₆₋₁₄ aryl groups are unsubstituted or substituted with one or more halogen atoms)), C₁₋₆ alkylsulfonylamino groups and C₁₋₆ alkylsulfonyloxy groups (the C₁₋₆ alkylsulfonylamino groups and the C₁₋₆ alkylsulfonyloxy groups are unsubstituted or substituted with one or more substituents independently selected from the group consisting of halogen atoms, a nitro group, C₁₋₆ alkoxy groups, and C₁₋₃ haloalkoxy groups)).

20. The compound, the tautomer of the compound, the pharmaceutically acceptable salt of the compound, or the solvate of the compound, the tautomer, or the pharmaceutically acceptable salt according to claim 19, in which D is a thienyl group or a thiazolyl group (the thienyl group and the thiazolyl group have one or more substituents independently selected from the group consisting of halogen atoms, a nitro group, C₁₋₆ alkyl groups, C₁₋₆ haloalkyl groups, C₁₋₆ alkoxy groups, and C₁₋₆ haloalkoxy groups).

21. AT-type calcium channel inhibitor comprising the compound, the tautomer of the compound, the pharmaceutically acceptable salt of the compound, or the solvate of the compound, the tautomer, or the pharmaceutically acceptable salt as claimed in any one of claims 1 to 20, as an active component.

22. A preventive agent, a therapeutic agent, and/or an improving agent for a disease treatable by a T-type calcium channel inhibitory activity comprising the T-type calcium channel inhibitor as claimed in claim 21 as an active component.

23. A therapeutic agent for neuropathic pain comprising the T-type calcium channel inhibitor as claimed in claim 21 as an active component.

24. A medicine comprising the compound, the tautomer of the compound, the pharmaceutically acceptable salt of the compound, or the solvate of the compound, the tautomer, or the pharmaceutically acceptable salt as claimed in any one of claims 1 to 20, as an active component.
